(19) Europäisches Patentamt
European Patent Office
Office européen des brevets

(11) EP 3 578 551 A1

(12) EUROPEAN PATENT APPLICATION

(43) Date of publication:
**11.12.2019 Bulletin 2019/50**

(21) Application number: **18175823.6**

(22) Date of filing: **04.06.2018**

(51) Int Cl.:
***C07D 277/52*** *(2006.01)* ***C07D 417/12*** *(2006.01)*
***C07D 417/14*** *(2006.01)* ***A61K 31/426*** *(2006.01)*
***A61K 31/4439*** *(2006.01)* ***A61K 31/444*** *(2006.01)*
***A61P 37/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Step Pharma S.A.S.**
**75272 Paris, Cedex 06 (FR)**

(72) Inventor: **The designation of the inventor has not yet been filed**

(74) Representative: **Kinch, Alison**
**Sagittarius IP**
**Marlow International**
**Parkway**
**Marlow SL7 1YL (GB)**

(54) **SULFONAMIDE DERIVATIVES**

(57) Compounds of formula (I):

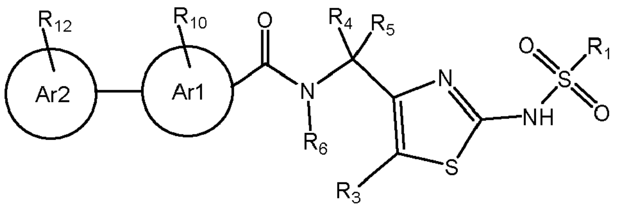

(I)

and use thereof as cytidine triphosphate synthase 1 (CTPS1) inhibitors in the treatment of related pathologies or pathological conditions such as rejection of transplanted cells and tissues, Graft-related diseases or disorders, allergies and autoimmune diseases.

EP 3 578 551 A1

## Description

### Field of the invention

The invention relates to novel benzamide compounds, processes for the manufacture of such compounds, related intermediates, compositions comprising such compounds and the use of such compounds as cytidine triphosphate synthase 1 inhibitors, particularly in the treatment or prophylaxis of disorders associated with cell proliferation.

### Background of the invention

Nucleotides are a key building block for cellular metabolic processes such as deoxyribonucleic acid (DNA) and ribonucleic acid (RNA) synthesis. There are two classes of nucleotides, purines and pyrimidines and both classes are important for metabolic processes. Based on this, many therapies have been developed to target different aspects of nucleotide synthesis, with some inhibiting generation of purine nucleotides and some pyrimidine nucleotides.

The pyrimidine nucleotide cytidine 5' triphosphate (CTP) is a precursor required not just for the metabolism of DNA and RNA but also phospholipids and sialyation of proteins. CTP originates from two sources: a salvage pathway and a *de novo* synthesis pathway that depends on two enzymes, the CTP synthases (or synthetases) 1 and 2 (CTPS1 and CTPS2) (Evans and Guy 2004; Higgins, *et al.* 2007; Ostrander, *et al.* 1998).

CTPS1 and CTPS2 catalyse the conversion of uridine triphosphate (UTP) and glutamine into cytidine triphosphate (CTP):

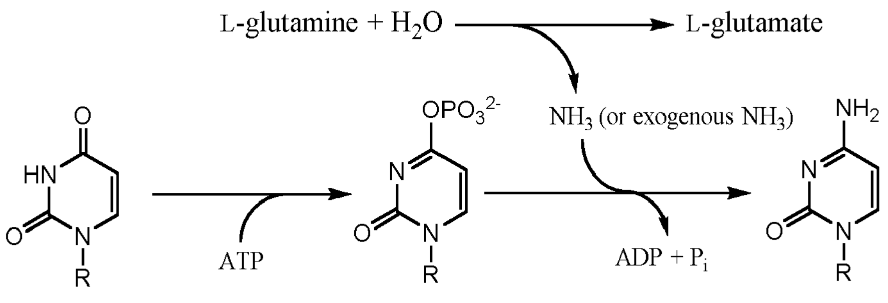

UTP    4-phospho-UTP    CTP

Both enzymes have two domains, an N-terminal synthetase domain and a C-terminal glutaminase domain (Kursula, *et al.* 2006). The synthetase domain transfers a phosphate from adenosine triphosphate (ATP) to the 4-position of UTP to create an activated intermediate, 4-phospho-UTP. The glutaminase domain generates ammonia from glutamine, via a covalent thioester intermediate with a conserved active site cysteine, generating glutamate. This ammonium is transferred from the glutaminase domain to the synthetase domain *via* a tunnel or can be derived from external ammonium. This ammonium is then used by the synthetase domain to generate CTP from the 4-phospho-UTP (Lieberman, 1956).

Although CTPS exists as two isoforms in humans and other eukaryotic organisms, CTPS1 and CTPS2, functional differences between the two isoforms are not yet fully elucidated (van Kuilenburg, *et al.* 2000).

The immune system provides protection from infections and has therefore evolved to rapidly respond to the wide variety of pathogens that the individual may be exposed to. This response can take many forms, but the expansion and differentiation of immune populations is a critical element and is hence closely linked to metabolic processes. Within this, CTP synthase activity appears to play an important role in DNA synthesis and the rapid expansion of lymphocytes following activation (Fairbanks, *et al.* 1995; van den Berg, *et al.* 1995).

Strong validation that CTPS1 is the critical enzyme in human lymphocyte proliferation came with the identification of a loss-of-function homozygous mutation (rs145092287) in this enzyme that causes a distinct and life-threatening immunodeficiency, characterized by an impaired capacity of activated T- and B-cells to proliferate in response to antigen receptor-mediated activation. Activated CTPS1-deficient cells were shown to have decreased levels of CTP. Normal T-cell proliferation was restored in CTPS1-deficient cells by expressing wild-type CTPS1 or by addition of exogenous CTP or its nucleoside precursor, cytidine. CTPS1 expression was found to be low in resting lymphocytes, but rapidly upregulated following activation of these cells. Expression of CTPS1 in other tissues was generally low. CTPS2 seems to be ubiquitously expressed in a range of cells and tissues but at low levels, and the failure of CTPS2, which is still intact in the patients, to compensate for the mutated CTPS1, supports CTPS1 being the critical enzyme for the immune populations affected in the patients (Martin, *et al.* 2014).

Overall, these findings suggest that CTPS1 is a critical enzyme necessary to meet the demands for the supply

of CTP required by several important immune populations.

Normally the immune response is tightly regulated to ensure protection from infection, whilst controlling any response targeting host tissues. In certain situations, the control of this process is not effective, leading to immune-mediated pathology. A wide range of human diseases are thought to be due to such inappropriate responses mediated by different elements of the immune system.

Given the role that cell populations, such as T and B lymphocytes, are thought to play in a wide range of autoimmune and other diseases, CTPS1 represents a target for a new class of immunosuppressive agents. Inhibition of CTPS1 therefore provides a novel approach to the inhibition of activated lymphocytes and selected other immune cell populations such as Natural Killer cells, Mucosal-Associated Invariant T (MAIT) and Invariant Natural Killer T cells, highlighted by the phenotype of the human mutation patients (Martin, *et al.* 2014).

As far as is known to date, no selective CTPS1 inhibitors have been developed. Several (presumed) non-selective CTPS inhibitors have been used for oncology indications up to phase I/II clinical trials, but were stopped due to toxicity and efficacy issues. More recently, the CTPS1 selective inhibitory peptide CTpep-3 has been identified. The inhibitory effects of CTpep-3 however, were seen in cell free assays but not in the cellular context. This was not unexpected though, since the peptide is unlikely to enter the cell and hence is not easily developable as a therapeutic (Sakamoto, *et al.* 2017).

In summary, the available information and data strongly suggest that inhibitors of CTPS1 will reduce the proliferation of a number of immune cell populations. Inhibitors of CTPS1 may therefore be expected to have utility for treatment or prophylaxis in a wide range of indications where the pathology is driven by these populations.

CTPS1 inhibitors represent a novel approach for inhibiting selected components of the immune system in various tissues, and the related pathologies or pathological conditions such as, in general terms, rejection of transplanted cells and tissues, Graft-related diseases or disorders, allergies and autoimmune diseases.

**Summary of the Invention**

The invention provides a compound of formula (I):

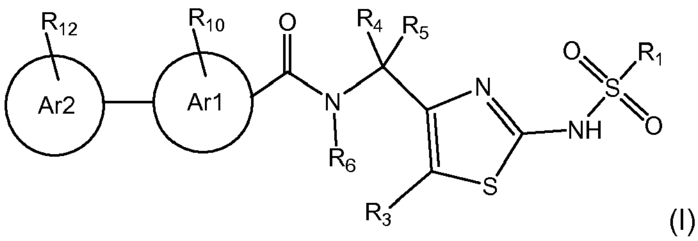

(I)

wherein

$R_1$ is $C_{1-4}$alkyl, $C_{1-2}$alkyleneO$C_{1-2}$alkyl or $C_{0-1}$alkylene$C_{3-4}$cycloalkyl which cycloalkyl is optionally substituted by $CH_3$;

$R_3$ is H, $CH_3$, F or Cl;

$R_4$ and $R_5$ are each independently H, $C_{1-4}$alkyl, $C_{0-2}$alkylene$C_{3-5}$cycloalkyl, $C_{1-3}$alkyleneO$C_{1-3}$alkyl, $C_{1-4}$alkylOH or $C_{1-4}$haloalkyl;

$R_6$ is H or $C_{1-3}$alkyl;

Ar1 is 6-membered aryl or heteroaryl;

Ar2 is a 6-membered aryl or heteroaryl and is attached to Ar1 in the para position relative to the amide;

$R_{10}$ is H, halo, $C_{1-3}$alkyl, O$C_{1-2}$alkyl, $C_{1-2}$haloalkyl, O$C_{1-2}$haloalkyl or CN;

$R_{12}$ is attached to Ar2 in the meta position relative to Ar1 and $R_{12}$ is H, halo, $C_{1-4}$alkyl, $C_2$alkynyl, C(=O)$C_{1-2}$alkyl, $C_{0-2}$alkylene$C_{3-5}$cycloalkyl, O$C_{1-4}$alkyl, $C_{1-3}$alkyleneO$C_{1-3}$alkyl, $C_{1-4}$haloalkyl, O$C_{1-4}$haloalkyl or CN.

A compound of formula (I) may be provided in the form of a salt and/or solvate thereof and/or derivative thereof.

Suitably, the compound of formula (I) may be provided in the form of a pharmaceutically acceptable salt and/or solvate thereof and/or derivative thereof. In particular, the compound of formula (I) may be provided in the form of a pharmaceutically acceptable salt and/or solvate, such as a pharmaceutically acceptable salt.

Also provided is a compound of formula (I), or a pharmaceutically acceptable salt and/or solvate thereof and/or derivative thereof, for use as a medicament, in particular for use in the inhibition of CTPS1 in a subject or the prophylaxis or treatment of associated diseases or disorders, such as those in which a reduction in T-cell and/or B-cell proliferation would be beneficial.

Further, there is provided a method for the inhibition of CTPS1 in a subject or the prophylaxis or treatment of associated diseases or disorders, such as those in which a reduction in T-cell and/or B-cell proliferation would be beneficial, by administering to a subject in need thereof a compound of formula (I) or a pharmaceutically acceptable salt and/or solvate thereof and/or derivative thereof.

Additionally provided is the use of a compound of formula (I), or a pharmaceutically acceptable salt and/or solvate thereof and/or derivative thereof, in the manufacture of a medicament for the inhibition of CTPS1 in a subject or the prophylaxis or treatment of associated diseases or disorders, such as those in which a reduction in T-cell and/or B-cell proliferation would be beneficial.

Suitably the disease or disorder is selected from: inflammatory skin diseases such as psoriasis or lichen planus; acute and/or chronic GVHD such as steroid resistant acute GVHD; acute lymphoproliferative syndrome (ALPS); systemic lupus erythematosus, lupus nephritis or cutaneous lupus; and transplantation.

Also provided are pharmaceutical compositions containing a compound of formula (I), or a pharmaceutically acceptable salt and/or solvate thereof and/or derivative thereof, and a pharmaceutically acceptable carrier or excipient.

Also provided are processes for preparing compounds of formula (I) and novel intermediates of use in the preparation of compounds of formula (I).

**Detailed description of the Invention**

The invention provides a compound of formula (I):

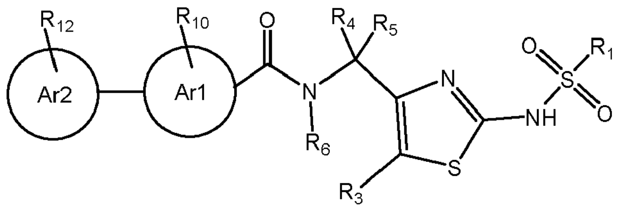

(I)

wherein

$R_1$ is $C_{1-4}$alkyl, $C_{1-2}$alkyleneO$C_{1-2}$alkyl or $C_{0-1}$alkylene$C_{3-4}$cycloalkyl which cycloalkyl is optionally substituted by $CH_3$;

$R_3$ is H, $CH_3$, F or Cl;

$R_4$ and $R_5$ are each independently H, $C_{1-4}$alkyl, $C_{0-2}$alkylene$C_{3-5}$cycloalkyl, $C_{1-3}$alkyleneO$C_{1-3}$alkyl, $C_{1-4}$alkylOH or $C_{1-4}$haloalkyl;

$R_6$ is H or $C_{1-3}$alkyl;

Ar1 is 6-membered aryl or heteroaryl;

Ar2 is a 6-membered aryl or heteroaryl and is attached to Ar1 in the para position relative to the amide;

$R_{10}$ is H, halo, $C_{1-3}$alkyl, O$C_{1-2}$alkyl, $C_{1-2}$haloalkyl, O$C_{1-2}$haloalkyl or CN;

$R_{12}$ is attached to Ar2 in the meta position relative to Ar1 and $R_{12}$ is H, halo, $C_{1-4}$alkyl, $C_2$alkynyl, C(=O)$C_{1-2}$alkyl, $C_{0-2}$alkylene$C_{3-5}$cycloalkyl, O$C_{1-4}$alkyl, $C_{1-3}$alkyleneO$C_{1-3}$alkyl, $C_{1-4}$haloalkyl, O$C_{1-4}$haloalkyl or CN;

or a salt and/or solvate thereof and/or derivative thereof.

The term 'alkyl' as used herein, such as in $C_{1-2}$alkyl, $C_{1-3}$alkyl or $C_{1-4}$alkyl, whether alone or forming part of a larger group such as an Oalkyl group (e.g. $OC_{1-2}$alkyl, $OC_{1-3}$alkyl or $OC_{1-4}$alkyl), is a straight or a branched fully saturated hydrocarbon chain containing the specified number of carbon atoms. Examples of alkyl groups include the $C_{1-4}$alkyl groups methyl, ethyl, *n*-propyl, *iso*-propyl, *n*-butyl, *iso*-butyl, *sec*-butyl and *tert*-butyl, in particular the $C_{1-3}$alkyl groups methyl, ethyl, *n*-propyl and *iso*-propyl such as $C_{1-2}$alkyl groups methyl and ethyl. Reference to "propyl" includes *n*-propyl and *iso*-propyl, and reference to "butyl" includes *n*-butyl, isobutyl, *sec*-butyl and *tert*-butyl. Examples of Oalkyl groups include the $OC_{1-4}$alkyl groups methoxy, ethoxy, propoxy (which includes *n*-propoxy and *iso*-propoxy) and butoxy (which includes *n*-butoxy, *iso*-butoxy, *sec*-butoxy and *tert*-butoxy).

The term 'alkylene' as used herein, such as in $C_{0-2}$alkylene$C_{3-5}$cycloalkyl, $C_{1-3}$alkylene$OC_{1-3}$alkyl, $C_{1-2}$alkylene$OC_{1-2}$alkyl or $C_{0-1}$alkylene$C_{3-4}$cycloalkyl is a bifunctional straight or a branched fully saturated hydrocarbon chain containing the specified number of carbon atoms. Examples of $C_{0-2}$alkylene groups are where the group is absent (i.e. Co), methylene ($C_1$) and ethylene ($C_2$). Examples of $C_{1-3}$alkylene groups are where the group is methylene ($C_1$), ethylene ($C_2$) and propylene ($C_3$). Examples of $C_{1-2}$alkylene groups are where the group is methylene ($C_1$) and ethylene ($C_2$). Examples of $C_{0-1}$alkylene groups are where the group is absent ($C_0$) and methylene ($C_1$).

The term 'cycloalkyl' as used herein, such as in $C_{3-5}$cycloalkyl or $C_{3-4}$cycloalkyl, whether alone or forming part of a larger group such as $C_{0-2}$alkylene$C_{3-5}$cycloalkyl or $C_{0-1}$alkylene$C_{3-4}$cycloalkyl is a fully saturated hydrocarbon ring containing the specified number of carbon atoms. Examples of cycloalkyl groups include the $C_{3-5}$cycloalkyl groups cyclopropyl, cyclobutyl and cyclopentyl.

The term 'alkynyl' as used herein, such as $C_2$alkynyl is an unbranched hydrocarbon chain containing the specified number of carbons (e.g. two carbons), two of which carbon atoms are linked by a carbon-carbon triple bond.

The term 'halo' or 'halogen' as used herein, refers to fluorine, chlorine, bromine or iodine. Particular examples of halo are fluorine and chlorine, especially fluorine.

The term 'haloalkyl' as used herein, such as in $C_{1-2}$haloalkyl or $C_{1-4}$haloalkyl, whether alone or forming part of a larger group such as an Ohaloalkyl group, such as in $OC_{1-2}$haloalkyl or $OC_{1-4}$haloalkyl, is a straight or a branched fully saturated hydrocarbon chain containing the specified number of carbon atoms and at least one halogen atom, such as fluoro or chloro, especially fluoro. An example of haloalkyl is $CF_3$. Examples of Ohaloalkyl include $OCF_3$, $OCHF_2$ and $OCH_2CF_3$.

The term '6-membered aryl' as used herein refers to a phenyl ring.

The term '6-membered heteroaryl' as used herein refers to 6-membered aromatic rings containing at least one heteroatom (e.g. nitrogen). Exemplary 6-membered heteroaryls include one nitrogen atom (pyridinyl), two nitrogen atoms (pyridazinyl, pyrimidinyl or pyrazinyl) and three nitrogen atoms (triazinyl).

The phrase 'in the para position relative to the amide' as used herein, such as in relation to the position of Ar2, means that compounds with the following substructure are formed:

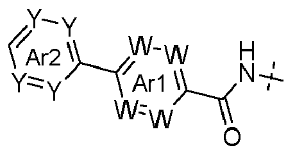

wherein W may be N, CH or $CR_{10}$, and Y may be N, CH or $CR_{12}$ as required by the definitions provided for compounds of formula (I).

The term 'meta' as used herein, such as when used in respect of defining the position of $R_{12}$ on Ar2 is with respect to Ar1 means:

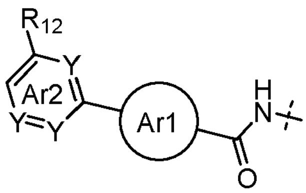

*meta*

In one embodiment of the invention $R_1$ is $C_{1-4}$alkyl. When $R_1$ is $C_{1-4}$alkyl, $R_1$ is methyl, ethyl, propyl (n-propyl or isopropyl) or butyl (n-butyl, isobutyl, sec-butyl or tert-butyl).

In a second embodiment of the invention $R_1$ is $C_{1-2}$alkylene$OC_{1-2}$alkyl. $R_1$ may be $C_{1}$-alkylene$OC_1$alkyl. $R_1$ may be $C_1$alkylene$OC_2$alkyl. $R_1$ may be $C_2$alkylene$OC_1$alkyl. $R_1$ may be $C_2$alkylene$OC_2$alkyl.

In a third embodiment of the invention $R_1$ is $C_{0-1}$alkylene$C_{3-4}$cycloalkyl which cycloalkyl is optionally substituted by $CH_3$. In some embodiments, $R_1$ is $C_{0-1}$alkylene$C_{3-4}$cycloalkyl. In other embodiments, $R_1$ is $C_{0-1}$alkylene$C_{3-4}$cycloalkyl which cycloalkyl is substituted by $CH_3$. $R_1$ may be $C_{3-4}$cycloalkyl, which cycloalkyl is optionally substituted by $CH_3$. $R_1$ may be $C_1$alkylene$C_{3-4}$cycloalkyl, which cycloalkyl is optionally substituted by $CH_3$. $R_1$ may be $C_{0-1}$alkylene$C_3$cycloalkyl, which cycloalkyl is optionally substituted by $CH_3$. $R_1$ may be $C_{0-1}$alkylene$C_4$cycloalkyl, which cycloalkyl is optionally substituted by $CH_3$. Suitably, where $C_{0-1}$alkylene$C_{3-4}$cycloalkyl is optionally substituted by $CH_3$, the $CH_3$ is at the point of attachment of the $C_{3-4}$cycloalkyl to the $C_{0-1}$alkylene.

Suitably $R_1$ is cyclopropyl.

In one embodiment $R_3$ is H. In a second embodiment $R_3$ is $CH_3$. In a third embodiment, $R_3$ is F. In a fourth embodiment, $R_3$ is Cl.

Suitably, $R_3$ is H.

In one embodiment, $R_4$ is H. In a second embodiment $R_4$ is $C_{1-4}$alkyl, i.e. methyl, ethyl, propyl (n-propyl or isopropyl) or butyl (n-butyl, isobutyl, sec-butyl or tert-butyl). In a third embodiment, $R_4$ is $C_{0-2}$alkylene$C_{3-5}$cycloalkyl, such as $C_{0-2}$alkylene$C_3$cycloalkyl, $C_{0-2}$alkylene$C_4$cycloalkyl, $C_{0-2}$alkylene$C_5$cycloalkyl, $C_0$alkylene$C_{3-5}$cycloalkyl, $C_1$alkylene$C_{3-5}$cycloalkyl and $C_2$alkylene$C_{3-5}$cycloalkyl. In a fourth embodiment $R_4$ is $C_{1-3}$alkyleneO$C_{1-3}$alkyl, in particular $C_{1-2}$alkyleneO$C_{1-2}$alkyl such as $C_1$alkyleneO$C_1$alkyl, $C_2$alkyleneO$C_1$alkyl, $C_1$alkyleneO$C_2$alkyl or $C_2$alkyleneO$C_2$alkyl. In a fifth embodiment $R_4$ is $C_{1-4}$alkylOH such as $C_1$alkylOH, $C_2$alkylOH, $C_3$alkylOH or $C_4$alkylOH wherein $C_{1-4}$alkyl is methyl, ethyl, propyl (n-propyl or isopropyl) and butyl (n-butyl, isobutyl, sec-butyl or tert-butyl). In a sixth embodiment, $R_4$ is $C_{1-4}$haloalkyl such as $C_1$haloalkyl (e.g. $CF_3$), $C_2$haloalkyl (e.g. $CH_2CF_3$), C3haloalkyl (e.g. $CH_2CH_2CF_3$), $C_4$haloalkyl (e.g. $CH_2CH_2CH_2CF_3$).

Suitably $R_4$ is H, $CH_3$ or ethyl, in particular $CH_3$ or ethyl.

In one embodiment, $R_5$ is H. In a second embodiment $R_5$ is $C_{1-4}$alkyl, i.e. methyl, ethyl, propyl (n-propyl or isopropyl) or butyl (n-butyl, isobutyl, sec-butyl or tert-butyl). In a third embodiment, $R_5$ is $C_{0-2}$alkylene$C_{3-5}$cycloalkyl, such as $C_{0-2}$alkylene$C_3$cycloalkyl, $C_{0-2}$alkylene$C_4$cycloalkyl, $C_{0-2}$alkylene$C_5$cycloalkyl, $C_0$alkylene$C_{3-5}$cycloalkyl, $C_1$alkylene$C_{3-5}$cycloalkyl and $C_2$alkylene$C_{3-5}$cycloalkyl. In a fourth embodiment $R_5$ is $C_{1-3}$alkyleneO$C_{1-3}$alkyl, in particular $C_{1-2}$alkyleneO$C_{1-2}$alkyl such as $C_1$alkyleneO$C_1$alkyl, $C_2$alkyleneO$C_1$alkyl, $C_1$alkyleneO$C_2$alkyl or $C_2$alkyleneO$C_2$alkyl. In a fifth embodiment $R_5$ is $C_{1-4}$alkylOH such as $C_1$alkylOH, $C_2$alkylOH, $C_3$alkylOH or $C_4$alkylOH wherein $C_{1-4}$alkyl is methyl, ethyl, propyl (n-propyl or isopropyl) and butyl (n-butyl, isobutyl, sec-butyl or tert-butyl). In a sixth embodiment, $R_5$ is $C_{1-4}$haloalkyl such as $C_1$haloalkyl (e.g. $CF_3$), $C_2$haloalkyl (e.g. $CH_2CF_3$), C3haloalkyl (e.g. $CH_2CH_2CF_3$), $C_4$haloalkyl (e.g. $CH_2CH_2CH_2CF_3$).

Suitably $R_5$ is H, $CH_3$ or ethyl, in particular $CH_3$ or ethyl.

Suitably $R_4$ is H, $CH_3$ or ethyl and $R_5$ is H, $CH_3$ or ethyl, in particular $R_4$ is $CH_3$ or ethyl and $R_5$ is $CH_3$ or ethyl. For example, $R_4$ and $R_5$ are H, $R_4$ and $R_5$ are methyl or $R_4$ and $R_5$ are ethyl.

In one embodiment Ar1 is a 6-membered aryl, i.e. phenyl. In a second embodiment Ar1 is a 6-membered heteroaryl, in particular containing one nitrogen atom (pyridyl) or two nitrogen atoms (pyridazinyl, pyrimidinyl or pyrazinyl).

In particular Ar1 is phenyl or 2-pyridyl, such as phenyl. The position numbering for Ar1 is in respect of the amide, with the carbon at the point of attachment designated position 1 and other numbers providing the relative location of the nitrogen atoms, for example:

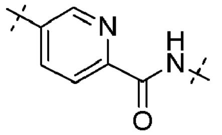

2-pyridyl

In one embodiment $R_{10}$ is H. In a second embodiment $R_{10}$ is halo, for example fluoro or chloro. In a third embodiment $R_{10}$ is $C_{1-3}$alkyl, i.e. $CH_3$, ethyl or propyl (e.g. n-propyl or *iso*-propyl). In a fourth embodiment $R_{10}$ is O$C_{1-2}$alkyl, such as $OCH_3$ or ethoxy. In a fifth embodiment, $R_{10}$ is $C_{1-2}$haloalkyl, such as $CF_3$ or $CH_2CF_3$. In a sixth embodiment $R_{10}$ is O$C_{1-2}$haloalkyl, such as $OCF_3$. In a seventh embodiment $R_{10}$ is CN.

Suitably $R_{10}$ is H, fluoro, $OCH_3$, $CH_3$ or $CF_3$, in particular H or fluoro, especially H.

Suitably $R_{10}$ is attached at the ortho position of Ar1 relative to the amide (i.e. proximal to the amide).

In one embodiment Ar2 is a 6-membered aryl, i.e. phenyl. In a second embodiment Ar2 is a 6-membered heteroaryl, in particular containing one nitrogen atom (pyridyl) or two nitrogen atoms (pyridazinyl, pyrimidinyl or pyrazinyl).

The position numbering for Ar2 is in respect of the point of attachment to Ar1, for example:

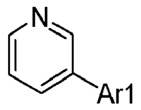 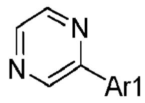

3-pyridyl 2,5-pyrazinyl.

In particular Ar2 is 3-pyridyl or 2,5-pyrazinyl, especially 2,5-pyrazinyl.

In one embodiment $R_{12}$ is H. In a second embodiment $R_{12}$ is halo, for example fluoro or chloro. In a third embodiment $R_{12}$ is $C_{1-4}$alkyl, i.e. methyl, ethyl, propyl (n-propyl or isopropyl) or butyl (n-butyl, isobutyl, sec-butyl or tert-butyl). In a fourth embodiment, $R_{12}$ is $C_2$alkynyl (i.e. C≡CH). In a fifth embodiment, $R_{12}$ is C(=O)$C_{1-2}$alkyl, such as C(=O)$C_1$alkyl or C(=O)$C_2$alkyl. In a sixth embodiment $R_{12}$ is $OC_{0-2}$alkylene$C_{3-5}$cycloalkyl, such as $OC_{3-5}$cycloalkyl (e.g. cyclopropoxy or cyclobutoxy), $OC_1$alkylene$C_{3-5}$cycloalkyl or $OC_2$alkylene$C_{3-5}$cycloalkyl. In a seventh embodiment $R_{12}$ is $OC_{1-4}$alkyl, such as $OCH_3$, ethoxy, iso-propoxy or n-propoxy. In an eighth embodiment, $R_{12}$ is $C_{1-3}$alkylene$OC_{1-3}$alkyl in particular $C_{1-2}$alkylene$OC_{1-2}$alkyl such as $C_1$alkylene$OC_1$alkyl, $C_2$alkylene$OC_1$alkyl, $C_1$alkylene$OC_2$alkyl or $C_2$alkylene$OC_2$alkyl. In a ninth embodiment $R_{12}$ is $C_{1-4}$haloalkyl, such as $CF_3$. In a tenth embodiment $R_{12}$ is $OC_{1-4}$haloalkyl, such as $OCF_3$, $OCHF_2$ or $OCH_2CF_3$. In an eleventh embodiment $R_{12}$ is CN.

$R_{12}$ is suitably H, fluoro, chloro, $CH_3$, Et, $OCH_3$, OEt, OiPr, $CF_3$ or $OCH_2CF_3$. In particular, $R_{12}$ is fluoro, chloro, $CH_3$, $OCH_3$, OEt, OiPr or $CF_3$, such as chloro, OEt or $CF_3$.

$R_{12}$ is attached at the meta position of Ar2.

Throughout the specification Ar1 and Ar2 may be depicted as follows:

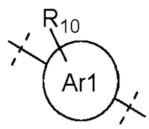 ; 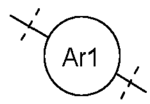 ; 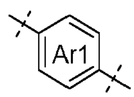 ; Ar1

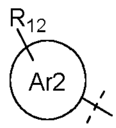 ; 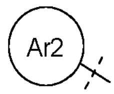 ; 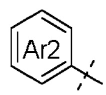 ; Ar2

All depictions with respect to Ar1 are equivalent and all depictions with respect to Ar2 are equivalent, unless the context requires otherwise, depictions of Ar1 and Ar2 should not be taken to exclude the presence of heteroatoms or substitutions.

The present invention provides the compounds described in any one of Examples R1 to R71.

The present invention provides the following compounds:

N-((2-(cyclopropanesulfonamido)thiazol-4-yl)methyl)-5-phenylpicolinamide;

N-((2-(cyclopropanesulfonamido)thiazol-4-yl)methyl)-4-(pyridin-3-yl)benzamide;

N-(1-(2-(cyclopropanesulfonamido)thiazol-4-yl)propyl)-4-(5-(trifluoromethyl)pyridin-3-yl)benzamide;

N-(1-(2-(cyclopropanesulfonamido)thiazol-4-yl)propyl)-4-(5-(trifluoromethyl)pyridin-3-yl)benzamide (R enantiomer);

N-(1-(2-(cyclopropanesulfonamido)thiazol-4-yl)propyl)-4-(5-(trifluoromethyl)pyridin-3-yl)benzamide (S enantiomer);

N-(1-(2-(cyclopropanesulfonamido)thiazol-4-yl)propyl)-4-(6-(trifluoromethyl)pyrazin-2-yl)benzamide;

N-(2-(2-(cyclopropanesulfonamido)thiazol-4-yl)propan-2-yl)-4-(6-ethoxypyrazin-2-yl)-2-fluorobenzamide;

N-(2-(2-(cyclopropanesulfonamido)thiazol-4-yl)propan-2-yl)-4-(6-ethoxypyrazin-2-yl)-2-methoxybenzamide;

N-((2-(cyclopropanesulfonamido)thiazol-4-yl)methyl)-[1,1'-biphenyl]-4-carboxamide;

N-((2-(cyclopropanesulfonamido)thiazol-4-yl)methyl)-2-fluoro-4-(6-(trifluoromethyl)pyrazin-2-yl)benzamide;

N-((2-(cyclopropanesulfonamido)thiazol-4-yl)methyl)-4-(6-ethoxypyrazin-2-yl)-2-fluorobenzamide;

N-((2-(cyclopropanesulfonamido)thiazol-4-yl)methyl)-4-(6-(trifluoromethyl)pyrazin-2-yl)benzamide;

N-((2-(cyclopropanesulfonamido)thiazol-4-yl)methyl)-4-(6-isopropoxypyrazin-2-yl)benzamide;

N-((2-(cyclopropanesulfonamido)thiazol-4-yl)methyl)-4-(6-ethoxypyrazin-2-yl)benzamide;

N-(3-(2-(cyclopropanesulfonamido)thiazol-4-yl)pentan-3-yl)-4-(5-(trifluoromethyl)pyridin-3-yl)benzamide;

N-(3-(2-(cyclopropanesulfonamido)thiazol-4-yl)pentan-3-yl)-4-(5-fluoropyridin-3-yl)benzamide;

N-(3-(2-(cyclopropanesulfonamido)thiazol-4-yl)pentan-3-yl)-4-(5-methylpyridin-3-yl)benzamide;

N-(3-(2-(cyclopropanesulfonamido)thiazol-4-yl)pentan-3-yl)-4-(pyridin-3-yl)benzamide;

N-(3-(2-(cyclopropanesulfonamido)thiazol-4-yl)pentan-3-yl)-4-(6-(trifluoromethyl)pyrazin-2-yl)benzamide;

4-(6-chloropyrazin-2-yl)-N-(3-(2-(cyclopropanesulfonamido)thiazol-4-yl)pentan-3-yl)benzamide;

N-(3-(2-(cyclopropanesulfonamido)thiazol-4-yl)pentan-3-yl)-4-(6-methylpyrazin-2-yl)benzamide;

N-(3-(2-(cyclopropanesulfonamido)thiazol-4-yl)pentan-3-yl)-4-(pyrazin-2-yl)benzamide;

N-(2-(2-(cyclopropanesulfonamido)thiazol-4-yl)propan-2-yl)-5-(6-ethoxypyrazin-2-yl)-3-fluoropicolinamide;

N-(2-(2-(cyclopropanesulfonamido)thiazol-4-yl)propan-2-yl)-5-(6-(trifluoromethyl)pyrazin-2-yl)picolinamide;

5-(6-chloropyrazin-2-yl)-N-(2-(2-(cyclopropanesulfonamido)thiazol-4-yl)propan-2-yl)picolinamide;

N-(2-(2-(cyclopropanesulfonamido)thiazol-4-yl)propan-2-yl)-5-(6-ethoxypyrazin-2-yl)picolinamide;

N-(2-(2-(cyclopropanesulfonamido)thiazol-4-yl)propan-2-yl)-[2,2'-bipyridine]-5-carboxamide;

4-(5-chloropyridin-3-yl)-N-(2-(2-(cyclopropanesulfonamido)thiazol-4-yl)propan-2-yl)benzamide;

N-(2-(2-(cyclopropanesulfonamido)thiazol-4-yl)propan-2-yl)-2-fluoro-4-(5-(trifluoromethyl)pyridin-3-yl)benzamide;

4-(5-chloropyridin-3-yl)-N-(2-(2-(cyclopropanesulfonamido)thiazol-4-yl)propan-2-yl)-2-fluorobenzamide;

N-(2-(2-(cyclopropanesulfonamido)thiazol-4-yl)propan-2-yl)-2-fluoro-4-(5-fluoropyridin-3-yl)benzamide;

N-(2-(2-(cyclopropanesulfonamido)thiazol-4-yl)propan-2-yl)-2-methoxy-4-(5-(trifluoromethyl)pyridin-3-yl)benzamide;

4-(5-acetylpyridin-3-yl)-N-(2-(2-(cyclopropanesulfonamido)thiazol-4-yl)propan-2-yl)benzamide;

N-(2-(2-(cyclopropanesulfonamido)thiazol-4-yl)propan-2-yl)-4-(5-(trifluoromethyl)pyridin-3-yl)benzamide;

N-(2-(2-(cyclopropanesulfonamido)thiazol-4-yl)propan-2-yl)-4-(5-fluoropyridin-3-yl)benzamide;

N-(2-(2-(cyclopropanesulfonamido)thiazol-4-yl)propan-2-yl)-4-(5-methylpyridin-3-yl)benzamide;

N-(2-(2-(cyclopropanesulfonamido)thiazol-4-yl)propan-2-yl)-4-(5-methoxypyridin-3-yl)benzamide;

N-(2-(2-(cyclopropanesulfonamido)thiazol-4-yl)propan-2-yl)-4-(pyridin-3-yl)benzamide;

N-(2-(2-(cyclopropanesulfonamido)thiazol-4-yl)propan-2-yl)-3'-(trifluoromethyl)-[1,1'-biphenyl]-4-carboxamide;

N-(2-(2-(cyclopropanesulfonamido)thiazol-4-yl)propan-2-yl)-4-(6-ethylpyrazin-2-yl)-2-fluorobenzamide;

N-(2-(2-(cyclopropanesulfonamido)thiazol-4-yl)propan-2-yl)-2-fluoro-4-(6-(trifluoromethyl)pyrazin-2-yl)benzamide;

N-(2-(2-(cyclopropanesulfonamido)thiazol-4-yl)propan-2-yl)-2-fluoro-4-(6-isopropoxypyrazin-2-yl)benzamide;

N-(2-(2-(cyclopropanesulfonamido)thiazol-4-yl)propan-2-yl)-2-fluoro-4-(6-(2,2,2-trifluoroethoxy)pyrazin-2-yl)benzamide;

N-(2-(2-(cyclopropanesulfonamido)thiazol-4-yl)propan-2-yl)-2-methyl-4-(6-(trifluoromethyl)pyrazin-2-yl)benzamide;

N-(2-(2-(cyclopropanesulfonamido)thiazol-4-yl)propan-2-yl)-4-(6-ethoxypyrazin-2-yl)-2-methylbenzamide;

N-(2-(2-(cyclopropanesulfonamido)thiazol-4-yl)propan-2-yl)-4-(6-ethoxypyrazin-2-yl)-2-(trifluoromethyl)benzamide;

N-(2-(2-(cyclopropanesulfonamido)thiazol-4-yl)propan-2-yl)-2-methoxy-4-(6-(trifluoromethyl)pyrazin-2-yl)benzamide;

4-(6-chloropyrazin-2-yl)-N-(2-(2-(cyclopropanesulfonamido)thiazol-4-yl)propan-2-yl)-2-methoxybenzamide;

4-(6-cyanopyrazin-2-yl)-N-(2-(2-(cyclopropanesulfonamido)thiazol-4-yl)propan-2-yl)-2-methoxybenzamide;

N-(2-(2-(cyclopropanesulfonamido)thiazol-4-yl)propan-2-yl)-4-(6-(trifluoromethyl)pyrazin-2-yl)benzamide;

4-(6-chloropyrazin-2-yl)-N-(2-(2-(cyclopropanesulfonamido)thiazol-4-yl)propan-2-yl)benzamide;

N-(2-(2-(cyclopropanesulfonamido)thiazol-4-yl)propan-2-yl)-4-(6-methylpyrazin-2-yl)benzamide;

N-(2-(2-(cyclopropanesulfonamido)thiazol-4-yl)propan-2-yl)-4-(6-methoxypyrazin-2-yl)benzamide;

N-(2-(2-(cyclopropanesulfonamido)thiazol-4-yl)propan-2-yl)-4-(6-ethoxypyrazin-2-yl)benzamide;

N-(2-(2-(cyclopropanesulfonamido)thiazol-4-yl)propan-2-yl)-4-(6-isopropoxypyrazin-2-yl)benzamide;

N-(2-(2-(cyclopropanesulfonamido)thiazol-4-yl)propan-2-yl)-4-(6-(2,2,2-trifluoroethoxy)pyrazin-2-yl)benzamide;

N-(2-(2-(cyclopropanesulfonamido)thiazol-4-yl)propan-2-yl)-4-(pyrazin-2-yl)benzamide;

N-(1-(2-(cyclopropanesulfonamido)thiazol-4-yl)propyl)-4-(5-fluoropyridin-3-yl)benzamide;

N-(1-(2-(cyclopropanesulfonamido)thiazol-4-yl)propyl)-4-(5-methylpyridin-3-yl)benzamide;

N-(1-(2-(cyclopropanesulfonamido)thiazol-4-yl)propyl)-4-(pyridin-3-yl)benzamide;

N-(1-(2-(cyclopropanesulfonamido)thiazol-4-yl)propyl)-4-(6-ethoxypyrazin-2-yl)-2-fluorobenzamide;

N-(1-(2-(cyclopropanesulfonamido)thiazol-4-yl)propyl)-4-(6-ethoxypyrazin-2-yl)-2-fluoro-N-methylbenzamide;

N-(1-(2-(cyclopropanesulfonamido)thiazol-4-yl)propyl)-2-fluoro-4-(6-isopropoxypyrazin-2-yl)benzamide;

4-(6-chloropyrazin-2-yl)-N-(1-(2-(cyclopropanesulfonamido)thiazol-4-yl)propyl)benzamide;

N-(1-(2-(cyclopropanesulfonamido)thiazol-4-yl)propyl)-4-(6-methylpyrazin-2-yl)benzamide;

N-(1-(2-(cyclopropanesulfonamido)thiazol-4-yl)propyl)-4-(pyrazin-2-yl)benzamide;

N-(1-(2-(cyclopropanesulfonamido)thiazol-4-yl)propyl)-4-(5-fluoropyridin-3-yl)benzamide (R enantiomer);

N-(1-(2-(cyclopropanesulfonamido)thiazol-4-yl)propyl)-4-(5-fluoropyridin-3-yl)benzamide (S enantiomer);

N-(1-(2-(cyclopropanesulfonamido)thiazol-4-yl)propyl)-4-(6-ethoxypyrazin-2-yl)-2-fluorobenzamide (R enantiomer); and

N-(1-(2-(cyclopropanesulfonamido)thiazol-4-yl)propyl)-4-(6-ethoxypyrazin-2-yl)-2-fluorobenzamide (S enantiomer);

or a salt and/or solvate thereof and/or derivative thereof.

The compounds of the invention may be provided in the form of a pharmaceutically acceptable salt and/or solvate thereof and/or derivative thereof. In particular, the compound of formula (I) may be provided in the form of a pharmaceutically acceptable salt and/or solvate, such as a pharmaceutically acceptable salt.

Compounds of the invention of particular interest are those demonstrating an $IC_{50}$ of 1uM or lower, especially 100nM or lower, in respect of CTPS1 enzyme, using the methods of the examples (or comparable methods).

It will be appreciated that for use in medicine the salts of the compounds of formula (I) should be pharmaceutically acceptable. Non-pharmaceutically acceptable salts of the compounds of formula (I) may be of use in other contexts. Suitable pharmaceutically acceptable salts will be apparent to those skilled in the art. Pharmaceutically acceptable salts include those described by Berge et al. (1977). Such pharmaceutically acceptable salts include acid and base addition salts. Pharmaceutically acceptable acid additional salts may be formed with inorganic acids e.g. hydrochloric, hydrobromic, sulfuric, nitric or phosphoric acid and organic acids e.g. succinic, maleic, acetic, fumaric, citric, tartaric, benzoic, p-toluenesulfonic, methanesulfonic or naphthalenesulfonic acid. Other salts e.g. oxalates or formates, may be used, for example in the isolation of compounds of formula (I) and are included within the scope of this invention.

Certain of the compounds of formula (I) may form acid or base addition salts with one or more equivalents of the acid or base. The present invention includes within its scope all possible stoichiometric and non-stoichiometric forms.

The compounds of formula (I) may be prepared in crystalline or non-crystalline form and, if crystalline, may optionally be solvated, e.g. as the hydrate. This invention includes within its scope stoichiometric solvates (e.g. hydrates) as well as compounds containing variable amounts of solvent (e.g. water).

It will be understood that the invention includes pharmaceutically acceptable derivatives of compounds of formula (I) and that these are included within the scope of the invention.

As used herein "pharmaceutically acceptable derivative" includes any pharmaceutically acceptable prodrug such as an ester or salt of such ester of a compound of formula (I) which, upon administration to the recipient is capable of providing (directly or indirectly) a compound of formula (I) or an active metabolite or residue thereof.

It is to be understood that the present invention encompasses all isomers of formula (I) and their pharmaceutically acceptable derivatives, including all geometric, tautomeric and optical forms, and mixtures thereof (e.g. racemic mixtures). Where additional chiral centres are present in compounds of formula (I), the present invention includes within its scope all possible diastereoisomers, including mixtures thereof. The different isomeric forms may be separated or resolved one from the other by conventional methods, or any given isomer may be obtained by conventional synthetic methods or by stereospecific or asymmetric syntheses.

The present disclosure includes all isotopic forms of the compounds of the invention provided herein, whether in a form (i) wherein all atoms of a given atomic number have a mass number (or mixture of mass numbers) which predominates in nature (referred to herein as the "natural isotopic form") or (ii) wherein one or more atoms are replaced by atoms having the same atomic number, but a mass number different from the mass number of atoms which predominates in nature (referred to herein as an "unnatural variant isotopic form"). It is understood that an atom may naturally exist as a mixture of mass numbers. The term "unnatural variant isotopic form" also includes embodiments in which the proportion of an atom of given atomic number having a mass number found less commonly in nature (referred to herein as an "uncommon isotope") has been increased relative to that which is naturally occurring e.g. to the level of >20%, >50%, >75%, >90%, >95% or > 99% by number of the atoms of that atomic number (the latter embodiment referred to as an "isotopically enriched variant form"). The term "unnatural variant isotopic form" also includes embodiments in which the proportion of an uncommon isotope has been reduced relative to that which is naturally occurring. Isotopic forms may include radioactive forms (i.e. they incorporate radioisotopes) and non-radioactive forms. Radioactive forms will typically be isotopically enriched variant forms.

An unnatural variant isotopic form of a compound may thus contain one or more artificial or uncommon isotopes such as deuterium ($^2H$ or D), carbon-11 ($^{11}C$), carbon-13 ($^{13}C$), carbon-14 ($^{14}C$), nitrogen-13 ($^{13}N$), nitrogen-15 ($^{15}N$), oxygen-15 ($^{15}O$), oxygen-17 ($^{17}O$), oxygen-18 ($^{18}O$), phosphorus-32 ($^{32}P$), sulphur-35 ($^{35}S$), chlorine-36 ($^{36}Cl$), chlorine-

37 ($^{37}Cl$), fluorine-18 ($^{18}F$) iodine-123 ($^{123}I$), iodine-125 ($^{125}I$) in one or more atoms or may contain an increased proportion of said isotopes as compared with the proportion that predominates in nature in one or more atoms.

Unnatural variant isotopic forms comprising radioisotopes may, for example, be used for drug and/or substrate tissue distribution studies. The radioactive isotopes tritium, i.e. $^{3}H$, and carbon-14, i.e. $^{14}C$, are particularly useful for this purpose in view of their ease of incorporation and ready means of detection. Unnatural variant isotopic forms which incorporate deuterium i.e $^{2}H$ or D may afford certain therapeutic advantages resulting from greater metabolic stability, for example, increased *in vivo* half-life or reduced dosage requirements, and hence may be preferred in some circumstances. Further, unnatural variant isotopic forms may be prepared which incorporate positron emitting isotopes, such as $^{11}C$, $^{18}F$, $^{15}O$ and $^{13}N$, and would be useful in Positron Emission Topography (PET) studies for examining substrate receptor occupancy.

In one embodiment, the compounds of the invention are provided in a natural isotopic form.

In one embodiment, the compounds of the invention are provided in an unnatural variant isotopic form. In a specific embodiment, the unnatural variant isotopic form is a form in which deuterium (i.e. $^{2}H$ or D) is incorporated where hydrogen is specified in the chemical structure in one or more atoms of a compound of the invention. In one embodiment, the atoms of the compounds of the invention are in an isotopic form which is not radioactive. In one embodiment, one or more atoms of the compounds of the invention are in an isotopic form which is radioactive. Suitably radioactive isotopes are stable isotopes. Suitably the unnatural variant isotopic form is a pharmaceutically acceptable form.

In one embodiment, a compound of the invention is provided whereby a single atom of the compound exists in an unnatural variant isotopic form. In another embodiment, a compound of the invention is provided whereby two or more atoms exist in an unnatural variant isotopic form.

Unnatural isotopic variant forms can generally be prepared by conventional techniques known to those skilled in the art or by processes described herein e.g. processes analogous to those described in the accompanying Examples for preparing natural isotopic forms. Thus, unnatural isotopic variant forms could be prepared by using appropriate isotopically variant (or labelled) reagents in place of the normal reagents employed in the Examples. Since the compounds of formula (I) are intended for use in pharmaceutical compositions it will readily be understood that they are each preferably provided in substantially pure form, for example at least 60% pure, more suitably at least 75% pure and preferably at least 85%, especially at least 98% pure (% are on a weight for weight basis). Impure preparations of the compounds may be used for preparing the more pure forms used in the pharmaceutical compositions.

In general, the compounds of formula (I) may be made according to the organic synthesis techniques known to those skilled in this field, as well as by the representative methods set forth below, those in the Examples, and modifications thereof.

**General Routes**

Generic routes by which compound examples of the invention may be conveniently prepared are summarised below.

**Scheme 1: Synthesis of dialkyl amides of formula (I)**

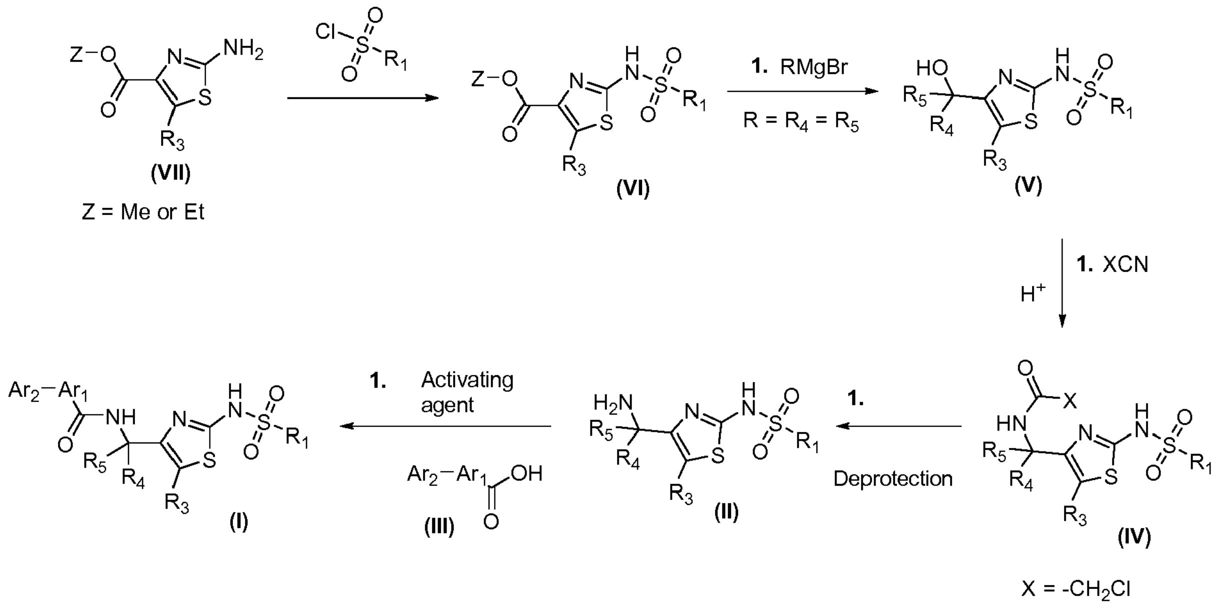


The thiazol-4-yl(propan-2-yl)benzamide derivatives of formula (**I**) in which $R_1$, $R_3$, $R_4$, $R_5$, $Ar_1$ and $Ar_2$ are defined above may be prepared as shown in Scheme 1 by sulfonation of the commercial amine such as **(VII)** followed by double Grignard addition to ethyl 2-(cyclopropanesulfonamido)thiazole-4-carboxylate **(VI)** in an aprotic solvent such as THF to form intermediates of formula (V). A Ritter type reaction may then be undertaken using an alkylnitrile, such as 2-chloroacetonitrile in the presence of an acid such as $H_2SO_4$. This intermediate of formula **(IV)** can be deprotected by reaction with thiourea in a protic solvent such as ethanol in the presence of acetic acid and heated under reflux to yield the free benzylamine derivative (II). A carboxylic acid precursor **(III)** (commercially available or prepared in two steps, as in Scheme 4) is reacted with an activating agent such as HATU, T3P or Ghosez's reagent (1-chloro-N,N,2-trimethylprop-1-en-1-amine), to generate a reactive, electrophilic carboxylic acid derivative, followed by subsequent reaction with benzylamine of formula (**II**), to yield the desired amide derivative of general formula (**I**).

**Scheme 2: Synthesis of des ($R_4$ and $R_5$ are H) and monosubstituted ($R_5$ is H) amides of formula (I)**

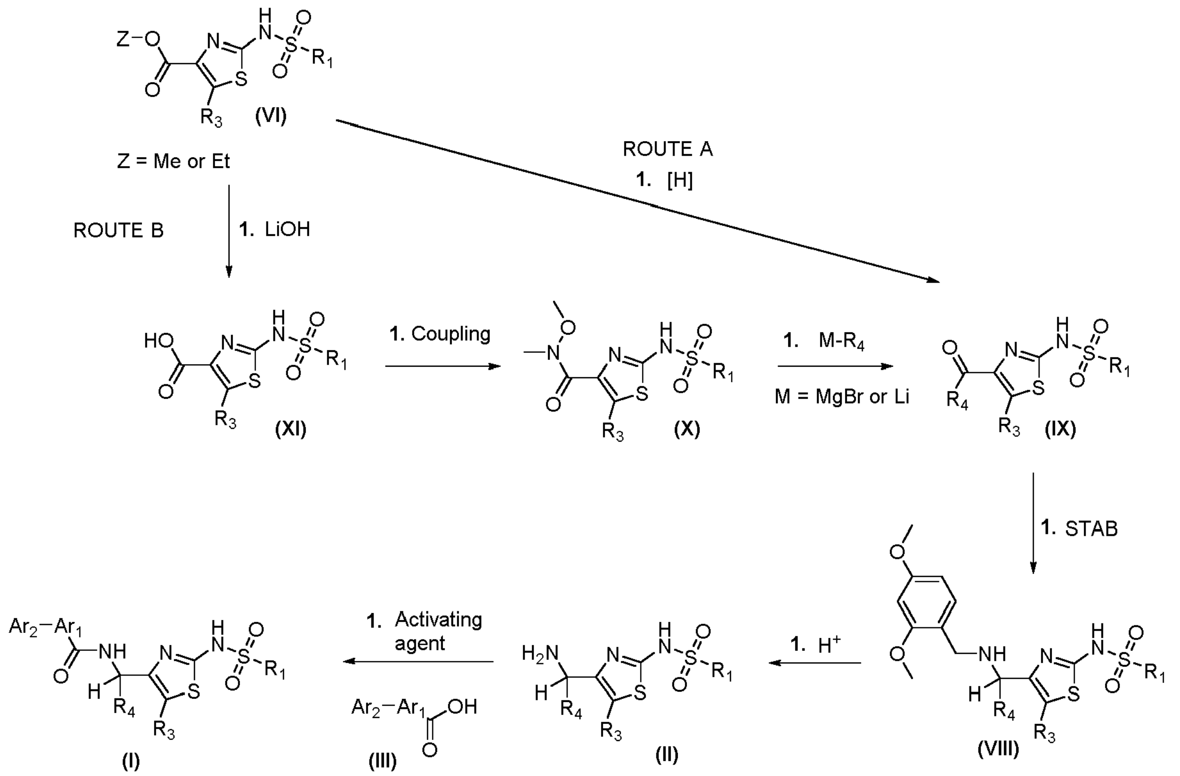


The thiazol-4-yl(propan-2-yl)benzamide derivatives of formula **(I)** in which $R_1$, $R_3$, $R_4$, $Ar_1$ and $Ar_2$ are defined above may be prepared by two different routes as shown in Scheme 2. The two routes then converge at compounds of general formula **(IX)** where they are then taken on to the final analogues by a two-step process.

ROUTE A: Reduction of the ester of general formula **(VI)** using a reducing agent such as DIBAL-H yields the aldehyde of general formula **(IX)** ($R_4$ is H). It will be understood by persons skilled in the art that such reactions need to be carefully controlled to avoid over-reduction.

ROUTE B: The alkyl ester of formula **(VI)** may be conveniently hydrolysed by exposure to a suitable inorganic base, for example lithium hydroxide, in an aqueous mixture of aprotic and protic solvents, such as THF:methanol:water. The carboxylic acid derivative **(XI)** can then be converted to the Weinreb amide by employing a coupling agent, for example, HATU in the presence of a suitable base, such as DIPEA in a solvent such as DMF. The Weinreb amide derivative **(X)** can then be exposed to a nucleophile such as EtMgBr in an aprotic solvent such as THF to yield the corresponding ketone of the general formula **(IX).**

A reductive amination may be undertaken using (2,4-dimethoxyphenyl)methanamine and a reducing agent such as sodium triacetoxyborohydride. This reaction can be carried out on the ketone of general formula **(IX)** (when $R_4$ is other than H) or the aldehyde of general formula **(IX)** (when $R_4$ is H), if the des-alkyl linker is desired. The intermediate of formula **(VIII)** may then be deprotected using a strong acid, such as TFA. Such reactions may be heated to 70 °C to yield the free benzylamine derivative **(II).** An amide coupling can then be undertaken as in Scheme 1.

## Scheme 3: Synthesis of compounds of formula (I) via Suzuki coupling

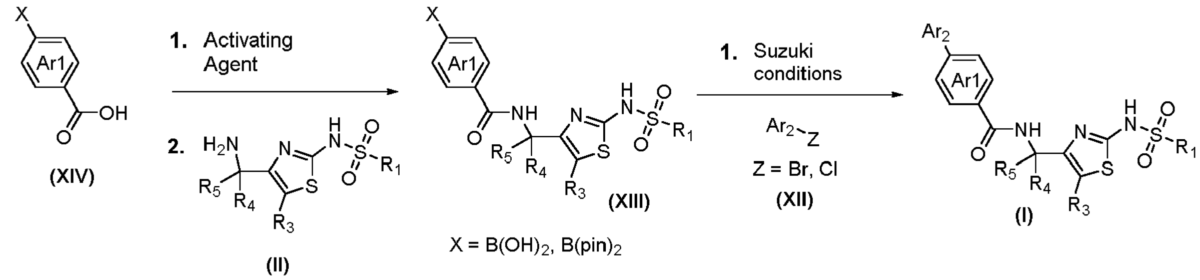

(XIV) (II) (XIII) (XII) (I)

Compounds of formula **(XIII)** may be obtained by a general process as shown in Scheme 3 whereby a carboxylic acid precursor **(XIV)** is reacted with an activating agent such as HATU, T3P or Ghosez's reagent, to generate a reactive, electrophilic carboxylic acid derivative, followed by subsequent reaction with an amine of formula **(II).** Intermediates of formula **(XIII)** are then converted to a compound of general formula **(I)** by coupling under Suzuki conditions with an aromatic halide of general formula **(XII),** of which X is defined in Scheme 3 and represents a dihydroxyboryl or dialkyloxyboryl group, such as a 4,4,5,5-tetramethyl-1,3,3,2-dioxaborolan-2-yl group. The couplings according to the Suzuki method are performed, for example, by heating in the presence of a catalyst such as bis(diphenylphosphino)ferrocene]dichloropalladium(II).$CH_2Cl_2$ adduct and an inorganic base such as potassium carbonate in a solvent mixture of dioxane and water under an inert atmosphere such as a nitrogen atmosphere. It will be understood by persons skilled in the art that many catalysts and conditions can be employed for such couplings.

## Scheme 4: Synthesis of carboxylic derivative (III)

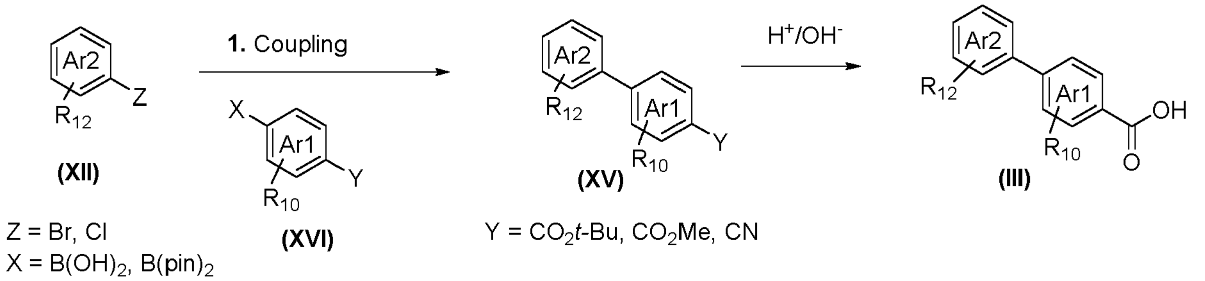

(XII) (XVI) (XV) (III)

Intermediates of formula **(III)** where $Ar_2$ is an unsubstituted or substituted 2-pyrazine ring or 3-pyridyl ring, may be synthesised as shown in Scheme 4 by coupling under Suzuki conditions of an aromatic halide of general formula **(XII),** of which $R_{10}$ and $R_{12}$ are defined above and Z represents a halide such as Br or Cl, to a boronate of general formula **(XVI)** where X denotes a dihydroxyboryl or dialkyloxyboryl group, such as a 4,4,5,5-tetramethyl-1,3,3,2-dioxaborolan-2-yl group. The couplings according to the Suzuki method are performed, for example, by heating in the presence of a catalyst such as [1,1'-bis(diphenylphosphino)ferrocene]dichloropalladium(II).$CH_2Cl_2$ adduct and an inorganic base such as cesium carbonate in a solvent mixture of dioxane and water under an inert atmosphere such as a nitrogen atmosphere. The carboxylic acids of general formula **(III)** are obtained by either deprotection of the t-butyl ester using a strong acid, such as TFA in a solvent of $CH_2Cl_2$, hydrolysis of the methyl ester using an alkali metal hydroxide such as NaOH in a solvent mixture such as THF/MeOH or hydrolysis of the nitrile using a strong acid such as concentrated HCl.

### Intermediates of the Invention

The present invention also relates to novel intermediates in the synthesis of compounds of formula (I) such as compounds of formula **(II)-(XVI).** Particular intermediates of interest are those of the following general formulae, wherein the variable groups and associated preferences are as defined previously for compounds of formula (I):

- a compound of formula (II):

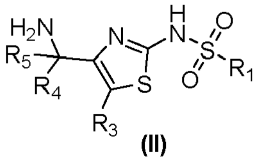

(II) ;

- a compound of formula (III):

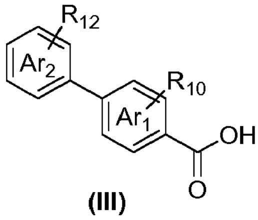

(III) ;

and

- a compound of formula (VIII):

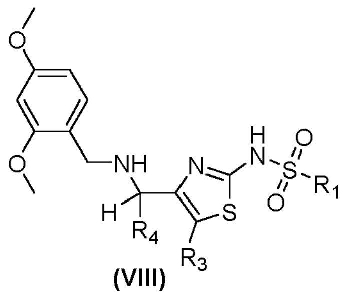

(VIII) .

Included as an aspect of the invention are all novel intermediates described in the examples, including:

- Intermediates INTE1 to INTE20; and
- Intermediates INTF1 to INTF53.

**Therapeutic Methods**

Compounds of formula (I) of the present invention have utility as inhibitors of CTPS1.

Suitably, the compounds of formula (I) of the present invention are selective for CTPS1 over CTPS2.

Therefore, the invention also provides a compound of formula (I), or a pharmaceutically acceptable salt and/or solvate (e.g. salt) and/or derivative thereof, for use as a medicament, in particular in the treatment or prophylaxis of a disease or disorder wherein an inhibitor of CTPS1 is beneficial, for example those diseases and disorders mentioned herein below.

The invention provides a method for the treatment or prophylaxis of a disease or disorder wherein an inhibitor of CTPS1 is beneficial, for example those diseases and disorders mentioned herein below, which comprises administering to a subject in need thereof an effective amount of a compound of formula (I) or a pharmaceutically acceptable salt and/or solvate (e.g. salt) and/or derivative thereof.

The invention also provides the use of a compound of formula (I), or a pharmaceutically acceptable salt and/or solvate thereof (e.g. salt) and/or derivative, in the manufacture of a medicament for the treatment or prophylaxis of a disease or disorder wherein an inhibitor of CTPS1 is beneficial, for example those diseases and disorders mentioned herein below.

More suitably, the disease or disorder wherein an inhibitor of CTPS1 is beneficial is a disease or disorder wherein a reduction in T-cell and/or B-cell proliferation would be beneficial.

The invention also provides a compound of formula (I), or a pharmaceutically acceptable salt and/or solvate

(e.g. salt) and/or derivative thereof, for use in the inhibition of CTPS1 in a subject.

The invention provides a method for the inhibition of CTPS1 in a subject, which comprises administering to the subject an effective amount of a compound of formula (I) or a pharmaceutically acceptable salt and/or solvate (e.g. salt) and/or derivative thereof.

The invention also provides the use of a compound of formula (I), or a pharmaceutically acceptable salt and/or solvate thereof (e.g. salt) and/or derivative, in the manufacture of a medicament for the inhibition of CTPS1 in a subject.

The invention also provides a compound of formula (I), or a pharmaceutically acceptable salt and/or solvate (e.g. salt) and/or derivative thereof, for use in the reduction of T-cell and/or B-cell proliferation in a subject.

The invention provides a method for the reduction of T-cell and/or B-cell proliferation in a subject, which comprises administering to the subject an effective amount of a compound of formula (I) or a pharmaceutically acceptable salt and/or solvate (e.g. salt) and/or derivative thereof.

The invention also provides the use of a compound of formula (I), or a pharmaceutically acceptable salt and/or solvate thereof (e.g. salt) and/or derivative, in the manufacture of a medicament for the reduction of T-cell and/or B-cell proliferation in a subject.

More suitably, the disease or disorder wherein an inhibitor of CTPS1 is beneficial is a disease or disorder wherein a reduction in T-cell and/or B-cell proliferation would be beneficial.

The term ’treatment’ or ’treating’ as used herein includes the control, mitigation, reduction, or modulation of the disease state or its symptoms.

The term ’prophylaxis’ or ’preventing’ is used herein to mean preventing symptoms of a disease or disorder in a subject or preventing recurrence of symptoms of a disease or disorder in an afflicted subject and is not limited to complete prevention of an affliction.

Suitably, the disease or disorder is selected from rejection of transplanted cells and tissues, Graft-related diseases or disorders, allergies and autoimmune diseases.

In one embodiment the disease or disorder is the rejection of transplanted cells and tissues. The subject may have been transplanted with a graft selected from the group consisting of heart, kidney, lung, liver, pancreas, pancreatic islets, brain tissue, stomach, large intestine, small intestine, cornea, skin, trachea, bone, bone marrow (or any other source of hematopoietic precursor cells and stem cells including hematopoietic cells mobilized from bone marrow into peripheral blood or umbilical cord blood cells), muscle, or bladder. The compounds of the invention may be of use in preventing or suppressing an immune response associated with rejection of a donor tissue, cell, graft or organ transplant in a subject.

In a further embodiment the disease or disorder is a Graft-related disease or disorder. Graft-related diseases or disorders include graft versus host disease (GVHD), such as GVHD associated with bone marrow transplantation, and immune disorders resulting from or associated with rejection of organ, tissue, or cell graft transplantation (e.g., tissue or cell allografts or xenografts), including, e.g., grafts of skin, muscle, neurons, islets, organs, parenchymal cells of the liver, etc, and Host-Versus-Graft-Disease (HVGD). The compounds of the invention may be of use in preventing or suppressing acute rejection of such transplant in the recipient and/or for long-term maintenance therapy to prevent rejection of such transplant in the recipient (e.g., inhibiting rejection of insulin-producing islet cell transplant from a donor in the subject recipient suffering from diabetes). Thus the compounds of the invention have utility in preventing Host-Versus-Graft-Disease (HVGD) and Graft-Versus-Host-Disease (GVHD).

A CTPS1 inhibitor may be administered to the subject before, after transplantation and/or during transplantation. In some embodiments, the CTPS1 inhibitor may be administered to the subject on a periodic basis before and/or after transplantation.

In another embodiment, the disease or disorder is an allergy.

In additional embodiments the immune related disease or disorder is an autoimmune disease. As used herein, an "autoimmune disease" is a disease or disorder directed at a subject’s own tissues. Examples of autoimmune diseases include, but are not limited to Addison’s Disease, Adult-onset Still’s disease, Alopecia Areata, Alzheimer’s disease, Anti-neutrophil Cytoplasmic Antibodies (ANCA)-Associated Vasculitis, Ankylosing Spondylitis, Anti-phospholipid Syndrome (Hughes’ Syndrome), Aplastic Anemia, Arthritis, Asthma, Atherosclerosis, Atherosclerotic plaque, Atopic Dermatitis, Autoimmune Hemolytic Anemia, Autoimmune Hepatitis, Autoimmune Hypophysitis (Lymphocytic Hypophysitis), Autoimmune Inner Ear Disease, Autoimmune Lymphoproliferative Syndrome, Autoimmune Myocarditis, Autoimmune Neutropenia, Autoimmune Oophoritis, Autoimmune Orchitis, Auto-Inflammatory Diseases requiring an immunosuppressive treatment, Azoospermia, Bechet’s Disease, Berger’s Disease, Bullous Pemphigoid, Cardiomyopathy, Cardiovascular disease, Celiac disease including Refractory Celiac Disease (type I and type II), Chronic Fatigue Immune Dysfunction Syndrome (CFIDS), Chronic Idiopathic Polyneuritis, Chronic Inflammatory Demyelinating Polyneuropathy (CIPD), Chronic Relapsing Polyneuropathy (Guillain-Barré syndrome), Churg-Strauss Syndrome (CSS), Cicatricial Pemphigoid, Cold Agglutinin Disease (CAD), chronic obstructive pulmonary disease (COPD), CREST Syndrome, Cryoglobulin Syndromes, Cutaneous Lupus, Dermatitis Herpetiformis, Dermatomyositis, Eczema, Epidermolysis Bullosa Acquisita, Essential Mixed Cryoglobulinemia, Evan’s Syndrome, Exophthalmos, Fibromyalgia, Goodpasture’s Syndrome, Grave’s disease,

Hemophagocytic Lymphohistiocytosis (HLH) (including Type 1 Hemophagocytic Lymphohistiocytosis), Histiocytosis/Histiocytic Disorders, Hashimoto's Thyroiditis, Idiopathic Pulmonary Fibrosis, Idiopathic Thrombocytopenia Purpura (ITP), IgA Nephropathy, Immunoproliferative Diseases or Disorders, Inflammatory Bowel Disease (IBD), Interstitial Lung Disease, Juvenile Arthritis, Juvenile Idiopathic Arthritis (JIA), Kawasaki's Disease, Lambert-Eaton Myasthenic Syndrome, Lichen Planus, Localized Scleroderma, Lupus Nephritis, Ménière's Disease, Microangiopathic Hemoytic Anemia, Microscopic Polyangitis, Miller Fischer Syndrome/Acute Disseminated Encephalomyeloradiculopathy, Mixed Connective Tissue Disease, Multiple Sclerosis (MS), Muscular Rheumatism, Myalgic Encephalomyelitis (ME), Myasthenia Gravis, Ocular Inflammation, Pemphigus Foliaceus, Pemphigus Vulgaris, Pernicious Anemia, Polyarteritis Nodosa, Polychondritis, Polyglandular Syndromes (Whitaker's syndrome), Polymyalgia Rheumatica, Polymyositis, Primary Agammaglobulinemia, Primary Biliary Cirrhosis/Autoimmune Cholangiopathy, Primary Glomerulonephritis, Primary Sclerosing Cholangitis, Psoriasis, Psoriatic Arthritis, Pure Red Cell Anemia, Raynaud's Phenomenon, Reiter's Syndrome/Reactive Arthritis, Relapsing Polychondritis, Restenosis, Rheumatic Fever, Rheumatic Disease, Rheumatoid Arthritis, Sarcoidosis, Schmidt's Syndrome, Scleroderma/Systemic Sclerosis, Sjörgen's Syndrome, Stiff-Man Syndrome, The Sweet Syndrome (Febrile Neutrophilic Dermatosis), Systemic Lupus Erythematosus (SLE), Systemic Scleroderma, Takayasu Arteritis, Temporal Arteritis/Giant Cell Arteritis, Thyroiditis, Type 1 diabetes, Type 2 diabetes, Uveitis, Vasculitis, Vitiligo, Wegener's Granulomatosis, and X-linked lymphoproliferative disease.

Of particular interest are diseases and disorders which are mainly driven by T-cell activation and proliferation, including:

- diseases and disorders which are not linked to alloreactivity including:

    Alopecia areata, atopic dermatitis, eczema, psoriasis, lichen planus, psoriatic arthritis, vitiligo;

    Uveitis;

    Ankylosing spondylitis, Reiter's syndrome/reactive arthritis;

    Aplastic anemia, autoimmune lymphoproliferative syndrome/disorders, hemophagocytic lymphohistiocytosis;

    Type 1 diabetes; and

    Refractory celiac disease;

- Acute rejection of grafted tissues and transplanted organs; acute graft versus host disease (GVHD) after transplantation of bone marrow cells or any other source of allogenic cells including hematopoietic precursors cells and/or stem cells.

Also of interest are diseases and disorders which are driven by both T- and B-cell activation and proliferation, with an important involvement of B-cells, including:

- diseases and disorders for which the involvement of pathogenic auto-antibodies is well characterized, including:

    - Allergy;
    - Cicatricial pemphigoid, bullous pemphigoid, epidermolysis bullosa acquisita, pemphigus foliaceus, pemphigus vulgaris, dermatitis herpetiformis;
    - ANCA-associated vasculitis and microscopic polyangitis, vasculitis, Wegener's granulomatosis; Churg-Strauss syndrome (CSS), polyarteritis nodosa, cryoglobulin syndromes and essential mixed cryglobulinemia;
    - Systemic lupus erythematosus (SLE), antiphospholipid syndrome (Hughes' syndrome), cutaneous lupus, lupus nephritis, mixed connective tissue disease;
    - Thyroiditis, Hashimoto thyroiditis, Grave's disease, exophthalmos;
    - Autoimmune hemolytic anemia, autoimmune neutropenia, ITP, pernicious anaemia, pure red cell anaemia, micro-angiopathic hemolytic anemia;

- Primary glomerulonephritis, Berger's disease, Goodpasture's syndrome, IgA nephropathy; and
- Chronic idiopathic polyneuritis, chronic inflammatory demyelinating polyneuropathy (CIPD), chronic relapsing polyneuropathy (Guillain-Barré

syndrome), Miller Fischer syndrome, Stiff man syndrome, Lambert-Eaton myasthenic syndrome, myasthenia gravis.

- diseases and disorders for which the involvement of B-cells is less clearly characterized (although sometimes illustrated by the efficacy of anti-CD20 monoclonal antibodies or intravenous immunoglobulin infusions) and may not correspond or be limited to the production of pathogenic antibodies (nevertheless, non-pathogenic antibodies are sometimes described or even often present and used as a diagnosis biomarker), including:
    - Addison's disease, autoimmune oophoritis and azoospermia, polyglandular syndromes (Whitaker's syndrome), Schmidt's syndrome;
    - Autoimmune myocarditis, cardiomyopathy, Kawasaki's disease;
    - Rheumatoid arthritis, Sjögren's syndrome, mixed connective tissue disease, polymyositis and dermatomyositis; polychondritis;
    - Primary glomerulonephritis;
    - Multiple sclerosis;
    - Autoimmune hepatitis, primary biliary cirrhosis/ autoimmune cholangiopathy,

Hyper acute rejection of transplanted organs;

Chronic rejection of graft or transplants;

Chronic Graft versus Host reaction / disease after transplantation of bone marrow cells or hematopoietic precursor cells.

Additionally of interest are diseases and disorders for which the mechanism is shared between activation/proliferation of T-cells and activation/proliferation of innate immune cells and other inflammatory cellular subpopulations (including myeloid cells such as macrophages or granulocytes) and resident cells (such as fibroblasts and endothelial cells), including:

COPD, idiopathic pulmonary fibrosis, interstitial lung disease, sarcoidosis;

Adult onset Still's disease, juvenile idiopathic arthritis, Systemic sclerosis, CREST syndrome where B cells and pathogen antibodies may also play a role; the Sweet syndrome; Takayasu arteritis, temporal arteritis/ giant cell arteritis;

Ulcerative cholangitis, inflammatory bowel disease (IBD) including Crohn's disease and ulcerative colitis, primary sclerosing cholangitis.

Also of interest are diseases and disorders for which the mechanism remains poorly characterized but involves the activation and proliferation of T-cells, including:

Alzheimer's disease, cardiovascular syndrome, type 2 diabetes, restenosis, chronic fatigue immune dysfuntion syndrome (CFIDS).

- Autoimmune Lymphoproliferative disorders, including:

Autoimmune Lymphoproliferative Syndrome and X-linked lymphoproliferative disease.

Suitably the disease or disorder is selected from: inflammatory skin diseases such as psoriasis or lichen planus; acute and/or chronic GVHD such as steroid resistant acute GVHD; acute lymphoproliferative syndrome; systemic lupus erythematosus, lupus nephritis or cutaneous lupus; or transplantation.

Suitably the subject is a mammal, in particular the subject is a human.

## Pharmaceutical Compositions

For use in therapy the compounds of the invention are usually administered as a pharmaceutical composition. The invention also provides a pharmaceutical composition comprising a compound of formula (I), or a pharmaceutically acceptable salt and/or solvate (e.g. salt) and/or derivative thereof, and a pharmaceutically acceptable carrier or excipient.

In one embodiment, there is provided a pharmaceutical composition comprising a compound of formula (I), or a pharmaceutically acceptable salt and/or solvate (e.g. salt) and/or derivative thereof, for use in the treatment or prophylaxis of a disease or disorder as described herein.

In a further embodiment, there is provided a method for the prophylaxis or treatment of a disease or disorder as described herein, which comprises administering to a subject in need thereof an effective amount of a pharmaceutical composition comprising a compound of formula (I) or a pharmaceutically acceptable salt and/or solvate (e.g. salt) and/or derivative thereof.

The invention also provides the use of a pharmaceutical composition comprising a compound of formula (I), or a pharmaceutically acceptable salt and/or solvate thereof (e.g. salt) and/or derivative thereof, in the manufacture of a medicament for the treatment or prophylaxis of a disease or disorder as described herein.

The compounds of formula (I) or their pharmaceutically acceptable salts and/or solvates and/or derivatives thereof may be administered by any convenient method, e.g. by oral, parenteral, buccal, sublingual, nasal, rectal or transdermal administration, and the pharmaceutical compositions adapted accordingly.

The compounds of formula (I) or their pharmaceutically acceptable salts and/or solvates and/or derivatives thereof which are active when given orally can be formulated as liquids or solids, e.g. as syrups, suspensions, emulsions, tablets, capsules or lozenges.

A liquid formulation will generally consist of a suspension or solution of the active ingredient (such as a compound of formula (I) or a pharmaceutically acceptable salt and/or solvate (e.g. salt) and/or derivative thereof) in a suitable liquid carrier(s) e.g. an aqueous solvent such as water, ethanol or glycerine, or a non-aqueous solvent, such as polyethylene glycol or an oil. The formulation may also contain a suspending agent, preservative, flavouring and/or colouring agent.

A composition in the form of a tablet can be prepared using any suitable pharmaceutical carrier(s) routinely used for preparing solid formulations, such as magnesium stearate, starch, lactose, sucrose and cellulose.

A composition in the form of a capsule can be prepared using routine encapsulation procedures, e.g. pellets containing the active ingredient (such as a compound of formula (I) or a pharmaceutically acceptable salt and/or solvate (e.g. salt) and/or derivative thereof) can be prepared using standard carriers and then filled into a hard gelatin capsule; alternatively a dispersion or suspension can be prepared using any suitable pharmaceutical carrier(s), e.g. aqueous gums, celluloses, silicates or oils and the dispersion or suspension then filled into a soft gelatin capsule.

Typical parenteral compositions consist of a solution or suspension of the active ingredient (such as a compound of formula (I) or a pharmaceutically acceptable salt and/or solvate (e.g. salt) and/or derivative thereof) in a sterile aqueous carrier or parenterally acceptable oil, e.g. polyethylene glycol, polyvinyl pyrrolidone, lecithin, arachis oil or sesame oil. Alternatively, the solution can be lyophilised and then reconstituted with a suitable solvent just prior to administration.

Compositions for nasal administration may conveniently be formulated as aerosols, drops, gels and powders. Aerosol formulations typically comprise a solution or fine suspension of the active ingredient in a pharmaceutically acceptable aqueous or non-aqueous solvent and are usually presented in single or multidose quantities in sterile form in a sealed container which can take the form of a cartridge or refill for use with an atomising device. Alternatively the sealed container may be a disposable dispensing device such as a single dose nasal inhaler or an aerosol dispenser fitted with a metering valve. Where the dosage form comprises an aerosol dispenser, it will contain a propellant which can be a compressed gas e.g. air, or an organic propellant such as a fluoro-chloro-hydrocarbon or hydrofluorocarbon. Aerosol dosage forms can also take the form of pump-atomisers.

Compositions suitable for buccal or sublingual administration include tablets, lozenges and pastilles where the active ingredient is formulated with a carrier such as sugar and acacia, tragacanth, or gelatin and glycerin.

Compositions for rectal administration are conveniently in the form of suppositories containing a conventional suppository base such as cocoa butter.

Compositions suitable for transdermal administration include ointments, gels and patches.

Suitably, the composition is in unit dose form such as a tablet, capsule or ampoule.

The composition may for example contain from 0.1% to 100% by weight, for example from 10 to 60% by weight, of the active material, depending on the method of administration. The composition may contain from 0% to 99% by weight, for example 40% to 90% by weight, of the carrier, depending on the method of administration. The composition

may contain from 0.05 mg to 2000 mg, for example from 1.0 mg to 500 mg, of the active material, depending on the method of administration. The composition may contain from 50 mg to 1000 mg, for example from 100 mg to 400 mg of the carrier, depending on the method of administration. The dose of the compound used in the treatment or prophylaxis of the aforementioned disorders will vary in the usual way with the seriousness of the disorders, the weight of the sufferer, and other similar factors. However, as a general guide suitable unit doses may be 0.05 mg to 1000 mg, more suitably 1.0 mg to 500 mg, and such unit doses may be administered more than once a day, for example two or three a day. Such therapy may extend for a number of weeks or months.

The invention provides, in a further aspect, a combination comprising a compound of formula (I) or a pharmaceutically acceptable, salt, solvate and/or derivative thereof (e.g. a combination comprising a compound of formula (I) or a pharmaceutically acceptable derivative thereof) together with a further pharmaceutically acceptable active ingredient or ingredients.

The invention provides a compound of formula (I), for use in combination with a further pharmaceutically acceptable active ingredient or ingredients.

When the compounds are used in combination with other therapeutic agents, the compounds may be administered separately, sequentially or simultaneously by any convenient route.

Optimal combinations may depend on the disease or disorder. Possible combinations include those with one or more active agents selected from the list consisting of: 5-aminosalicylic acid, or a prodrug thereof (such as sulfasalazine, olsalazine or bisalazide); corticosteroids (e.g. prednisolone, methylprednisolone, or budesonide); immunosuppressants (e.g. cyclosporin, tacrolimus, sirolimus, methotrexate, azathioprine mycophenolate mofetil, leflunomide, cyclophosphamide, 6-mercaptopurine or antilymphocyte (or thymocyte) globulins); anti-TNF-alpha antibodies (e.g., infliximab, adalimumab, certolizumab pegol or golimumab); anti-IL12/IL23 antibodies (e.g., ustekinumab); anti-IL6 or anti-IL6R antibodies, anti-IL17 antibodies or small molecule IL12/IL23 inhibitors (e.g., apilimod); Anti-alpha-4-beta-7 antibodies (e.g., vedolizumab); MAdCAM-1 blockers (e.g., PF-00547659); antibodies against the cell adhesion molecule alpha-4-integrin (e.g., natalizumab); antibodies against the IL2 receptor alpha subunit (e.g., daclizumab or basiliximab); JAK inhibitors including JAK1 and JAK3 inhibitors (e.g., tofacitinib, baricitinib, R348); Syk inhibitors and prodrugs thereof (e.g., fostamatinib and R-406); Phosphodiesterase-4 inhibitors (e.g., tetomilast); HMPL-004; probiotics; Dersalazine; semapimod/CPSI-2364; and protein kinase C inhibitors (e.g. AEB-071).

Some of the combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations. The individual components of combinations may also be administered separately, through the same or different routes.

When a compound of formula (I) or a pharmaceutically acceptable derivative thereof is used in combination with a second therapeutic agent active against the same disease state the dose of each compound may differ from that when the compound is used alone. Appropriate doses will be readily appreciated by those skilled in the art.

The invention is further exemplified by the following non-limiting examples.

## EXAMPLES

Abbreviations used herein are defined below. Any abbreviations not defined are intended to convey their generally accepted meaning.

### Abbreviations

| | |
|---|---|
| AcOH | glacial acetic acid |
| $AlMe_3$ | trimethylaluminium |
| aq | aqueous |
| Ar | aromatic ring |
| BEH | ethylene bridged hybrid |
| Bispin | bis(pinacolato)diboron; 4,4,4',4',5,5,5',5'-Octamethyl-2,2'-bi-1,3,2-dioxaborolane |
| BOC | *tert*-butyloxycarbonyl protecting group |
| $Cs_2CO_3$ | cesium carbonate |
| CSH | charged surface hybrid |
| d | doublet |
| DCM | dichloromethane |

| | |
|---|---|
| DIBAL-H | diisobutylaluminium hydride |
| DIPEA | *N,N*-diisopropylethylamine |
| dioxane | 1,4-dioxane |
| DMF | *N,N*-dimethylformamide |
| DMSO | dimethyl sulfoxide |
| dppf | 1,1'-bis(diphenylphosphino)ferrocene |
| $(ES^+)$ | electrospray ionisation, positive mode |
| $(ES^-)$ | electrospray ionisation, negative mode |
| ESI | electrospray ionisation |
| Et | ethyl |
| EtMgBr | ethyl magnesium bromide |
| EtOAc | ethyl acetate |
| EtOH | ethanol |
| g | grams |
| HATU | 1-[bis(dimethylamino)methylene]-1*H*-1,,2,3-triazolo[4,5-*b*]pyridinium 3-oxid hexafluorophosphate |
| HCl | hydrochloric acid |
| HPLC | high performance liquid chromatography |
| hr(s) | hour(s) |
| $H_2SO_4$ | sulfuric acid |
| $IC_{50}$ | 50% inhibitory concentration |
| $K_2CO_3$ | potassium carbonate |
| LCMS | liquid chromatography-mass spectrometry |
| LiOH | lithium hydroxide |
| $(M+H)^+$ | protonated molecular ion |
| $(M-H)^-$ | unprotonated molecular ion |
| M | molar concentration |
| mL | millilitre |
| mm | millimeter |
| mmol | millimole |
| $MgSO_4$ | magnesium sulfate |
| Me | methyl |
| MeCN | acetonitrile |
| MeMgBr | methyl magnesium bromide |
| MeOH | methanol |
| MHz | megahertz |
| min(s) | minute(s) |
| MSD | mass selective detector |
| m/z | mass-to-charge ratio |
| $N_2$ | nitrogen gas |
| $NH_3$ | ammonia |
| $NH_4Cl$ | ammonium chloride |
| NaH | sodium hydride |
| $Na_2SO_4$ | sodium sulfate |
| $NaHCO_3$ | sodium bicarbonate |
| nM | nanomolar |
| nm | nanometre |
| NMR | nuclear magnetic resonance (spectroscopy) |
| PDA | photodiode array |
| $PdCl_2(dppf).CH_2Cl_2$ | [1,1'-bis(diphenylphosphino)ferrocene]dichloropalladium(II) dichloromethane |
| prep HPLC | preparative high performance liquid chromatography |
| Ph | phenyl |
| pos/neg | positive/negative |
| q | quartet |
| RT | room temperature |
| Rt | retention time |
| RP | reverse phase |
| s | singlet |

| | |
|---|---|
| $S_NAr$ | nucleophilic aromatic substitution |
| sat | saturated |
| SCX | solid supported cation exchange (resin) |
| t | triplet |
| T3P | 2,4,6-tripropyl-1,3,5,2,4,6-trioxatriphosphorinane-2,4,6-trioxide |
| TBME | *tert*-butyl methyl ether |
| TFA | trifluoroacetic acid |
| TMSOK | potassium trimethylsilanolate |
| THF | tetrahydrofuran |
| UPLC | ultra performance liquid chromatography |
| UV | ultraviolet |
| v/v | volume/volume |
| VWD | variable wave detector |
| wt | weight |
| um | micrometre |
| uM | micromolar |
| uL | microlitre |

**General Procedures**

All starting materials and solvents were obtained either from commercial sources or prepared according to the literature. Unless otherwise stated all reactions were stirred. Organic solutions were routinely dried over anhydrous magnesium sulfate or sodium sulfate. Hydrogenations were performed on a Thales H-cube flow reactor under the conditions stated.

Column chromatography was performed on pre-packed silica (230-400 mesh, 40-63 um) cartridges using the amount indicated. SCX was purchased from Supelco and treated with 1 M hydrochloric acid prior to use. Unless stated otherwise the reaction mixture to be purified was first diluted with MeOH and made acidic with a few drops of AcOH. This solution was loaded directly onto the SCX and washed with MeOH. The desired material was then eluted by washing with 0.7 M $NH_3$ in MeOH.

*Preparative Reverse Phase High Performance Liquid Chromatography (HPLC)*

**Prep HPLC**

***Acidic prep***

Waters X-Select CSH column C18, 5 um (19 x 50 mm), flow rate 28 mL $min^{-1}$ eluting with a $H_2O$-MeCN gradient containing 0.1 % v/v formic acid over 6.5 min using UV detection at 254 nm.

***Basic prep***

Waters X-Bridge Prep column C18, 5 um (19 x 50 mm), flow rate 28 mL $min^{-1}$ eluting with a 10 mM $NH_4HCO_3$-MeCN gradient over 6.5 min using UV detection at 254 nm.

**Prep Chiral HPLC**

**Chiral Method A:** Chiralpak® IA (Daicel Ltd.) column (2 x 25 cm), flow rate 13.5 mL $min^{-1}$ eluting with a mixture of (30 %) EtOH in a 4:1 mixture of heptane + 0.2 %TFA and $CHCl_3$, UV detection at 254 nm. Samples were loaded onto the column via an at-column dilution pump, pumping (EtOH) (1.5 mL $min^{-1}$) for the duration of the run, giving a combined flow rate of 15 mL $min^{-1}$

**Chiral Method B:** Chiralpak® IA (Daicel Ltd.) column (2 x 25 cm), flow rate 13.5 mL $min^{-1}$ eluting with a mixture of (40 %) EtOH in a 4:1 mixture of heptane + 0.2 %TFA and $CHCl_3$, UV detection at 254 nm. Samples were loaded onto the column via an at-column dilution pump, pumping (EtOH) (1.5 mL $min^{-1}$) for the duration of the run, giving a combined flow rate of 15 mL $min^{-1}$

**Analytical Methods**

*Reverse Phase HPLC Conditions for the LCMS Analytical Methods*

**HPLC acidic:** Acidic LCMS 4 minute (5-95%)

Analytical LCMS was carried out using a Waters X-Select CSH C18, 2.5 um, 4.6 x 30 mm column eluting with a gradient of 0.1 % formic acid in MeCN in 0.1 % formic acid in water. The gradient from 5-95 % 0.1 % formic acid in MeCN occurred between 0.00-3.00 minutes at 2.5 mL/min with a flush from 3.01-3.5 minutes at 4.5 mL/min. A column re-equilibration to 5% MeCN was from 3.60-4.00 minutes at 2.5 mL/min. UV spectra of the eluted peaks were measured using an Agilent 1260 Infinity VWD at 254 nm. Mass spectra were measured using an Agilent 6120 MSD running with positive/negative switching.

**HPLC basic:** Basic LCMS 4 minute (5-95%)

Analytical LCMS was carried out using a Waters X-Select BEH C18, 2.5 um, 4.6x30 mm column eluting with a gradient of MeCN in aqueous 10mM ammonium bicarbonate. The gradient from 5-95% MeCN occurred between 0.00-3.00 minutes at 2.5mL/min with a flush from 3.01-3.5 minutes at 4.5 mL/min. A column re-equilibration to 5% MeCN was from 3.60-4.00 minutes at 2.5mL/min. UV spectra of the eluted peaks were measured using an Agilent 1260 Infinity VWD at 254nm. Mass spectra were measured using an Agilent 6120 MSD running with positive/negative switching.

*Reverse Phase HPLC Conditions for the UPLC Analytical Methods*

**UPLC acidic:** Acidic UPLC 3 minute

Analytical UPLC/MS was carried out using a Waters Acquity CSH C18, 1.7 um, 2.1 x 30 mm column eluting with a gradient of 0.1% formic acid in MeCN in 0.1 % formic acid in water. The gradient was structured with a starting point of 5 % MeCN held from 0.0-0.11 minutes. The gradient from 5-95 % occurred between 0.11-2.15 minutes with a flush from 2.15-2.56 minutes. A column re-equilibration to 5 % MeCN was from 2.56-2.83 minutes. UV spectra of the eluted peaks were measured using an Acquity PDA and mass spectra were recorded using an Acquity QDa detector with ESI positive/negative switching.

**UPLC basic:** Basic UPLC 3 minute

Analytical UPLC/MS was carried out using a Waters Acquity BEH C18, 1.7 um, 2.1 x 30 mm column eluting with a gradient of MeCN in aqueous 10 mM ammonium bicarbonate. The gradient was structured with a starting point of 5 % MeCN held from 0.0-0.11 minutes. The gradient from 5-95% occurred between 0.11-2.15 minutes with a flush from 2.15-2.56 minutes. A column re-equilibration to 5% MeCN was from 2.56-2.83 minutes. UV spectra of the eluted peaks were measured using an Acquity PDA and mass spectra were recorded using an Acquity QDa detector with ESI positive/negative switching.

Column temperature was 40 °C in all runs. Injection volume was 3 uL and the flow rate was 0.77 mL/min. PDA scan from 210-400 nm was conducted on all runs.

*Normal Phase HPLC Conditions for the Chiral Analytical Methods*

**Chiral IA method 1:** Chiral HPLC (Daicel Chiralpak IA, 5 um, 4.6 x 250 mm, 1.0 mL/min, 5-95% (gradient over 45 min) EtOH (0.2 % TFA) in [4:1 heptane (0.2 % TFA):$CHCl_3$].

**Chiral IA method 2:** Chiral HPLC (Daicel Chiralpak IA, 5 um, 4.6 x 250 mm, 1.0 mL/min, Isocratic 40 % EtOH (0.2 % TFA) in [4:1 heptane (0.2 % TFA):$CHCl_3$].

*$^1H$ NMR Spectroscopy*

$^1H$ NMR spectra were acquired on a Bruker Avance III spectrometer at 400 MHz or Bruker Avance III HD spectrometer at 500 MHz using residual undeuterated solvent as reference and unless specified otherwise were run in DMSO-$d_6$.

**Preparation of Intermediates**

Known synthetic intermediates were procured from commercial sources or were obtained using published literature procedures. Additional intermediates were prepared by the representative synthetic processes described herein.

Ethyl 2-(cyclopropanesulfonamido)thiazole-4-carboxylate **INTE1**

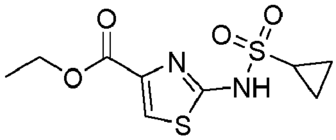

A solution of ethyl 2-aminothiazole-4-carboxylate (6.49 g, 37.7 mmol) and cyclopropanesulfonyl chloride (4 mL, 39.5 mmol) in pyridine (15 mL) was warmed to 40 °C and stirred for 48 hrs. The reaction mixture was taken up in DMSO (20 mL) and the crude product was purified by reverse phase chromatography on C18 silica (330 g column, 10-20 % MeCN/10 mM ammonium bicarbonate) to afford the product which was then taken up in EtOAc (300 mL) and washed with 1 M HCl (aq, 300 mL) and brine (150 mL). The organic layer was dried ($Na_2SO_4$), filtered and concentrated to afford ethyl 2-(cyclopropanesulfonamido)thiazole-4-carboxylate (4.7 g, 15.31 mmol, 41 % yield) as an orange oil; Rt 1.36 min (HPLC acidic); m/z 277 $(M+H)^+$ $(ES^+)$; $^1H$ NMR (400 MHz, DMSO-$d_6$) δ 13.14 (s, 1H), 7.72 (s, 1H), 4.28 (q, J = 7.1 Hz, 2H), 2.72 - 2.58 (m, 1H), 1.29 (t, J = 7.1 Hz, 3H), 1.01 - 0.81 (m, 4H).

*N*-(4-Formylthiazol-2-yl)cyclopropanesulfonamide **INTE2**

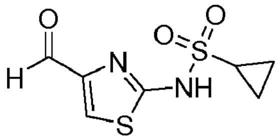

A solution of ethyl 2-(cyclopropanesulfonamido)thiazole-4-carboxylate **INTE1** (1.0 g, 3.62 mmol) in DCM (10 mL) was cooled to -78 °C whereupon DIBAL-H (1 M in DCM) (14.5 mL, 14.5 mmol) was added dropwise over 30 mins. The reaction was then stirred at this temperature for 2 hrs. MeOH (1.5 mL) was added cautiously before the reaction mixture was allowed to warm to RT. DCM (50 mL) was then added, followed by 1 M HCl (aq, 90 mL) with vigorous stirring. The mixture was stirred for 10 mins before being passed through a phase separator and further extracting with DCM (50 mL). The organic layers were combined and concentrated onto silica (2 g) and the crude product was purified by chromatography on silica (40 g column, 20-80 % EtOAc/*iso*-hexanes) to afford N-(4-formylthiazol-2-yl)cyclopropanesulfonamide (122 mg, 0.5 mmol, 14 % yield) as a colourless gum; Rt 0.53 min (UPLC acidic); m/z 233 $(M+H)^+$ $(ES^+)$; $^1H$ NMR (400 MHz, DMSO-$d_6$) δ 13.17 (s, 1H), 9.52 (s, 1H), 8.06 (s, 1H), 2.73 - 2.57 (m, 1H), 1.04 - 0.67 (m, 4H).

2-(Cyclopropanesulfonamido)thiazole-4-carboxylic acid **INTE3**

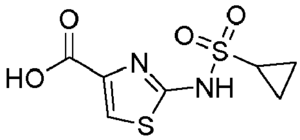

A solution of ethyl 2-(cyclopropanesulfonamido)thiazole-4-carboxylate **INTE1** (1.3 g, 4.70 mmol) in 2 M LiOH (aq, 4.70 mL, 9.41 mmol), MeOH (1 mL) and THF (6 mL) was stirred at RT. The reaction mixture was part concentrated (to approx. 6 mL) then acidified with 1 M HCl (aq. 12 mL). The aqueous layer was then extracted with EtOAc (3 x 20 mL), then the organic phases were combined, dried ($Na_2SO_4$), filtered and concentrated to afford 2-(cyclopropanesulfonamido)thiazole-4-carboxylic acid (910 mg, 3.59 mmol, 76 % yield) as a colourless solid; Rt 0.65 min (UPLC acidic); m/z 249 $(M+H)^+$ $(ES^+)$; $^1H$ NMR (400 MHz, DMSO-$d_6$) δ 13.83 - 12.58 (br. s, 2H), 7.61 (s, 1H), 2.67 - 2.58 (m, 1H), 1.07 - 0.73 (m, 4H).

2-(Cyclopropanesulfonamido)-*N*-methoxy-*N*-methylthiazole-4-carboxamide **INTE4**

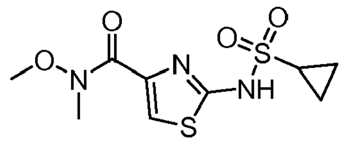

A solution of 2-(cyclopropanesulfonamido)thiazole-4-carboxylic acid **INTE3** (910 mg, 3.67 mmol) and *N*,O-dimethylhydroxylamine hydrochloride (358 mg, 3.67 mmol) in DMF (6 mL) was treated with DIPEA (2.2 mL, 12.8 mmol) and stirred for 5 mins before HATU (1.3 g, 3.67 mmol) was added, the reaction mixture was stirred at RT for 20 hrs. The reaction mixture was taken up in 1 M HCl (aq, 50 mL) which was extracted with EtOAc (3 x 50 mL). The organic phases were combined, dried ($Na_2SO_4$), filtered and concentrated onto silica (5 g). The crude product was purified by chromatography on silica (24 g column, 0-100 % EtOAc/*iso*-hexanes) to afford 2-(cyclopropanesulfonamido)-N-methoxy-N-methylthiazole-4-carboxamide (786 mg, 2.64 mmol, 72 % yield) as a colourless solid; Rt 0.73 min (UPLC acidic); m/z 292 $(M+H)^+$ $(ES^+)$; $^1H$ NMR (400 MHz, DMSO-$d_6$) $\delta$ 12.75 (s, 1H), 7.58 (s, 1H), 3.73 (s, 3H), 3.26 (s, 3H), 2.68 - 2.58 (m, 1H), 1.00 - 0.84 (m, 4H).

N-(4-Propionylthiazol-2-yl)cyclopropanesulfonamide **INTE5**

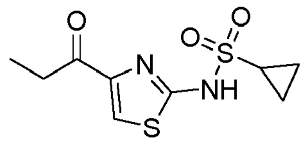

A suspension of 2-(cyclopropanesulfonamido)-*N*-methoxy-*N*-methylthiazole-4-carboxamide **INTE4** (2.5 g, 8.58 mmol) in THF (100 mL) at 40 °C was treated dropwise with 2 M EtMgCl (THF, 8.6 mL, 17.2 mmol). The reaction mixture was maintained at 40 °C for 18 hrs. The reaction mixture was quenched by addition of $NH_4Cl$ (sat. aq, 30 mL) followed by EtOAc (50 mL) and water (20 mL). The phases were partitioned and the aqueous layer was extracted with EtOAc (50 mL), the organic phases were combined, dried ($Na_2SO_4$), filtered and concentrated onto silica (10 g). The crude product was purified by chromatography on silica (24 g column, 0-50 % EtOAc/*iso*-hexanes) to afford N-(4-propionylthiazol-2-yl)cyclopropanesulfonamide (1.9 g, 5.84 mmol, 68 % yield) as an off white gum; Rt 1.24 min (HPLC acidic); m/z 261 $(M+H)^+$ $(ES^+)$; $^1H$ NMR (400 MHz, DMSO-$d_6$) $\delta$ 12.92 (s, 1H), 7.95 (s, 1H), 2.86 (q, J = 7.2 Hz, 2H), 2.68 - 2.58 (m, 1H), 1.05 (t, J = 7.2 Hz, 3H), 0.97 - 0.80 (m, 4H).

**Method A: Reductive amination**

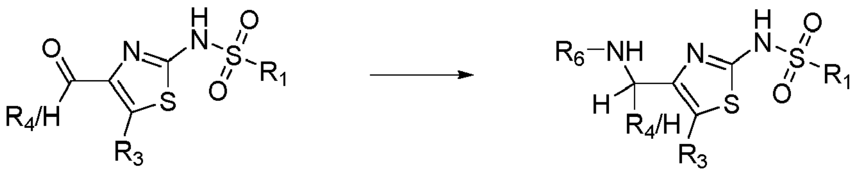

A solution of aldehyde/ketone (1 eq.) in THF was treated with AcOH (1 eq.), amine (1 eq.) and a reducing agent such as STAB (1.2 eq.) and stirred at RT for 1 hr. The reaction mixture was quenched by addition of MeOH then loaded directly on to SCX (1 g/mmol of substrate), washed with MeOH and the product was eluted with 1 M $NH_3$ in MeOH. The crude product was then concentrated onto silica and purified by normal phase chromatography.

**Table 1**: The following intermediates were made according to Method A.

| INT | Name/Structure (All examples containing chiral centres are racemates unless stated) | Synthesis Method, [LCMS Method], m/z $(M+H)^+$, (Rt/min) | $^1H$ NMR Chemical Shift Data (DMSO-$d_6$ unless stated) |
|---|---|---|---|
| **INTE6** | *N*-(4-(((2,4-dimethoxybenzyl)amino) methyl)thiazol-2-yl) cyclopropanesulfonamide 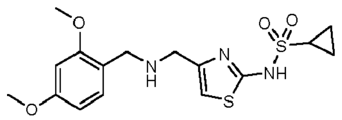 | Using **INTE2** [HPLC acidic], 384 (0.99). | 7.23 (d, J = 8.3 Hz, 1H), 6.56 (d, J = 2.4 Hz, 1H), 6.52 (d, J = 2.4 Hz, 1H), 6.50 (d, J = 2.3 Hz, 1H), 3.79 (s, 3H), 3.76 (s, 3H), 3.74 (s, 2H), 3.63 - 3.61 (m, 2H), 2.49 - 2.45 (m, 1H), 0.89 - 0.72 (m, 4H), zwitterion, 2 x N-H not observed. |
| **INTE7** | *N*-(4-(1-((2,4-dimethoxybenzyl) amino)propyl)thiazol-2-yl) cyclopropanesulfonamide 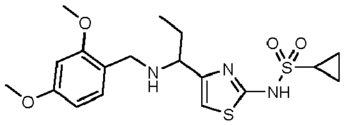 | Using **INTE5** [UPLC acidic], 412 (0.62). | 7.18 (d, J = 8.3 Hz, 1H), 6.59 - 6.37 (m, 3H), 3.76 (s, 3H), 3.75 (s, 3H), 3.64 - 3.43 (m, 3H), 2.58 - 2.52 (m, 1H), 1.76 - 1.57 (m, 2H), 0.91 - 0.81 (m, 4H), 0.78 (t, J = 7.4 Hz, 3H), 2 x N-H not observed. |
| **INTE8** | N-(4-(1-((4-methoxybenzyl)(methyl) amino)propyl)thiazol-2-yl) cyclopropanesulfonamide 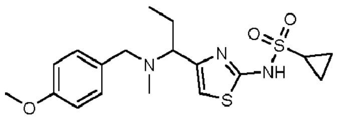 | Using **INTE5,** [HPLC basic], 396 (1.63). | 12.11 (v. br. s, 1H), 7.26 - 7.18 (m, 2H), 6.91 - 6.83 (m, 2H), 6.58 (s, 1H), 3.73 (s, 3H), 3.55 - 3.42 (m, 2H), 3.39 - 3.26 (m, 1H), 2.65 - 2.57 (m, 1H), 1.99 (s, 3H), 1.91 - 1.57 (m, 2H), 0.97 - 0.84 (m, 5H), 0.75 - 0.65 (m, 2H). |

**Method B**: **Benzylamine deprotection (TFA)**

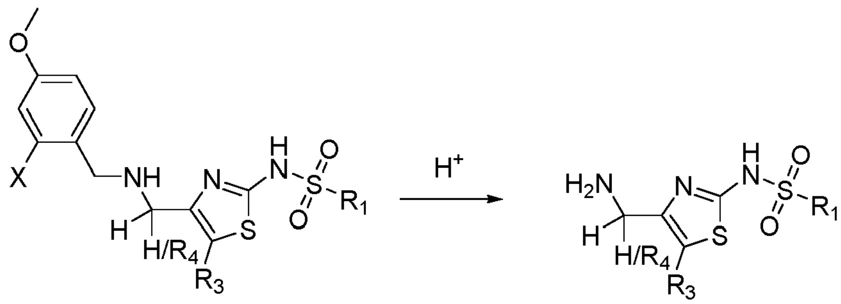


X = H or OMe

Benzylamine derivative (1 eq.) was dissolved in TFA (50 eq.) and heated to 70 °C for 1 - 24 hrs. The reaction was allowed to cool to RT, then was loaded on to SCX (1 g/mmol of substrate) and washed with MeOH. The required compound was eluted with 1% $NH_3$ in MeOH.

**Table 2:** The following intermediates were made according to Method B.

| INT | Name/Structure (All examples containing chiral centres are racemates unless stated) | Synthesis Method, [LCMS Method], m/z $(M+H)^+$, (Rt/min) | $^1$H NMR Chemical Shift Data (DMSO-$d_6$ unless stated) |
|---|---|---|---|
| **INTE9** | *N*-(4-(aminomethyl)thiazol-2-yl) cyclopropanesulfonamide 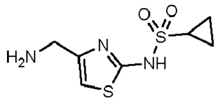 | Using **INTE6,** [UPLC basic], 234 (0.23). | 6.44 (s, 1H), 3.79 - 3.70 (m, 2H), 2.46 - 2.33 (m, 1H), 0.85 - 0.75 (m, 2H), 0.74 - 0.61 (m, 2H), 3 x NH not observed. |
| **INTE10** | *N*-(4-(1-aminopropyl)thiazol-2-yl) cyclopropanesulfonamide 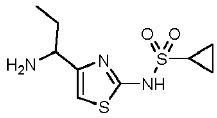 | Using **INTE7,** [HPLC basic], 262 $(M+H)^+$ $(ES^+)$; (0.51). | 8.00 (v. br. s, 3H), 6.43 (s, 1H), 3.98 - 3.73 (m, 1H, obscured by impurity), 2.42 - 2.28 (m, 1H), 1.92 - 1.62 (m, 2H), 0.95 - 0.40 (m, 7H). |
| **INTE11** | N-(4-(1-(methylamino)propyl)thiazol-2-yl)cyclopropanesulfonamide 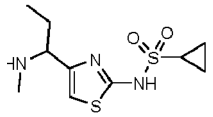 | Using **INTE8,** [HPLC acidic], 276 (0.34). | 6.49 (s, 1H), 3.70 - 3.57 (m, 1H), 2.44 - 2.34 (m, 1H), 2.32 (s, 3H), 1.82 - 1.69 (m, 2H), 0.84 - 0.74 (m, 5H), 0.74 - 0.66 (m, 2H), 2 x NH not observed |

*N*-(4-(2-Hydroxypropan-2-yl)thiazol-2-yl)cyclopropanesulfonamide **INTE12**

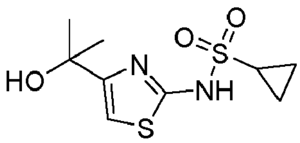

A solution of ethyl 2-(cyclopropanesulfonamido)thiazole-4-carboxylate **INTE1** (2.7 g, 9.77 mmol) in THF (30 mL) was cooled to 0 °C then MeMgBr (3.4 M in THF, 14.4 mL, 48.9 mmol) was added dropwise, over approx. 20 mins, maintaining the temperature below 10 °C. Once addition was complete the reaction mixture was allowed to warm to RT and was stirred for 24 hrs. The reaction mixture was cooled with an ice bath and $NH_4Cl$ (sat. aq, 10 mL) was added cautiously, resulting in precipitate formation. 1 M HCl (aq, 50 mL) was added and the reaction mixture was extracted with EtOAc (3 x 50 mL). The organic phases were combined, dried ($MgSO_4$), filtered and concentrated onto silica (10 g). The product was then purified by chromatography on silica (24 g column, 0-100 % EtOAc/*iso*-hexanes) to afford N-(4-(2-hydroxypropan-2-yl)thiazol-2-yl)cyclopropanesulfonamide (1.03 g, 3.53 mmol, 36 % yield) as a pale yellow solid: Rt 1.05 min (HPLC acidic); m/z 263 $(M+H)^+$ $(ES^+)$; $^1$H NMR (400 MHz, DMSO-$d_6$) δ 12.44 (s, 1H), 6.42 (s, 1H), 5.35 (s, 1H), 2.64 - 2.54 (m, 1H), 1.40 (s, 6H), 0.96 - 0.74 (m, 4H).

2-Chloro-*N*-(2-(2-(cyclopropanesulfonamido)thiazol-4-yl)propan-2-yl)acetamide **INTE13**

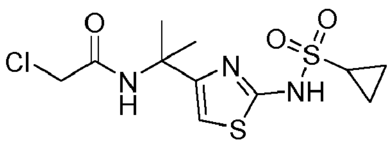

A mixture of *N*-(4-(2-hydroxypropan-2-yl)thiazol-2-yl)cyclopropanesulfonamide **INTE12** (500 mg, 1.91 mmol)

and 2-chloroacetonitrile (0.72 mL, 11.4 mmol) in AcOH (0.87 mL, 15 mmol) was cooled in an ice bath before $H_2SO_4$ (0.92 mL, 17.2 mmol) was added. The reaction was allowed to warm to RT and was stirred for 18 hrs. The solution was poured onto ice water (10 mL) and extracted with DCM (3 x 10 mL), partitioning with a phase separator. The crude product was purified by chromatography on silica (12 g column, 0-100 % EtOAc/*iso*-hexanes) to afford 2-chloro-N-(2-(2-(cyclopropanesulfonamido)thiazol-4-yl)propan-2-yl)acetamide (574 mg, 1.61 mmol, 85 % yield) as an orange gum; Rt 0.78 min (UPLC acidic); m/z 337 ($^{35}$Cl M+H)$^+$ (ES$^+$); $^1$H NMR (400 MHz, DMSO-$d_6$) $\delta$ 12.46 (s, 1H), 8.29 (s, 1H), 6.44 (s, 1H), 4.11 - 3.87 (m, 2H), 2.63 - 2.53 (m, 1H), 1.51 (s, 6H), 0.97 - 0.83 (m, 4H).

*N*-(4-(2-Aminopropan-2-yl)thiazol-2-yl)cyclopropanesulfonamide **INTE14**

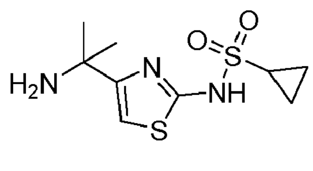

A suspension of thiourea (154 mg, 2.03 mmol) and 2-chloro-*N*-(2-(2-(cyclopropanesulfonamido)thiazol-4-yl)propan-2-yl)acetamide **INTE13** (570 mg, 1.69 mmol) in EtOH (8 mL) was treated with AcOH (1.6 mL, 28.7 mmol) and heated to reflux for 20 hrs. The reaction mixture was allowed to cool to RT. The resulting precipitate was filtered and the filtrate was loaded onto SCX (5 g), washed with MeOH and the required product was isolated by eluting with 1 % $NH_3$ in MeOH to afford *N*-(4-(2-aminopropan-2-yl)thiazol-2-yl)cyclopropanesulfonamide (479 mg, 1.80 mmol, quant. yield) as a slightly pink solid; Rt 0.50 min (UPLC basic); m/z 262 (M+H)$^+$ (ES$^+$); $^1$H NMR (400 MHz, DMSO-$d_6$) $\delta$ 6.38 (s, 1H), 2.44 - 2.21 (m, 1H), 1.49 (s, 6H), 0.89 - 0.35 (m, 4H), 3 x H exchangeable protons were very broad and are not reported.

*N*-(4-(3-hydroxypentan-3-yl)thiazol-2-yl)cyclopropanesulfonamide **INTE15**

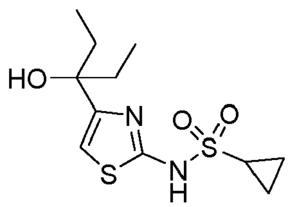

A solution of ethyl 2-(cyclopropanesulfonamido)thiazole-4-carboxylate **INTE1** (3 g, 10.86 mmol) in THF (50 mL) was cooled to 0 ° C, then EtMgBr (2 M in THF) (27.1 mL, 54.3 mmol) was added dropwise over approx. 20 mins, maintaining the temperature below 20 ° C. Once addition was complete the reaction mixture was warmed to RT and was stirred for 1 hr after which the reaction mixture was cooled with an ice bath and 1 M HCl (aq, 65 mL) was added cautiously. The aqueous layer was extracted with EtOAc (3 x 50 mL). The organic phases were combined, dried ($Na_2SO_4$), filtered and concentrated onto silica (10 g). The product was purified by chromatography on silica (24 g column, 0-100 % EtOAc/*iso*-hexanes) to afford *N*-(4-(3-hydroxypentan-3-yl)thiazol-2-yl)cyclopropane sulfonamide (2.13 g, 6.97 mmol, 64 % yield) as a colourless solid. Rt 0.86 min (UPLC, acidic); m/z 313.2 (M+Na)$^+$ (ES$^+$); $^1$H NMR (400 MHz, DMSO-$d_6$) $\delta$ 12.29 (s, 1H), 6.37 (s, 1H), 4.91 (s, 1H), 2.63 - 2.53 (m, 1H), 1.82 - 1.47 (m, 4H), 0.95 - 0.82 (m, 4H), 0.70 (t, J = 7.3 Hz, 6H).

2-Chloro-N-(3-(2-(cyclopropanesulfonamido)thiazol-4-yl)pentan-3-yl)acetamide **INTE16**

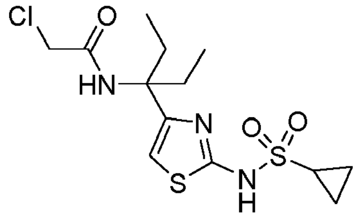

A mixture of *N*-(4-(3-hydroxypentan-3-yl)thiazol-2-yl)cyclopropanesulfonamide **INTE15** (2.13 g, 7.33 mmol) and 2-chloroacetonitrile (2.8 mL, 44.5 mmol) in AcOH (3.4 mL, 59.4 mmol) was cooled in an ice bath before sulfuric acid (3.5 mL, 65.7 mmol) was added to afford a solution. The reaction was warmed to RT and was stirred for 18 hrs. The solution was poured onto ice water (100 mL) and extracted with DCM (3 x 100 mL) partitioning with a phase separator. The material was concentrated onto silica (20 g) and the crude product was purified by chromatography on silica (80 g column, 0-100 % EtOAc/*iso*-hexanes) to afford 2-chloro-*N*-(3-(2-(cyclopropanesulfonamido)thiazol-4-yl)pentan-3-yl)acetamide (1.62 g, 4.21 mmol, 57 % yield) as a colourless solid. Rt 0.76 min (UPLC, acidic); m/z 366.2 ($^{15}$Cl M+H)$^+$ (ES$^+$); $^1$H NMR (400 MHz, DMSO-$d_6$) δ 12.36 (s, 1H), 7.99 (s, 1H), 6.44 (s, 1H), 4.09 (s, 2H), 2.62 - 2.53 (m, 1H), 2.05 - 1.86 (m, 2H), 1.80 - 1.66 (m, 2H), 0.95 - 0.83 (m, 4H), 0.69 (t, J = 7.3 Hz, 6H).

*N*-(4-(3-aminopentan-3-yl)thiazol-2-yl)cyclopropanesulfonamide **INTE17**

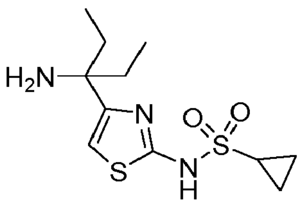

A solution of thiourea (0.40 g, 5.25 mmol) and 2-chloro-N-(3-(2-(cyclopropanesulfonamido)thiazol-4-yl)pentan-3-yl)acetamide **INTE16** (1.6 g, 4.37 mmol) in EtOH (20 mL) was treated with acetic acid (4.3 mL, 75 mmol) and heated to reflux for 18 hrs. The reaction mixture was cooled to RT, the resulting precipitate was filtered and the filtrate was loaded onto SCX (50 g), washed with MeOH and the product was isolated by eluting with 1 % $NH_3$ in MeOH to afford *N*-(4-(3-aminopentan-3-yl)thiazol-2-yl)cyclopropanesulfonamide (1.05 g, 3.27 mmol, 75 % yield) as a colourless solid. Rt 0.56 min (HPLC, basic); m/z 273.0 (M-$NH_2$+H)$^+$ (ES$^+$); $^1$H NMR (400 MHz, DMSO-$d_6$) δ 7.70 (v. br s, 3H), 6.34 (s, 1H), 2.41 - 2.29 (m, 1H), 1.80 (app. q, J = 7.4 Hz, 4H), 0.83 - 0.76 (m, 6H), 0.73 - 0.60 (m, 4H).

**Table 3:** The following intermediates were made according to Method 1a or 1b (see preparation of examples section for details).

| INT | Name/Structure (All examples containing chiral centres are racemates unless stated) | Synthesis Method, [LCMS Method], m/z (M+H)$^+$, (Rt/min) | $^1$H NMR Chemical Shift Data (DMSO-$d_6$ unless stated) |
|---|---|---|---|
| **INTE18** | 4-Bromo-*N*-(2-(2-(cyclopropanesulfonamido)thiazol-4-yl)propan-2-yl)-2-methoxybenzamide 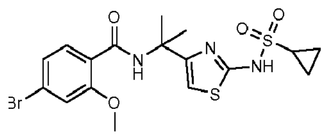 | Method 1b, Using **INTE14,** [UPLC acidic], 474 (1.27). | 12.52 (s, 1H), 8.24 (s, 1H), 7.68 (d, J = 8.3 Hz, 1H), 7.40 (d, J = 1.8 Hz, 1H), 7.26 (dd, J = 8.3, 1.8 Hz, 1 H), 6.49 (s, 1H), 3.96 (s, 3H), 2.61 - 2.54 (m, 1H), 1.61 (s, 6H), 0.94 - 0.84 (m, 4H). |

(continued)

| INT | Name/Structure (All examples containing chiral centres are racemates unless stated) | Synthesis Method, [LCMS Method], m/z $(M+H)^+$, (Rt/min) | $^1H$ NMR Chemical Shift Data (DMSO-$d_6$ unless stated) |
|---|---|---|---|
| **INTE19** | *N*-(2-(2-(Cyclopropanesulfonamido)thiazol-4-yl)propan-2-yl)-4-(4,4,5,5-tetramethyl-1,3,2-dioxaborolan-2-yl)benzamide 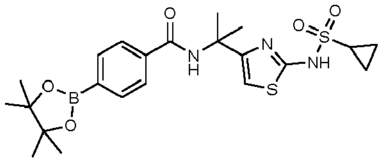 | Method 1a, Using **INTE14,** [UPLC acidic], 492 (1.38). | 12.55 (s, 1H), 8.27 (s, 1H), 7.90 - 7.85 (m, 2H), 7.76 - 7.71 (m, 2H), 6.46 (s, 1H), 2.60 - 2.52 (m, 1H), 1.61 (s, 6H), 1.31 (s, 12H), 0.92 - 0.79 (m, 4H). |
| **INTE20** | *N*-(2-(2-(Cyclopropanesulfonamido)thiazol-4-yl)propan-2-yl)-2-fluoro-4-(4,4,5,5-tetramethyl-1,3,2-dioxaborolan-2-yl) benzamide 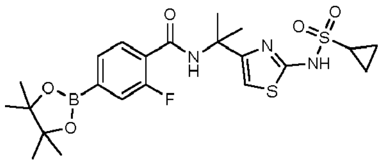 | Method 1a, Using **INTE14,** [UPLC acidic], 510 (1.44). | 12.57 (s, 1H), 8.35 - 8.27 (m, 1H), 7.78 - 7.70 (m, 1H), 7.56 - 7.50 (m, 1H), 7.43 - 7.35 (m, 1H), 6.47 (s, 1H), 2.63 - 2.54 (m, 1H), 1.60 (s, 6H), 1.31 (s, 12H), 0.93 - 0.82 (m, 4H). |

**Synthesis of biaryl acids and their intermediates**

2-Ethoxy-6-(4,4,5,5-tetramethyl-1,3,2-dioxaborolan-2-yl)pyrazine **INTF1**

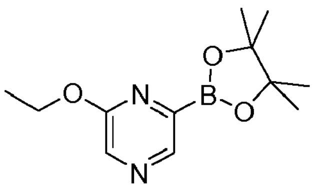

To a solution of 2-chloro-6-ethoxypyrazine (10.0 g, 59.9 mmol) in dioxane (200 mL) was successively added bispin (16.7 g, 65.9 mmol), KOAc (23.5 g, 240 mmol) and $PdCl_2(dppf)-CH_2Cl_2$ (2.45 g, 3.00 mmol) at RT. The resulting mixture was degassed ($N_2$) before heating at 110 °C for 2.5 hrs. The mixture was cooled to RT, filtered through celite and the solvent was removed *in vacuo.* The crude product was purified by chromatography on silica (220 g cartridge, 20-100 % EtOAc/*iso*-hexanes). The crude product was then dissolved in EtOAc (20 mL) and washed with water (3 x 10 mL). The organic layer was dried ($Na_2SO_4$), filtered and the solvent was removed *in vacuo.* The product was re-purified by chromatography on silica (330 g cartridge, 0-100 % EtOAc/*iso*-hexanes) and the product was dissolved in EtOAc (50 mL) and washed with water (4 x 30 mL). The organic layer was dried ($Na_2SO_4$), filtered and concentrated *in vacuo* to afford 2-ethoxy-6-(4,4,5,5-tetramethyl-1,3,2-dioxaborolan-2-yl)pyrazine (2.0 g, 7.60 mmol, 13 % yield) as a thick pale yellow oil. $^1H$ NMR (500 MHz, DMSO-$d_6$) $\delta$ 8.38 (s, 1H), 8.30 (s, 1H), 4.36 (q, J = 7.1 Hz, 2H), 1.34 (t, J = 7.1 Hz, 3H), 1.32 (s, 12H).

Methyl 4-(6-ethoxypyrazin-2-yl)-2-methylbenzoate **INTF2**

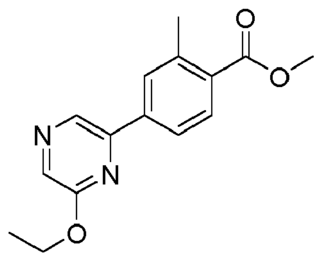

A solution of 2-ethoxy-6-(4,4,5,5-tetramethyl-1,3,2-dioxaborolan-2-yl)pyrazine **INTF1** (200 mg, 0.80 mmol), methyl 4-bromo-2-methylbenzoate (183 mg, 0.80 mmol) and $Cs_2CO_3$ (700 mg, 2.15 mmol) in a mixture of dioxane (5 mL) and water (1 mL) at 35 °C was degassed ($N_2$) then $PdCl_2$(dppf)-$CH_2Cl_2$ (35 mg, 0.043 mmol) was added. The mixture was degassed ($N_2$) then heated to 90 °C for 18 hrs. The reaction mixture was concentrated (to approx. 2 mL) then taken up with water (5 mL) and EtOAc (10 mL) and filtered through celite, eluting with EtOAc (20 mL). The phases were then diluted with water (10 mL) and partitioned. The organic phase was washed with brine (10 mL), dried ($Na_2SO_4$), filtered and concentrated onto silica (1 g). The crude product was purified by chromatography on silica (40 g cartridge, 0-30 % EtOAc/*iso*-hexanes to afford methyl 4-(6-ethoxypyrazin-2-yl)-2-methylbenzoate (124 mg, 0.45 mmol, 56 % yield) as a colourless solid. Rt 2.54 min (HPLC, acidic); m/z 273 $(M+H)^+$ $(ES^+)$; $^1H$ NMR (500 MHz, DMSO-$d_6$) $\delta$ 8.88 (s, 1H), 8.29 (s, 1H), 8.11 - 8.08 (m, 1H), 8.05 (dd, J = 8.2, 1.8 Hz, 1H), 7.95 (d, J = 8.2 Hz, 1H), 4.49 (q, J = 7.0 Hz, 2H), 3.86 (s, 3H), 2.61 (s, 3H), 1.41 (t, J = 7.0 Hz, 3H).

Methyl 2-fluoro-4-(6-vinylpyrazin-2-yl)benzoate **INTF3**

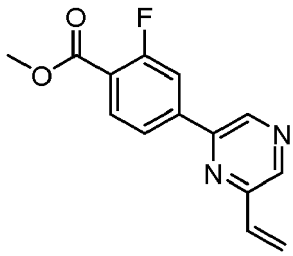

A stirred solution of potassium trifluoro(vinyl)borate (900 mg, 6.72 mmol), 2,6-dichloropyrazine (1 g, 6.71 mmol) and 2 M $K_2CO_3$ (10 mL, 20.0 mmol) in dioxane (80 mL) at 40 °C was degassed ($N_2$) then treated with $PdCl_2$(dppf)-$CH_2Cl_2$ (274 mg, 0.336 mmol), degassed ($N_2$), then heated to 80 °C for 4 hrs. The reaction mixture was cooled to RT then (3-fluoro-4-(methoxycarbonyl)phenyl)boronic acid (1.33 g, 6.71 mmol) was added and the reaction mixture was heated to 100 °C for 18 hrs. The reaction mixture was cooled and concentrated (to approx. 10 mL). 1 M HCl (100 mL) and EtOAc (100 mL) were added and the reaction mixture was filtered through celite, further eluting with EtOAc (50 mL). The phases were partitioned and the organic phase was washed with brine (50 mL). The organic phase was dried ($MgSO_4$), filtered and concentrated onto silica (5 g). The crude product was purified by chromatography on silica (40 g cartridge, 0-50 % EtOAc/*iso*-hexanes) to afford methyl 2-fluoro-4-(6-vinylpyrazin-2-yl)benzoate (490 mg, 1.803 mmol, 27 % yield) as a brown gum. Rt 2.24 min (HPLC, acidic); m/z 259 $(M+H)^+$ $(ES^+)$; $^1H$ NMR (500 MHz, DMSO-$d_6$) $\delta$ 9.27 (s, 1H), 8.83 (s, 1H), 8.19 (d, J = 1.0 Hz, 1H), 8.18 - 8.15 (m, 1H), 8.08 - 7.99 (m, 1H), 6.98 (dd, J = 17.5, 10.9 Hz, 1H), 6.56 (dd, J = 17.5, 1.3 Hz, 1H), 5.74 (dd, J = 10.9, 1.4 Hz, 1H), 3.90 (s, 3H).

Methyl 4-(6-ethylpyrazin-2-yl)-2-fluorobenzoate **INTF4**

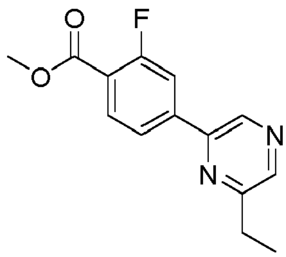

A solution of methyl 2-fluoro-4-(6-vinylpyrazin-2-yl)benzoate **INTF3** (490 mg, 1.88 mmol) in MeOH (10 mL) was prepared. The reaction mixture was hydrogenated in the H-Cube (10 % Pd/C, 30x4 mm, full hydrogen, 35 °C, 1 mL/min). The reaction mixture was concentrated to afford methyl 4-(6-ethylpyrazin-2-yl)-2-fluorobenzoate (470 mg; 1.77 mmol; 93 % yield); Rt 2.27 min (HPLC, acidic); m/z 261 $(M+H)^+$ $(ES^+)$; $^1H$ NMR (500 MHz, DMSO-$d_6$) $\delta$ 9.20 (s, 1H), 8.63 (s, 1H), 8.15 - 8.09 (m, 2H), 8.07 - 8.00 (m, 1H), 3.89 (s, 3H), 2.89 (q, J = 7.6 Hz, 2H), 1.32 (t, J = 7.6 Hz, 3H).

**Method C: Suzuki coupling**

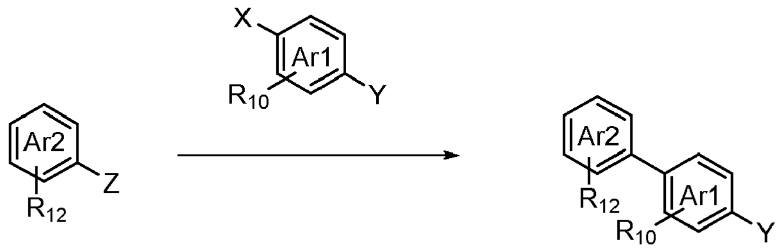

Z = Br, Cl
X = $B(OH)_2$, $B(pin)_2$

Y = $CO_2$t-Bu, $CO_2$Me, CN

A solution of boronic acid (1 eq), aryl halide (1.05 eq.) and $Cs_2CO_3$ (3 eq.) in a mixture of dioxane (40 volumes) and water (6 volumes) was degassed ($N_2$, 5 mins). $PdCl_2$(dppf).$CH_2Cl_2$ (5 mol %) was added and the reaction was further degassed ($N_2$) before being heated to 90 °C for 18 hrs. The reaction mixture was filtered through celite before an aqueous workup was undertaken, followed by purification by normal phase chromatography.

**Table 3:** The following intermediates were made according to Method C.

| INT | Name/Structure | Synthesis Method, [LCMS Method], m/z $(M+H)^+$, (Rt/min) | $^1H$ NMR Chemical Shift Data (DMSO-$d_6$ unless stated) |
|---|---|---|---|
| **INTF5** | *tert*-butyl 4-(5-methylpyridin-3-yl)benzoate 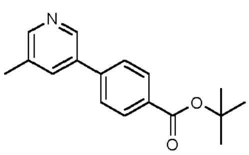 | Using Ar2Br, [HPLC acidic], 270 (1.93). | 8.75 (d, J = 2.2 Hz, 1H), 8.51 - 8.39 (m, 1H), 8.03 - 7.98 (m, 3H), 7.88 - 7.82 (m, 2H), 2.4 (s, 3H), 1.57 (s, 9H). |
| **INTF6** | *tert*-butyl 4-(5-(trifluoromethyl) pyridin-3-yl)benzoate 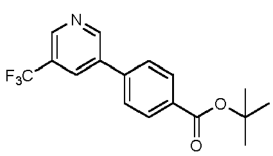 | Using Ar2Br, [HPLC acidic], 324 (2.78). | 9.27 (d, J = 2.2 Hz, 1H), 9.03 (dd, J = 2.2, 1.0 Hz, 1H), 8.61 - 8.41 (m, 1H), 8.22 - 7.83 (m, 4H), 1.58 (s, 9H). |

(continued)

| INT | Name/Structure | Synthesis Method, [LCMS Method], m/z (M+H)+, (Rt/min) | 1H NMR Chemical Shift Data (DMSO-$d_6$ unless stated) |
|---|---|---|---|
| **INTF7** | *tert*-butyl 4-(5-fluoropyridin-3-yl) benzoate 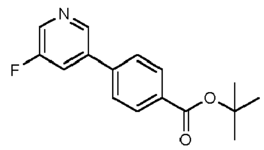 | Using Ar2Br, [HPLC acidic], 274 (2.56). | 8.87 (d, J = 1.9 Hz, 1H), 8.64 (d, J = 2.7 Hz, 1H), 8.22 - 8.09 (m, 1H), 8.06 - 7.98 (m, 2H), 7.98 - 7.89 (m, 2H), 1.57 (s, 9H). |
| **INTF8** | *tert*-butyl 4-(6-chloropyrazin-2-yl)benzoate 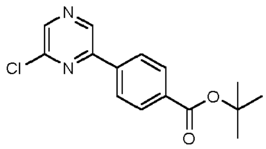 | Using Ar2Cl, [UPLC acidic], 290 $^{35}$Cl isotope, (1.53). | No $^1$H NMR recorded. |
| **INTF9** | *tert*-butyl 4-(6-(trifluoromethyl) pyrazin-2-yl)benzoate 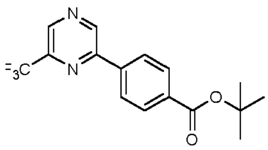 | Using Ar2Cl, [HPLC acidic], no ionisation (2.83). | 9.70 (s, 1H), 9.23 (s, 1H), 8.40 - 8.30 (m, 2H), 8.12 - 8.03 (m, 2H), 1.59 (s, 9H). |
| **INTF10** | *tert*-butyl 4-(6-methylpyrazin-2-yl)benzoate 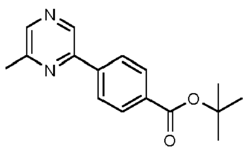 | Using Ar2Cl, [HPLC acidic], 271 (2.54). | 9.24 - 9.02 (m, 1H), 8.57 (s, 1H), 8.44 - 8.14 (m, 2H), 8.14 - 7.68 (m, 2H), 2.63 - 2.52 (m, 3H), 1.57 (s, 9H). |
| **INTF11** | *tert*-butyl 4-(6-isopropoxypyrazin-2-yl) benzoate 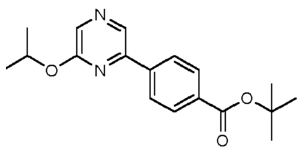 | Using Ar2Cl, [UPLC acidic], 315 (1.97). | 8.86 (s, 1H), 8.30 - 8.21 (m, 3H), 8.06 - 8.02 (m, 2H), 5.41 (hept, J = 6.2 Hz, 1H), 1.58 (s, 9H), 1.40 (d, J = 6.2 Hz, 6H). |
| **INTF12** | methyl 4-(5-chloropyridin-3-yl) benzoate 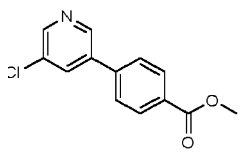 | Using Ar2Cl, [HPLC acidic], 247 $^{35}$Cl isotope, (2.20). | 8.94 (d, J = 2.0 Hz, 1H), 8.69 (d, J = 2.2 Hz, 1H), 8.35 (t, J = 2.2 Hz, 1H), 8.10 - 8.01 (m, 2H), 8.00 - 7.89 (m, 2H), 3.89 (s, 3H). |

(continued)

| INT | Name/Structure | Synthesis Method, [LCMS Method], m/z $(M+H)^+$, (Rt/min) | $^1H$ NMR Chemical Shift Data (DMSO-$d_6$ unless stated) |
|---|---|---|---|
| **INTF13** | methyl 2-fluoro-4-(5-(trifluoromethyl)pyridin-3-yl)benzoate 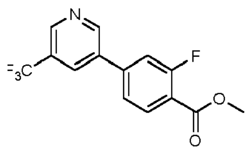 | Using Ar2Cl [HPLC acidic], 300 (2.30). | No $^1H$ NMR recorded. |
| **INTF14** | methyl 4-(5-chloropyridin-3-yl)-2-fluorobenzoate 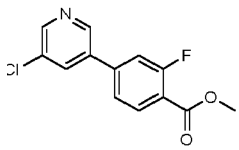 | Using Ar2Br [HPLC acidic], 266 $^{35}Cl$ isotope (2.20). | No $^1H$ NMR recorded. |
| **INTF15** | methyl 2-fluoro-4-(6-(trifluoromethyl)pyrazin-2-yl)benzoate 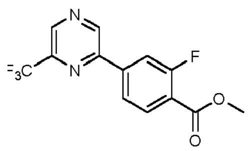 | Using Ar2Cl, [UPLC acidic], 301 (1.53). | 9.73 (s, 1H), 9.25 (s, 1H), 8.23 - 8.13 (m, 2H), 8.09 (dd, J = 8.5, 7.5 Hz, 1H), 3.90 (s, 3H). |
| **INTF16** | methyl 4-(6-ethoxypyrazin-2-yl)-2-fluorobenzoate 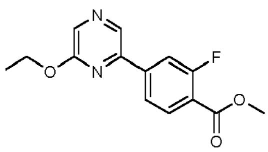 | Using Ar2Cl, [UPLC acidic], 277 (1.53). | 8.94 (s, 1H), 8.34 (s, 1H), 8.12 - 8.08 (m, 2H), 8.04 - 8.00 (m, 1H), 4.50 (q, J = 7.0 Hz, 2H), 3.89 (s, 3H), 1.41 (t, J = 7.0 Hz, 3H). |
| **INTF17** | methyl 2-fluoro-4-(6-isopropoxypyrazin-2-yl) benzoate 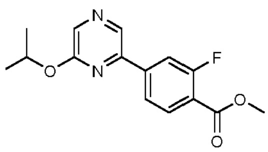 | Using Ar2Cl, [UPLC acidic], 291 (1.63). | 8.91 (s, 1H), 8.28 (s, 1H), 8.13 - 7.94 (m, 3H), 5.43 (hept, J = 6.1 Hz, 1H), 3.89 (s, 3H), 1.39 (d, J = 6.2 Hz, 6H). |
| **INTF18** | *tert*-butyl 4-(6-ethoxypyrazin-2-yl)benzoate 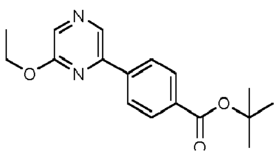 | Using Ar2Cl, [HPLC acidic], 301 (2.89). | 8.87 (s, 1H), 8.30 (s, 1H), 8.26 - 8.22 (m, 2H), 8.06 - 7.98 (m, 2H), 4.48 (q, J = 7.1 Hz, 2H), 1.57 (s, 9H), 1.40 (t, J = 7.0 Hz, 3H). |

(continued)

| INT | Name/Structure | Synthesis Method, [LCMS Method], m/z $(M+H)^+$, (Rt/min) | $^1H$ NMR Chemical Shift Data (DMSO-$d_6$ unless stated) |
|---|---|---|---|
| **INTF19** | methyl 2-fluoro-4-(6-(2,2,2-trifluoroethoxy)pyrazin-2-yl) benzoate 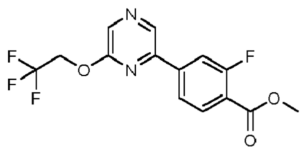 | Using Ar2Cl, [HPLC acidic], 331 (2.44). | 9.11 (s, 1H), 8.56 (s, 1H), 8.23 (dd, J = 12.3, 1.7 Hz, 1H), 8.18 (dd, J = 8.2, 1.7 Hz, 1H), 8.06 - 8.01 (m, 1H), 5.24 (q, J = 9.0 Hz, 2H), 3.90 (s, 3H). |
| **INTF20** | *tert*-butyl 4-(6-(2,2,2-trifluoroethoxy)pyrazin-2-yl) benzoate 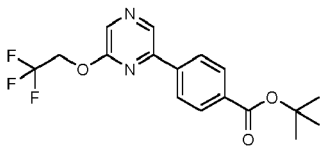 | Using Ar2Cl, [UPLC acidic], 355 (2.88) | 9.06 (s, 1H), 8.52 (s, 1H), 8.44 - 8.24 (m, 2H), 8.06 - 8.01 (m, 2H), 5.22 (q, J = 9.0 Hz, 2H), 1.58 (s, 9H). |
| **INTF21** | methyl 4-(6-ethoxypyrazin-2-yl)-2-(trifluoromethyl)benzoate 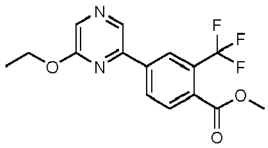 | Using Ar2Cl, [UPLC acidic], 327 (2.59) | 9.01 (d, J = 1.6 Hz, 1H), 8.56 - 8.52 (m, 2H), 8.37 (d, J = 1.6 Hz, 1H), 8.01 (d, J = 8.4 Hz, 1H), 4.58 - 4.41 (m, 2H), 3.91 (s, 3H), 1.42 (t, J = 7.0 Hz, 3H). |
| **INTF22** | methyl 2-methyl-4-(6-(trifluoromethyl)pyrazin-2-yl)benzoate 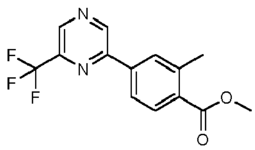 | Using Ar2Cl, [UPLC acidic], 297 (1.62) | 9.69 (s, 1H), 9.21 (s, 1H), 8.19 (d, J = 1.8 Hz, 1H), 8.14 (dd, J = 8.2, 1.8 Hz, 1H), 8.01 (d, J = 8.2 Hz, 1H), 3.88 (s, 3H), 2.64 (s, 3H). |
| **INTF23** | 5-(6-ethoxypyrazin-2-yl)-3-fluoropicolinonitrile 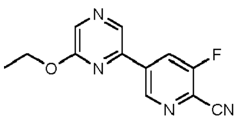 | Using Ar2Cl, [HPLC acidic], 245 (2.17) | 9.38 (t, J = 1.6 Hz, 1H), 9.05 (s, 1H), 8.76 (dd, J = 10.2, 1.6 Hz, 1H), 8.43 (s, 1H), 4.53 (q, J = 7.0 Hz, 2H), 1.41 (t, J = 7.0 Hz, 3H). |

**Method D: Ester deprotection with TFA**

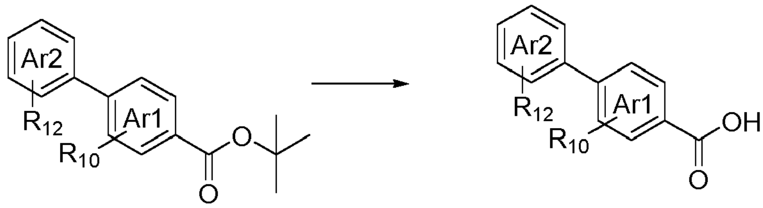

A solution of the ester (1 eq) in DCM (20 volumes) was treated with TFA (10 eq.) and stirred at RT for 3 hrs. The reaction mixture was then concentrated and azeotroped with MeOH and MeCN. No further purification was undertaken.

**Method E: Ester deprotection with base**

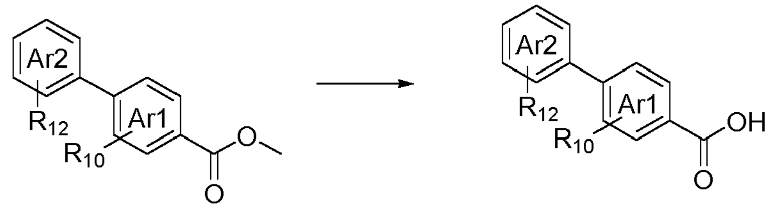

A solution of the ester (1 eq) in a mixture of THF/MeOH (4/1 volumes) was treated with LiOH (2.2-6 eq.) and stirred between RT and 50 °C for between 3 hrs and 18hrs. The organic solvents were removed *in vacuo* then acidified with 1 M HCl and extracted with EtOAc. The organic phases were combined, dried ($Na_2SO_4$), filtered and concentrated. The products were used directly in the next step with no further purification undertaken.

**Method F: Potassium salt formation**

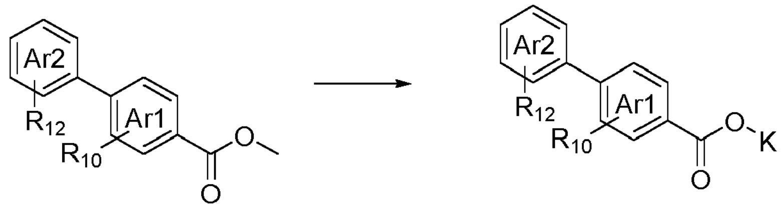

A solution of the ester (1 eq.) in THF (4 volumes) was treated with TMSOK (1 eq.) and stirred at RT for 2 hrs before the reaction mixtures were filtered and washed with *iso*-hexanes. The products were used directly in the next step with no further purification undertaken.

**Table 4:** The following intermediates were made according to Method D, E or F.

| INT | Name/Structure | Synthesis Method, [LCMS Method], m/z $(M+H)^+$, (Rt/min) | $^1H$ NMR Chemical Shift Data (DMSO-$d_6$ unless stated) |
|---|---|---|---|
| **INTF24** | 4-(5-methylpyridin-3-yl) benzoic acid 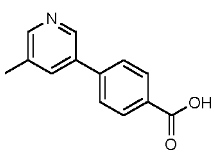 | Method D, Using **INTF5**, [HPLC acidic], 214 (0.95). | 9.02 - 8.94 (m, 1H), 8.70 - 8.63 (m, 1H), 8.47 (s, 1H), 8.14 - 8.06 (m, 2H), 8.00 - 7.90 (m, 2H), 2.49 (s, 3H), O-H not observed. |
| **INTF25** | 4-(5-(trifluoromethyl)pyridin-3-yl)benzoic acid 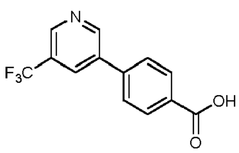 | Method D, Using **INTF6**, [HPLC acidic], 268 (2.01). | 13.12 (s, 1H), 9.28 (d, J = 2.2 Hz, 1H), 9.03 (dd, J = 2.2, 1.0 Hz, 1H), 8.56 (d, J = 2.2 Hz, 1H), 8.13 - 8.04 (m, 2H), 8.04 - 7.86 (m, 2H). |

(continued)

| INT | Name/Structure | Synthesis Method, [LCMS Method], m/z (M+H)$^+$, (Rt/min) | $^1$H NMR Chemical Shift Data (DMSO-$d_6$ unless stated) |
|---|---|---|---|
| **INTF26** | 4-(5-fluoropyridin-3-yl) benzoic acid 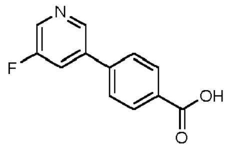 | Method D, Using **INTF7,** [HPLC acidic], 218 (1.67). | 8.87 (t, J = 1.8 Hz, 1H), 8.64 (d, J = 2.7 Hz, 1H), 8.17 (ddd, J = 10.3, 2.7, 1.8 Hz, 1H), 8.07 - 8.03 (m, 2H), 7.97 - 7.90 (m, 2H), OH not observed. |
| **INTF27** | 4-(6-chloropyrazin-2-yl) benzoic acid 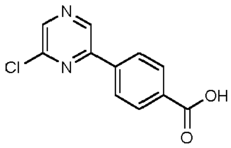 | Method D, Using **INTF8,** [HPLC acidic], 234 $^{35}$Cl isotope, (1.91). | No NMR recorded. |
| **INTF28** | 4-(6-(trifluoromethyl)pyrazin-2-yl)benzoic acid 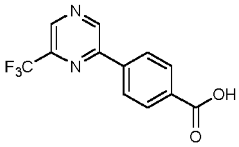 | Method D, Using **INTF9,** [UPLC acidic], 269 (1.33). | 13.25 (s, 1H), 9.70 (s, 1H), 9.23 (s, 1H), 8.42 - 8.20 (m, 2H), 8.20 - 8.00 (m, 2H). |
| **INTF29** | 4-(6-methylpyrazin-2-yl) benzoic acid 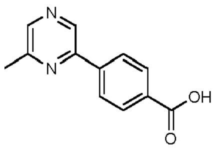 | Method D, Using **INTF10,** [HPLC acidic], 215 (1.60). | 9.13 (s, 1H), 8.57 (s, 1H), 8.31 - 8.23 (m, 2H), 8.09 - 8.04 (m, 2H), 2.59 (s, 3H), OH not observed. |
| **INTF30** | 4-(6-isopropoxypyrazin-2-yl) benzoic acid 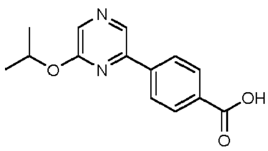 | Method D, Using **INTF11,** [UPLC acidic], 259 (1.40). | 13.13 (s, 1H) 8.87 (s, 1H), 8.27 - 8.20 (m, 3H), 8.09 - 8.05 (m, 2H), 5.43 (p, J = 6.2 Hz, 1H), 1.40 (d, J = 6.2 Hz, 6H). |
| **INTF31** | potassium 4-(5-chloropyridin-3-yl)benzoate 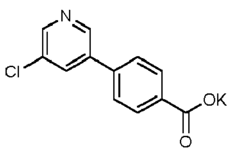 | Method F, Using **INTF12,** [UPLC acidic], 234 $^{35}$Cl isotope, ionises as free acid, (1.18). | 8.87 (d, J = 2.0 Hz, 1H), 8.59 (d, J = 2.2 Hz, 1H), 8.23 (t, J = 2.2 Hz, 1H), 7.95 - 7.86 (m, 2H), 7.72 - 7.55 (m, 2H). |

(continued)

| INT | Name/Structure | Synthesis Method, [LCMS Method], m/z $(M+H)^+$, (Rt/min) | $^1H$ NMR Chemical Shift Data (DMSO-$d_6$ unless stated) |
|---|---|---|---|
| **INTF32** | potassium 2-fluoro-4-(5-(trifluoromethyl)pyridin-3-yl)benzoate 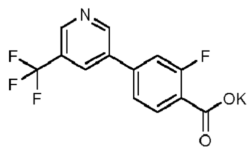 | Method F, Using **INTF13,** [HPLC acidic], 286 ionises as free acid, (2.01). | 9.22 (d, J = 2.2 Hz, 1H), 8.95 (d, J = 2.1 Hz, 1H), 8.48 (d, J = 2.3 Hz, 1H), 7.64 - 7.45 (m, 3H). |
| **INTF33** | potassium 4-(5-chloropyridin-3-yl)-2-fluorobenzoate 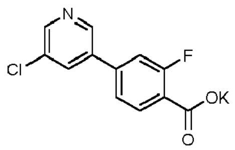 | Method F, Using **INTF14,** [HPLC acidic], 251 $^{35}Cl$ isotope, ionises as free acid, (1.88). | 8.91 - 8.85 (m, 1H), 8.63 - 8.54 (m, 1H), 8.30 - 8.20 (m, 1H), 7.59 - 7.49 (m, 1H), 7.49 - 7.34 (m, 2H). |
| **INTF34** | 2-fluoro-4-(6-(trifluoromethyl) pyrazin-2-yl)benzoic acid 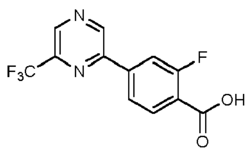 | Method E, Using **INTF15,** [HPLC acidic], 287 (2.08). | 13.53 (s, 1H), 9.72 (s, 1H), 9.25 (s, 1H), 8.19 - 8.12 (m, 2H), 8.11 - 7.99 (m, 1H). |
| **INTF35** | 4-(6-ethoxypyrazin-2-yl)-2-fluorobenzoic acid 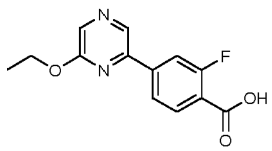 | Method E, Using **INTF16,** [HPLC acidic], 263 (2.07). | 13.40 (s, 1H), 8.94 (s, 1H), 8.34 (s, 1H), 8.12 - 8.03 (m, 2H), 8.03 - 7.92 (m, 1H), 4.50 (q, J = 7.0 Hz, 2H), 1.41 (t, J = 7.0 Hz, 3H). |
| **INTF36** | 2-fluoro-4-(6-isopropoxypyrazin-2-yl) benzoic acid 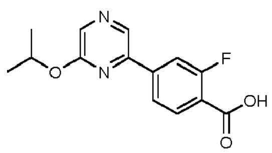 | Method E, Using **INTF17,** [HPLC acidic], 277 (2.24). | 13.53 (s, 1H), 8.90 (s, 1H), 8.27 (s, 1H), 8.08 - 7.87 (m, 3H), 5.43 (hept, J = 6.2 Hz, 1H), 1.39 (d, J = 6.2 Hz, 6H). |
| **INTF37** | 4-(6-ethoxypyrazin-2-yl) benzoic acid 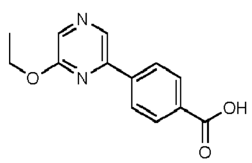 | Method D, Using **INTF18,** [UPLC acidic], 245 (1.29) | 13.15 (v. br. s, 1H), 8.89 (s, 1H), 8.31 (s, 1H), 8.29 - 8.22 (m, 2H), 8.11 - 8.01 (m, 2H), 4.51 (q, J = 7.0 Hz, 2H), 1.42 (t, J = 7.0 Hz, 3H). |

(continued)

| INT | Name/Structure | Synthesis Method, [LCMS Method], m/z $(M+H)^+$, (Rt/min) | $^1H$ NMR Chemical Shift Data (DMSO-$d_6$ unless stated) |
|---|---|---|---|
| **INTF38** | 4-(6-ethoxypyrazin-2-yl)-2-methylbenzoic acid 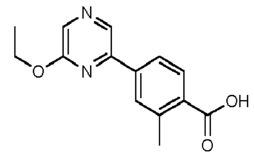 | Method E, Using **INTF2,** [HPLC acidic], 259 (2.17) | 12.97 (s, 1H), 8.87 (s, 1H), 8.29 (s, 1H), 8.07 - 8.05 (m, 1H), 8.03 (dd, J = 8.2, 1.9 Hz, 1H), 7.94 (d, J = 8.2 Hz, 1H), 4.49 (q, J = 7.0 Hz, 2H), 2.62 (s, 3H), 1.41 (t, J = 7.0 Hz, 3H). |
| **INTF39** | 4-(6-ethylpyrazin-2-yl)-2-fluorobenzoic acid 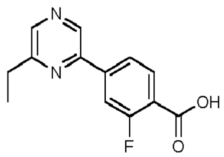 | Method E, Using **INTF4,** [HPLC acidic], 247 (1.91) | 13.40 (s, 1H), 9.19 (s, 1H), 8.63 (s, 1H), 8.13 - 8.05 (m, 2H), 8.01 (t, J = 7.9 Hz, 1H), 2.89 (q, J = 7.6 Hz, 2H), 1.32 (t, J = 7.6 Hz, 3H). |
| **INTF40** | 2-fluoro-4-(6-(2,2,2-trifluoro ethoxy)pyrazin-2-yl)benzoic acid 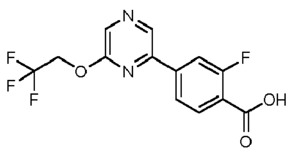 | Method E, Using **INTF19,** [UPLC acidic], 317 (1.38) | 13.44 (s, 1H), 9.10 (s, 1H), 8.55 (s, 1H), 8.23 - 8.12 (m, 2H), 8.03 - 7.95 (m, 1H), 5.24 (q, J = 9.0 Hz, 2H). |
| **INTF41** | 4-(6-(2,2,2-trifluoroethoxy) pyrazin-2-yl)benzoic acid 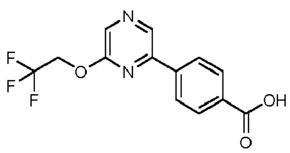 | Method D, Using **INTF20** [UPLC acidic], 299 (1.37) | 13.16 (s, 1H), 9.06 (s, 1H), 8.52 (s, 1H), 8.41 - 8.27 (m, 2H), 8.13 - 8.03 (m, 2H), 5.22 (q, J = 9.0 Hz, 2H). |
| **INTF42** | 4-(6-ethoxypyrazin-2-yl)-2-(trifluoromethyl)benzoic acid 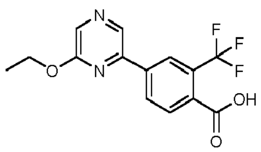 | Method E, Using **INTF21,** [UPLC acidic], 313 (2.30) | 13.75 (s, 1H), 8.98 (s, 1H), 8.52 - 8.46 (m, 2H), 8.35 (s, 1H), 7.97 (d, J = 7.9 Hz, 1H), 4.49 (q, J = 7.0 Hz, 2H), 1.42 (t, J = 7.0 Hz, 3H). |
| **INTF43** | 2-methyl-4-(6-(trifluoromethyl)pyrazin-2-yl)benzoic acid 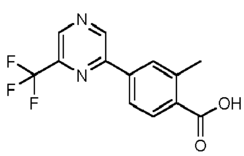 | Method E, Using **INTF22,** [HPLC acidic], 283 (2.20) | 13.06 (s, 1H), 9.66 (s, 1H), 9.18 (s, 1H), 8.14 - 8.11 (m, 1H), 8.09 (dd, J = 8.1, 1.9 Hz, 1H), 7.99 (d, J = 8.1 Hz, 1H), 2.63 (s, 3H). |

5-(6-Ethoxypyrazin-2-yl)-3-fluoropicolinic acid **INTF44**

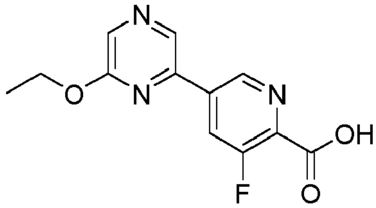

A suspension of 5-(6-ethoxypyrazin-2-yl)-3-fluoropicolinonitrile **INTF23** (630 mg, 2.58 mmol) in conc. HCl (10 mL) was heated to reflux for 1 hr. The reaction mixture was allowed to cool to RT. This was then concentrated *in vacuo,* then suspended in TBME (20 mL) and filtered, washing with iso-hexanes (10 mL) to afford 5-(6-ethoxypyrazin-2-yl)-3-fluoropicolinic acid (750 mg, 2.28 mmol, 88 % yield) as an orange solid. Rt 1.74 min (HPLC, acidic); m/z 264 $(M+H)^+$ $(ES^+)$; $^1H$ NMR (500 MHz, DMSO-$d_6$) δ 9.26 (t, J = 1.6 Hz, 1H), 9.02 (s, 1H), 8.54 (dd, J = 11.5, 1.6 Hz, 1H), 8.39 (s, 1H), 4.52 (q, J = 7.0 Hz, 2H), 1.41 (t, J = 7.0 Hz, 3H), O-H not observed.

(5-(6-(Trifluoromethyl)pyrazin-2-yl)pyridin-2-yl)methanol **INTF45**

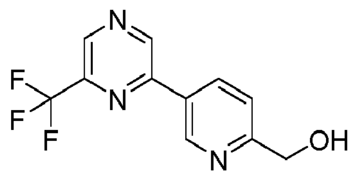

A suspension of (5-bromopyridin-2-yl)methanol (1 g, 5.32 mmol), Bispin (1.5 g, 5.91 mmol) and KOAc (1.6 g, 16.0 mmol) in dioxane (20 mL) was heated to 30 °C then degassed ($N_2$). $PdCl_2$(dppf)-$CH_2Cl_2$ (0.217 g, 0.266 mmol) was added and the reaction mixture heated to 90 °C for 2 hrs. The reaction mixture was cooled to 40 °C whereupon 2-chloro-6-(trifluoromethyl)pyrazine (0.97 g, 5.32 mmol), $Cs_2CO_3$ (3.47 g, 10.6 mmol) and water (5 mL) were added. The mixture was degassed ($N_2$), then $PdCl_2$(dppf)-$CH_2Cl_2$ (0.217 g, 0.266 mmol) was added and the mixture was again degassed ($N_2$). The reaction mixture was then heated to 90 °C for 18 hrs. The reaction mixture was part concentrated (to approx. 5 mL) then taken up with water (20 mL) and EtOAc (50 mL) and passed through celite, eluting with EtOAc (20 mL). The phases were then diluted with water (20 mL) and partitioned. The organic phase was washed with brine (30 mL), dried ($Na_2SO_4$), filtered and concentrated onto silica (5 g). The crude product was purified by chromatography on silica (40 g cartridge, 0-100% EtOAc/*iso*-hexanes) to afford (5-(6-(trifluoromethyl)pyrazin-2-yl)pyridin-2-yl)methanol (980 mg, 3.76 mmol, 71 % yield) as an off-white solid. Rt 1.38 min (HPLC, acidic); m/z 256 $(M+H)^+$ $(ES^+)$; $^1H$ NMR (500 MHz, DMSO-$d_6$) δ 9.69 (s, 1H), 9.28 (d, J = 2.3 Hz, 1H), 9.20 (s, 1H), 8.56 (dd, J = 8.2, 2.3 Hz, 1H), 7.70 (d, J = 8.2 Hz, 1H), 5.59 (t, J = 5.8 Hz, 1H), 4.67 (d, J = 5.8 Hz, 2H).

(5-(6-Chloropyrazin-2-yl)pyridin-2-yl)methanol **INTF46**

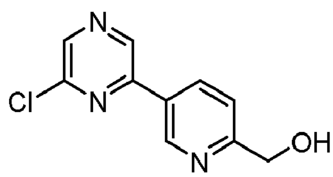

Prepared as for **INTF45** using (5-bromopyridin-2-yl)methanol and 2,6-dichloropyrazine to afford (5-(6-chloropyrazin-2-yl)pyridin-2-yl)methanol (27 % yield) as a brown solid. Rt 1.10 min (HPLC, acidic); m/z 222 $(^{35}Cl\ M+H)^+$ $(ES^+)$; $^1H$ NMR (500 MHz, DMSO-$d_6$) δ 9.36 (s, 1H), 9.22 (s, 1H), 8.81 (s, 1H), 8.50 (d, J = 8.2 Hz, 1H), 7.66 (d, J = 8.2 Hz,

1H), 5.57 (t, J = 6.0 Hz, 1H), 4.66 (d, J = 6.0 Hz, 2H).

(5-(6-Ethoxypyrazin-2-yl)pyridin-2-yl)methanol **INTF47**

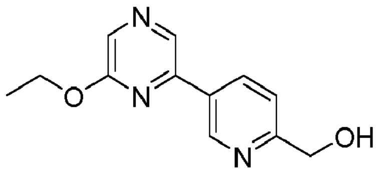

Prepared as for **INTF45** using (5-bromopyridin-2-yl)methanol and 2-chloro-6-ethoxypyrazine to afford (5-(6-ethoxypyrazin-2-yl)pyridin-2-yl)methanol (54 % yield) as a brown solid. Rt 1.34 min (HPLC, acidic); m/z 232 $(M+H)^+$ $(ES^+)$; $^1H$ NMR (500 MHz, DMSO-$d_6$) δ 9.27 - 9.09 (m, 1H), 8.87 (s, 1H), 8.49 (dd, J = 8.2, 2.3 Hz, 1H), 8.29 (s, 1H), 7.62 (d, J = 8.2 Hz, 1H), 5.53 (t, J = 5.9 Hz, 1H), 4.64 (d, J = 5.9 Hz, 2H), 4.50 (q, J = 7.1 Hz, 2H), 1.41 (t, J = 7.1 Hz, 3H).

5-(6-(Trifluoromethyl)pyrazin-2-yl)picolinaldehyde **INTF48**

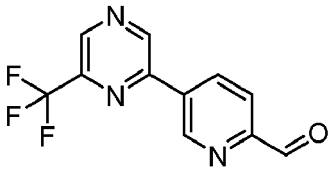

A solution of (5-(6-(trifluoromethyl)pyrazin-2-yl)pyridin-2-yl)methanol **INTF45** (980 mg, 3.84 mmol) in $CH_2Cl_2$ (15 mL) was treated with manganese dioxide (3 g, 34.5 mmol). The reaction was stirred for 4 hrs at RT then filtered through celite and concentrated onto silica (4 g). The crude product was purified by chromatography on silica (24 g cartridge, 0-100 % EtOAc/*iso*-hexanes) to afford 5-(6-(trifluoromethyl)pyrazin-2-yl)picolinaldehyde (541 mg, 2.04 mmol, 55 % yield) as a colourless solid. Rt 1.82 min (HPLC, acidic); m/z 254 $(M+H)^+$ $(ES^+)$; $^1H$ NMR (500 MHz, DMSO-$d_6$) δ 10.09 (s, 1H), 9.82 (s, 1H), 9.60 (dd, J = 2.2, 0.8 Hz, 1H), 9.30 (s, 1H), 8.79 (dd, J = 8.1, 2.2 Hz, 1H), 8.14 (dd, J = 8.1, 0.8 Hz, 1H).

5-(6-Chloropyrazin-2-yl)picolinaldehyde **INTF49**

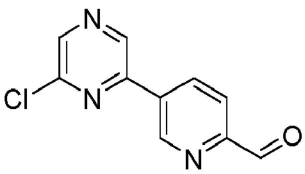

Prepared as for **INTF48** using (5-(6-chloropyrazin-2-yl)pyridin-2-yl)methanol **INTF46** to afford 5-(6-chloropyrazin-2-yl)picolinaldehyde (39 % yield) as a colourless solid. Rt 1.63 min (HPLC, acidic); m/z 220 $(^{35}Cl\ M+H)^+$ $(ES^+)$; $^1H$ NMR (500 MHz, DMSO-$d_6$) δ 10.08 (d, J = 0.8 Hz, 1H), 9.55 (dd, J = 2.2, 0.9 Hz, 1H), 9.50 (s, 1H), 8.92 (s, 1H), 8.74 (ddd, J = 8.1, 2.2, 0.9 Hz, 1H), 8.11 (dd, J = 8.1, 0.9 Hz, 1H).

5-(6-Ethoxypyrazin-2-yl)picolinaldehyde **INTF50**

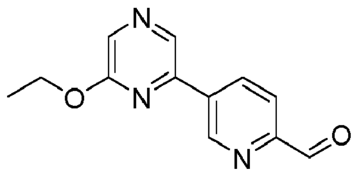

Prepared as for **INTF48** using (5-(6-ethoxypyrazin-2-yl)pyridin-2-yl)methanol **INTF47** to afford 5-(6-ethoxypyrazin-2-yl)picolinaldehyde (42 % yield) as a colourless solid. Rt 1.85 min (HPLC, acidic); m/z 230 $(M+H)^+$ $(ES^+)$; $^1H$ NMR (500 MHz, DMSO-$d_6$) δ 10.07 (d, J = 0.8 Hz, 1H), 9.55 (dd, J = 2.2, 0.9 Hz, 1H), 9.03 (s, 1H), 8.73 (ddd, J = 8.1, 2.2, 0.8 Hz, 1H), 8.39 (s, 1H), 8.08 (dd, J = 8.1, 0.9 Hz, 1H), 4.53 (q, J = 7.0 Hz, 2H), 1.42 (t, J = 7.0 Hz, 3H).

5-(6-(Trifluoromethyl)pyrazin-2-yl)picolinic acid **INTF51**

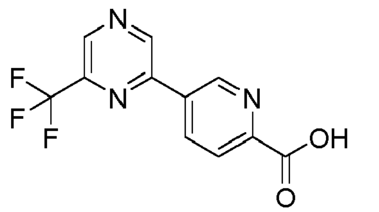

A solution of 5-(6-(trifluoromethyl)pyrazin-2-yl)picolinaldehyde **INTF48** (200 mg, 0.79 mmol) in DMF (2 mL) was treated with oxone (650 mg, 1.06 mmol). The reaction mixture was stirred at RT for 4 days. The reaction mixture was diluted with water (10 mL) and filtered. The filtrate was then taken up in EtOAc (10 mL) and heated to 40 °C to afford a free flowing suspension. This was then treated dropwise with iso-hexanes (10 mL), cooled to RT and filtered to afford 5-(6-(trifluoromethyl)pyrazin-2-yl)picolinic acid (180 mg, 0.56 mmol, 68 % yield) as a colourless solid. $^1H$ NMR (500 MHz, DMSO-$d_6$) δ 9.78 (s, 1H), 9.48 (dd, J = 2.4, 0.8 Hz, 1H), 9.28 (s, 1H), 8.72 (dd, J = 8.2, 2.3 Hz, 1H), 8.24 (dd, J = 8.2, 0.8 Hz, 1H), v. br OH observed, not reported.

5-(6-chloropyrazin-2-yl)picolinic acid **INTF52**

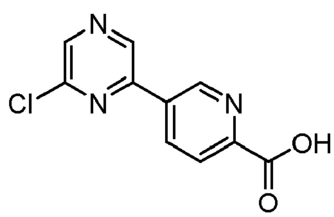

Prepared as for **INTF51** using 5-(6-chloropyrazin-2-yl)picolinaldehyde **INTF49** to afford 5-(6-chloropyrazin-2-yl)picolinic acid (82 % yield) as a colourless solid. Rt 1.32 min (HPLC, acidic); m/z 236 $(^{35}Cl\ M+H)^+$ $(ES^+)$; $^1H$ NMR (500 MHz, DMSO-$d_6$) δ 13.30 (s, 1H), 9.46 (s, 1H), 9.42 (d, J = 2.1 Hz, 1H), 8.90 (s, 1H), 8.66 (dd, J = 8.2, 2.1 Hz, 1H), 8.21 (d, J = 8.2 Hz, 1H).

5-(6-ethoxypyrazin-2-yl)picolinic acid **INTF53**

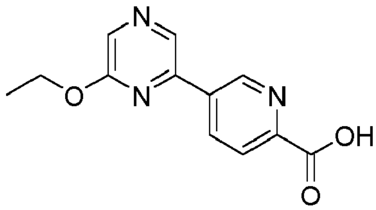

Prepared as for **INTF51** using 5-(6-ethoxypyrazin-2-yl)picolinaldehyde **INTF50** to afford 5-(6-ethoxypyrazin-2-yl)picolinic acid (71 % yield) as a colourless solid. Rt 1.45 min (HPLC, acidic); m/z 246 $(M+H)^+$ $(ES^+)$; $^1H$ NMR (500 MHz, DMSO-$d_6$) δ 13.31 (s, 1H), 9.46 - 9.38 (m, 1H), 8.98 (s, 1H), 8.64 (dd, J = 8.1, 2.3 Hz, 1H), 8.36 (s, 1H), 8.17 (dd, J = 8.1, 0.8 Hz, 1H), 4.51 (q, J = 7.0 Hz, 2H), 1.42 (t, J = 7.0 Hz, 3H).

**Preparation of examples**

**Method 1: Amide coupling**

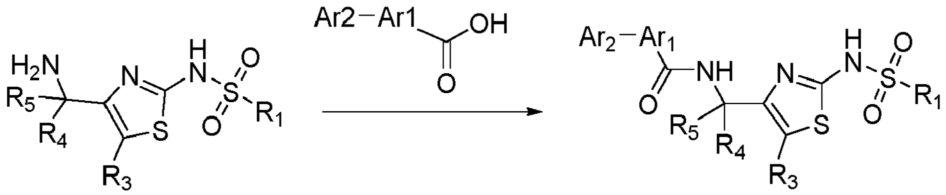

**Method 1a:** HATU (1.2 eq.) was added to a solution of appropriate acid (1 eq.), amine (1 eq.) and DIPEA (3 eq.) in DMF (10 volumes) at RT. The reaction was stirred at RT for 18 hrs. The solvent was removed and the crude product was purified by normal phase chromatography, reverse phase chromatography or trituration from an appropriate solvent.

**Method 1b:** 1-chloro-*N,N*,2-trimethylprop-1-en-1-amine (2 eq.) was added to a solution of appropriate acid (1 eq.) in DCM (20 volumes). The reaction mixture was stirred at RT for 2 hrs. The reaction mixture was concentrated *in vacuo* and the residue dissolved in DCM (20 volumes) before addition of DIPEA (3 eq.) and the appropriate amine (1 eq). The reaction mixture was stirred at RT for 2 hrs. An aqueous work up was performed and the crude product was purified by normal phase chromatography, reverse phase chromatography or trituration from an appropriate solvent.

**Method 1c:** T3P (50wt% in EtOAc, 2.5 eq.) was added to a solution of appropriate acid (1 eq.), amine (1 eq.) and pyridine (3 eq.) in a mixture of EtOAc (20 volumes) and DMF (10 volumes). The reaction was stirred for 1 hr at RT. An aqueous work up was performed and the crude product was purified by normal phase chromatography, reverse phase chromatography or trituration from an appropriate solvent.

**Method 2a: Suzuki [ArB$(OR)_2$ core]**

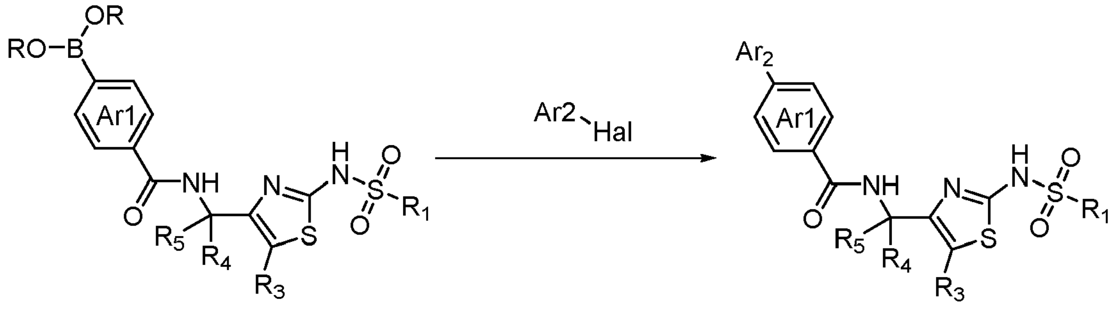

$PdCl_2$(dppf)-$CH_2Cl_2$ (10 mol %) or other appropriate catalyst was added to a degassed ($N_2$, 5 mins) solution of Ar1-B$(OR)_2$ (1 eq.), Ar2-halide (1 eq.) and $K_2CO_3$ (3 eq.) in dioxane (10 volumes) and water (1 volumes). The solution

was then degassed further ($N_2$, 5 mins) and heated to 90 °C for 1-2 hrs. The reaction mixture was allowed to cool to RT. An aqueous workup was performed and the crude product was purified by normal phase chromatography, reverse phase chromatography or trituration from an appropriate solvent.

**Method 2b: Telescoped Miyaura Borylation/Suzuki Protocol**

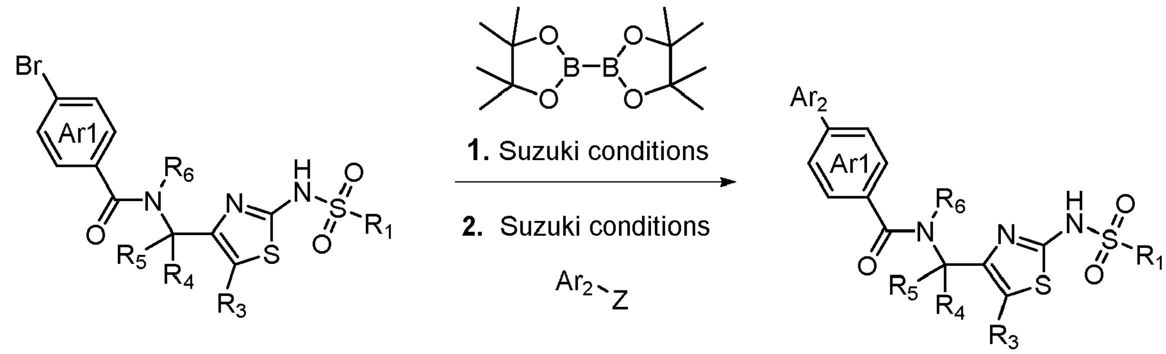


Z = Br, Cl

A suspension of Ar1-Br (1 eq.), Bispin (1.1 eq.) and KOAc (2 eq.) in dioxane (50 volumes) was degassed ($N_2$) then charged with $PdCl_2$(dppf).$CH_2Cl_2$ (5 mol %) and again degassed ($N_2$). The reaction mixture was heated to 90 °C for 1-24 hrs, recharging the Pd-catalyst if required. On formation of the boronate ester the reaction was allowed to cool to RT. Ar2-Z (1 eq.) and 2 M $K_2CO_3$ (aq, 2 eq.) were added, degassed ($N_2$) and the reaction was then heated to 90 °C for 18 hrs. The reaction was allowed to cool to RT, an aqueous work up was performed and the crude compound was purified by normal phase chromatography.

**Representative for Method 1a**

*N*-((2-(cyclopropanesulfonamido)thiazol-4-yl)methyl)-4-(pyridin-3-yl)benzamide **R1**

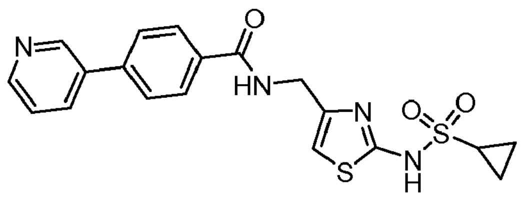

A solution of *N*-(4-(aminomethyl)thiazol-2-yl)cyclopropanesulfonamide **INTE9** (64 mg, 0.274 mmol), 4-(pyridin-3-yl)benzoic acid (54.6 mg, 0.274 mmol) and DIPEA (0.14 mL, 0.82 mmol) in DMF (0.5 mL) was treated with HATU (110 mg, 0.288 mmol) and stirred at RT for 18 hrs. EtOAc (20 mL) was added and the organic phase was washed with water (10 mL) and brine (10 mL), dried ($Na_2SO_4$), filtered and concentrated onto silica (300 mg). The crude product was purified by chromatography on silica (12 g column, 0-7 % (0.7 M ammonia/MeOH)/DCM). The crude product was further purified by reverse phase chromatography on C18 silica (12 g column, 10-40 % MeCN/water 0.1 % formic acid) to afford *N*-((2-(cyclopropanesulfonamido)thiazol-4-yl)methyl)-4-(pyridin-3-yl)benzamide (18 mg, 0.041 mmol, 15 % yield) as a colourless solid. Rt 1.08 min (HPLC, HPLC Acidic); m/z 415 $(M+H)^+$ $(ES^+)$; $^1H$ NMR (400 MHz, DMSO-$d_6$) δ 12.57 (s, 1H), 9.04 - 8.94 (m, 2H), 8.62 (dd, J = 4.8, 1.6 Hz, 1H), 8.17-8.14 (m, 1H), 8.06 - 7.98 (m, 2H), 7.92 - 7.84 (m, 2H), 7.50-7.48 (m, 1H), 6.53 (s, 1H), 4.35-4.33 (m, 2H), 2.64 - 2.52 (m, 1H), 0.09-0.87 (m, 4H).

**Representative for Method 1b**

N-(1-(2-(cyclopropanesulfonamido)thiazol-4-yl)propyl)-4-(5-(trifluoromethyl)pyridin-3-yl)benzamide **R2**

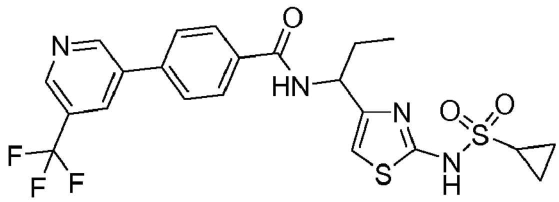

A suspension of 4-(5-(trifluoromethyl)pyridin-3-yl)benzoic acid **INTF25** (46.2 mg, 0.173 mmol) in DCM (2 mL) was treated with 1-chloro-*N,N*,2-trimethylprop-1-en-1-amine (0.046 mL, 0.346 mmol) and stirred at RT for 1 hr before being concentrated. The residue was taken up in DCM (3 mL), treated with DIPEA (0.091 mL, 0.518 mmol) and stirred for 5 mins before *N*-(4-(1-aminopropyl)thiazol-2-yl)cyclopropanesulfonamide **INTE10** (45 mg, 0.17 mmol) was added and the reaction mixture was stirred at RT for 16 hrs. The reaction mixture was treated with $NH_4Cl$ (sat. aq., 5 mL) and passed through a phase separator, further extracting with DCM (5 mL). The organic phase was concentrated onto silica (500 mg) and the crude product was purified by chromatography on silica [12 g column, 0-100 % EtOAc (2 % MeOH)/*iso*-hexanes] to afford N-(1-(2-(cyclopropanesulfonamido)thiazol-4-yl)propyl)-4-(5-(trifluoromethyl)pyridin-3-yl)benzamide (46 mg, 0.085 mmol, 49 % yield) as a colourless solid. Rt 1.99 min (HPLC, acidic); m/z 511 $(M+H)^+$ $(ES^+)$; $^1H$ NMR (400 MHz, DMSO-d6) δ 12.60 (s, 1H), 9.30 (d, J = 2.1 Hz, 1H), 9.03 (d, J = 2.1 Hz, 1H), 8.74 (d, J = 8.3 Hz, 1H), 8.56 (s, 1H), 8.09 - 7.97 (m, 4H), 6.56 (s, 1H), 4.90 (q, J = 7.9 Hz, 1H), 2.64 - 2.51 (m, 1H), 1.98 - 1.74 (m, 2H), 0.98 - 0.81 (m, 7H).

The racemic mixture **R2** was separated by chiral preparative HPLC using **chiral method A.** A salt exchange (TFA to HCl) was undertaken by adding 1.25 M HCl (EtOH, 2 mL x 5) and removing solvent to afford:

**Peak 1: Stereochemistry of product was not defined R3**

*N*-(1-(2-(cyclopropanesulfonamido)thiazol-4-yl)propyl)-4-(5-(trifluoromethyl)pyridin-3-yl)benzamide. HCl **R3** (13 mg, 0.023 mmol, 37 % yield) was isolated as a colourless solid. Rt = 1.30 min (UPLC, acidic); m/z 511 $(M+H)^+$ $(ES^+)$; $^1H$ NMR (500 MHz, DMSO-$d_6$) δ 12.61 (s, 1H), 9.30 (s, 1H), 9.03 (d, J = 2.2 Hz, 1H), 8.75 (d, J = 8.3 Hz, 1H), 8.56 (s, 1H), 8.09 - 8.04 (m, 2H), 8.03 - 7.98 (m, 2H), 6.57 (s, 1H), 4.94 - 4.87 (m, 1H), 2.64-2.51 (m, 1H), 1.97 - 1.74 (m, 2H), 0.96 - 0.85 (m, 7H). Signal for HCl not observed

The product was analysed by Chiral HPLC, **chiral IA method 1:** [Daicel Chiralpak IA, 5 um, 4.6x250 mm, 45 min method, 1.0 mL/min, 5-95 % (gradient over 45 min) EtOH (0.2 % TFA) in iso-hexanes (0.2 % TFA)]; Rt = 14.8 min, >98 % @ 254 nm.

**Peak 2: Stereochemistry of product was not defined R4**

*N*-(1-(2-(cyclopropanesulfonamido)thiazol-4-yl)propyl)-4-(5-(trifluoromethyl)pyridin-3-yl)benzamide. HCl **R4** (12 mg, 0.021 mmol, 34 % yield) was obtained as a colourless solid. Rt = 1.30 min (UPLC, acidic); ); m/z 511 $(M+H)^+$ $(ES^+)$; $^1H$ NMR (500 MHz, DMSO-$d_6$) δ 12.61 (s, 1H), 9.30 (d, J = 2.2 Hz, 1H), 9.03 (d, J = 2.1 Hz, 1H), 8.75 (d, J = 8.1 Hz, 1H), 8.56 (s, 1H), 8.08 - 8.04 (m, 2H), 8.03 - 7.99 (m, 2H), 6.57 (s, 1H), 4.95 - 4.85 (m, 1H), 2.64-2.51 (m, 1H), 1.97 - 1.73 (m, 2H), 0.97 - 0.82 (m, 7H). Signal for HCl not observed

The product was analysed by Chiral HPLC, **chiral IA method 1:** [Daicel Chiralpak IA, 5 um, 4.6x250 mm, 45 min method, 1.0 mL/min, 5-95 % (gradient over 45 min) EtOH (0.2 % TFA) in iso-hexanes (0.2 % TFA)]; Rt = 16.8 min, >98 % @ 254 nm.

**Representative for Method 1c**

*N*-(1-(2-(cyclopropanesulfonamido)thiazol-4-yl)propyl)-4-(6-(trifluoromethyl)pyrazin-2-yl)benzamide **R5**

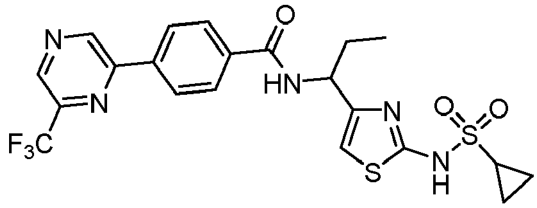

A solution of 4-(6-(trifluoromethyl)pyrazin-2-yl)benzoic acid **INTF28** (15.4 mg, 0.057 mmol) and *N*-(4-(1-aminopropyl)thiazol-2-yl)cyclopropanesulfonamide **INTE10** (15 mg, 0.057 mmol) in DMF (0.25 mL) was treated with pyridine (0.014 mL, 0.172 mmol) and T3P (50% wt. in DMF, 0.125 mL, 0.172 mmol). The reaction mixture was allowed to stir at RT over 16 hrs. The crude product was treated with water (0.05 mL) and purified by reverse phase chromatography on C18 silica (12 g column, 10-50 % MeCN/10 mM ammonium bicarbonate) to afford *N*-(1-(2-(cyclopropanesulfonamido)thiazol-4-yl)propyl)-4-(6-(trifluoromethyl)pyrazin-2-yl)benzamide (2.5 mg, 0.004 mmol, 8 % yield) as a colourless solid. Rt 1.34 min (UPLC, acidic); m/z 512 $(M+H)^+$ $(ES^+)$. $^1H$ NMR (400 MHz, Chloroform-d) $\delta$ 9.30 (s, 1H), 8.95 (s, 1H), 8.18 (d, J = 8.3 Hz, 2H), 8.12 (d, J = 84 Hz, 2H), 8.04 (d, J = 8.1 Hz, 1H), 6.51 (s, 1H), 5.09 - 4.95 (m, 1H), 2.56.2.53 (m, 1H), 2.17 - 1.89 (m, 2H), 1.32 - 1.09 (m, 2H), 1.09 - 0.71 (m, 5H), N-H not observed.

**Representative for Method 2a**

*N*-(2-(2-(cyclopropanesulfonamido)thiazol-4-yl)propan-2-yl)-4-(6-ethoxypyrazin-2-yl)-2-fluorobenzamide **R6**

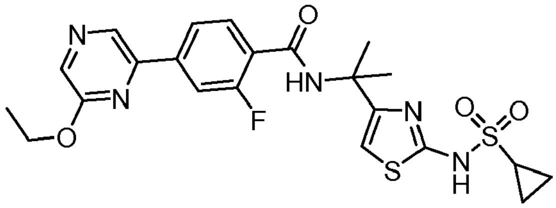

A solution of N-(2-(2-(cyclopropanesulfonamido)thiazol-4-yl)propan-2-yl)-2-fluoro-4-(4,4,5,5-tetramethyl-1,3,2-dioxaborolan-2-yl)benzamide **INTE20** (80 mg, 0.157 mmol), 2 M potassium carbonate (aq, 0.16 mL, 0.314 mmol) and 2-chloro-6-ethoxypyrazine (25 mg, 0.157 mmol) in MeCN (15 mL) and water (2 mL) was degassed ($N_2$) at 40 °C whereupon $PdCl_2(dppf)-CH_2Cl_2$ (6.4 mg, 0.008 mmol) was added to form a suspension. The mixture was degassed ($N_2$) then heated to 90 °C for 3 hrs. The reaction was cooled to RT, concentrated directly onto silica (1 g) and was purified by chromatography on silica (12 g column, 0-100 % EtOAc/*iso*-hexanes) to afford *N*-(2-(2-(cyclopropanesulfonamido)thiazol-4-yl)propan-2-yl)-4-(6-ethoxypyrazin-2-yl)-2-fluorobenzamide (13 mg, 0.025 mmol, 16 % yield) as a colourless solid. Rt 1.34 min (UPLC, acidic); m/z 506 $(M+H)^+$ $(ES^+)$. $^1H$ NMR (400 MHz, DMSO-$d_6$) $\delta$ 12.58 (s, 1H), 8.93 (s, 1H), 8.36 (s, 1H), 8.32 (s, 1H), 8.11 - 7.96 (m, 2H), 7.84 (t, J = 7.7 Hz, 1H), 6.50 (d, J = 8.5 Hz, 1H), 4.50 (q, J = 7.0 Hz, 2H), 2.63 - 2.54 (m, 1H), 1.63 (s, 6H), 1.41 (t, J = 7.0 Hz, 3H), 0.95 - 0.83 (m, 4H).

**Representative for Method 2b**

*N*-(2-(2-(cyclopropanesulfonamido)thiazol-4-yl)propan-2-yl)-4-(6-ethoxypyrazin-2-yl)-2-methoxybenzamide **R7**

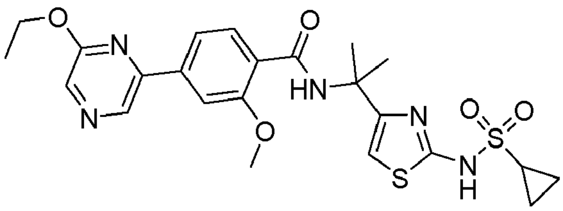

A suspension of 4-bromo-N-(2-(2-(cyclopropanesulfonamido)thiazol-4-yl)propan-2-yl)-2-methoxybenzamide

**INTE18** (100 mg, 0.211 mmol), Bispin (59 mg, 0.232 mmol) and KOAc (41mg, 0.422 mmol) in dioxane (5 mL) was degassed ($N_2$) then charged with $PdCl_2$(dppf)-$CH_2Cl_2$ (8.6 mg, 0.011 mmol) and degassed ($N_2$). The reaction mixture was heated to 90 °C for 16 hrs. The reaction mixture was allowed to cool then filtered through celite. This was then recharged with KOAc (41 mg, 0.422 mmol), Bispin (59 mg, 0.232 mmol) and $PdCl_2$(dppf)-$CH_2Cl_2$ (8.6 mg, 0.011 mmol), degassed ($N_2$) then heated to 90 °C for 2 hrs. To the reaction mixture was added 2-chloro-6-ethoxypyrazine (33.4 mg, 0.211 mmol) and 2 M potassium carbonate (aq, 0.21 mL, 0.422 mmol) and heated to 90 °C for 16 hrs. The reaction mixture was allowed to cool to RT then diluted with EtOAc (10 mL) and water (10 mL), filtered over celite eluting with EtOAc (10 mL). The phases were then partitioned, washed with brine (10 mL), dried ($Na_2SO_4$), filtered and concentrated onto silica (500 mg). The crude product was purified by chromatography on silica (12 g cartridge, 0-100 % EtOAc/*iso*-hexanes). The resulting product was re-purified by preparative HPLC (Varian, Basic (0.1 % ammonium bicarbonate), 15-50% MeCN in water) to afford N-(2-(2-(cyclopropanesulfonamido)thiazol-4-yl)propan-2-yl)-4-(6-ethoxypyrazin-2-yl)-2-methoxybenzamide (20 mg, 0.037 mmol, 17 % yield) as a colourless solid. Rt 2.17 min (HPLC acidic); m/z 518 $(M+H)^+$ $(ES^+)$. $^1H$ NMR (500 MHz, DMSO-$d_6$) $\delta$ 12.56 (s, 1H), 8.94 (s, 1H), 8.39 (s, 1H), 8.30 (s, 1H), 7.91 (d, J = 8.1 Hz, 1H), 7.86 (d, J = 1.5 Hz, 1H), 7.82 (dd, J = 8.1, 1.5 Hz, 1H), 6.49 (s, 1H), 4.50 (q, J = 7.0 Hz, 2H), 4.07 (s, 3H), 2.59 - 2.52 (m, 1H), 1.65 (s, 6H), 1.41 (t, J = 7.0 Hz, 3H), 0.95 - 0.76 (m, 4H).

**Table 5**: The following final compounds were made according to general methods

| R | Name/Structure (All examples containing chiral centres are racemic unless stated) | Synthesis Method, [LCMS Method], m/z $(M+H)^+$, (RT/Min) | $^1H$ NMR Chemical Shift Data (DMSO-d6 unless stated) |
|---|---|---|---|
| R8 | N-((2-(cyclopropanesulfonamido) thiazol-4-yl)methyl)-5-phenylpicolinamide 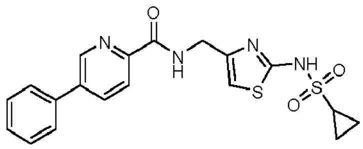 | Method 1a, Using **INTE9** and commercial acid, [UPLC Basic], 415, (1.23) | 12.55 (s, 1H), 9.26 (t, J = 6.2 Hz, 1H), 8.99 (dd, J = 2.3, 0.8 Hz, 1H), 8.31 (dd, J = 8.2, 2.3 Hz, 1H), 8.13 (dd, J = 8.2, 0.8 Hz, 1H), 7.87 - 7.77 (m, 2H), 7.61 - 7.53 (m, 2H), 7.52 - 7.43 (m, 1H), 6.50 (s, 1H), 4.38 (dd, J = 6.3, 1.2 Hz, 2H), 2.62 - 2.55 (m, 1H), 0.96 - 0.81 (m, 4H). |
| R9 | N-((2-(cyclopropanesulfonamido) thiazol-4-yl)methyl)-[1,1'-biphenyl]-4-carboxamide 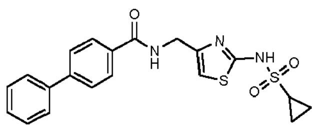 | Method 1a, Using **INTE9** and commercial acid [HPLC Basic], 414, (1.72) | 12.58 (s, 1H), 8.97 (t, J = 5.6 Hz, 1H), 8.05 - 7.95 (m, 2H), 7.84-7.70 (m, 4H), 7.55 - 7.46 (m, 2H), 7.45 - 7.36 (m, 1H), 6.53 (s, 1H), 4.34 (d, J = 5.4 Hz, 2H), 2.63 - 2.54 (m, 1H), 0.93 - 0.86 (m, 4H). |
| R10 | N-((2-(cyclopropanesulfonamido) thiazol-4-yl)methyl)-2-fluoro-4-(6-(trifluoromethyl)pyrazin-2-yl) benzamide 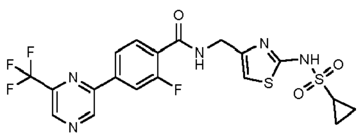 | Method 1a, Using **INTE9** and **INTF34,** [UPLC Acidic], 502, (1.24) | 12.60 (s, 1H), 9.73 (s, 1H), 9.24 (s, 1H), 9.04 - 8.78 (m, 1H), 8.23 - 8.08 (m, 2H), 7.98 - 7.81 (m, 1H), 6.54 (s, 1H), 4.35 (d, J = 5.6 Hz, 2H), 2.62 - 2.55 (m, 1H), 0.94 - 0.79 (m, 4H). |
| R11 | N-((2-(cyclopropanesulfonamido) thiazol-4-yl)methyl)-4-(6-ethoxypyrazin-2-yl)-2-fluorobenzamide 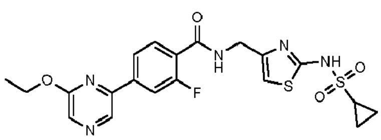 | Method 1a, Using **INTE9** and **INTF35,** [UPLC Acidic], 478, (1.22) | 12.58 (s, 1H), 8.93 (s, 1H), 8.88 - 8.83 (m, 1H), 8.32 (s, 1H), 8.11 - 8.04 (m, 2H), 7.88 - 7.79 (m, 1H), 6.54 (s, 1H), 4.50 (q, J = 7.0 Hz, 2H), 4.34 (d, J = 5.6 Hz, 2H), 2.62 - 2.54 (m, 1H), 1.41 (t, J = 7.0 Hz, 3H), 0.93 - 0.81 (m, 4H). |

(continued)

| R | Name/Structure (All examples containing chiral centres are racemic unless stated) | Synthesis Method, [LCMS Method], m/z (M+H)$^+$, (RT/Min) | $^1$H NMR Chemical Shift Data (DMSO-d6 unless stated) |
|---|---|---|---|
| R12 | N-((2-(cyclopropanesulfonamido)thiazol-4-yl)methyl)-4-(6-(trifluoromethyl)pyrazin-2-yl)benzamide 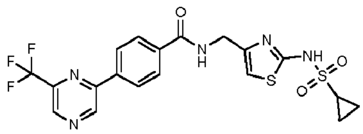 | Method 1a, Using **INTE9** and **INTF28,** [UPLC Acidic], 484, (1.21) | 12.60 (s, 1H), 9.71 (s, 1H), 9.20 (s, 1H), 9.14 - 9.01 (m, 1H), 8.35 - 8.31 (m, 2H), 8.11 - 8.06 (m, 2H), 6.55 (s, 1H), 4.35 (d, J = 5.5 Hz, 2H), 2.61 - 2.55 (m, 1H), 0.94 - 0.77 (m, 4H). |
| R13 | N-((2-(cyclopropanesulfonamido) thiazol-4-yl)methyl)-4-(6-isopropoxypyrazin-2-yl)benzamide 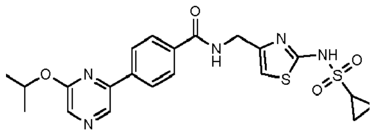 | Method 1a, Using **INTE9** and **INTF30,** [UPLC Acidic], 474, (1.28) | 12.59 (s, 1H), 9.05 - 8.99 (m, 1H), 8.87 (s, 1H), 8.26 - 8.19 (m, 3H), 8.05 - 8.01 (m, 2H), 6.54 (s, 1H), 5.48 - 5.37 (m, 1H), 4.35 (d, J = 5.4 Hz, 2H), 2.61 - 2.55 (m, 1H), 1.39 (d, J = 6.1 Hz, 6H), 0.92 - 0.82 (m, 4H). |
| R14 | N-((2-(cyclopropanesulfonamido) thiazol-4-yl)methyl)-4-(6-ethoxypyrazin-2-yl)benzamide 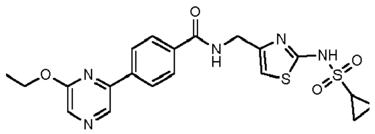 | Method 1a, Using **INTE9** and **INTE37,** [UPLC Acidic], 460, (1.18) | 12.60 (s, 1H), 9.03 (t, J = 5.6 Hz, 1H), 8.90 (s, 1H), 8.30 (s, 1H), 8.28 - 8.22 (m, 2H), 8.08 - 8.00 (m, 2H), 6.54 (s, 1H), 4.51 (q, J = 7.1 Hz, 2H), 4.35 (d, J = 5.4 Hz, 2H), 2.61 - 2.55 (m, 1H), 1.42 (t, J = 7.0 Hz, 3H), 0.95 - 0.84 (m, 4H). |
| R15 | N-(3-(2-(cyclopropanesulfonamido) thiazol-4-yl)pentan-3-yl)-4-(5-(trifluoromethyl)pyridin-3-yl) benzamide 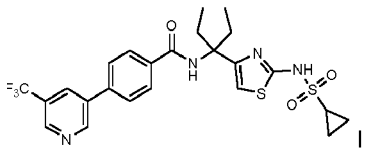 | Method 1b, Using **INTE17** and **INTF25,** [HPLC acidic], 539, (2.13) | 12.52 (s, 1H), 9.30 (d, J = 2.1 Hz, 1H), 9.02 (dd, J = 2.1, 0.9 Hz, 1H), 8.56 (s, 1H), 8.12 - 7.95 (m, 5H), 6.50 (s, 1H), 2.60 - 2.53 (m, 1H), 2.27 - 2.13 (m, 2H), 1.91 - 1.70 (m, 2H), 0.93 - 0.85 (m, 4H), 0.77 - 0.69 (m, 6H). |
| R16 | N-(3-(2-(cyclopropanesulfonamido) thiazol-4-yl)pentan-3-yl)-4-(5-fluoropyridin-3-yl)benzamide 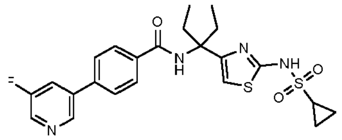 | Method 1b, Using **INTE17** and **INTF26,** [HPLC Acidic], 489, (1.9) | 12.50 (s, 1H), 8.89 (t, J = 1.8 Hz, 1H), 8.62 (d, J = 2.7 Hz, 1H), 8.17 (ddd, J = 10.3, 2.8, 1.9 Hz, 1H), 8.07 - 7.98 (m, 3H), 7.97-7.86 (m, 2H), 6.48 (s, 1H), 2.60 - 2.52 (m, 1H), 2.29 - 2.15 (m, 2H), 1.87 - 1.71 (m, 2H), 0.92 - 0.82 (m, 4H), 0.73 (t, J = 7.3 Hz, 6H). |
| R17 | N-(3-(2-(cyclopropanesulfonamido) thiazol-4-yl)pentan-3-yl)-4-(5-methylpyridin-3-yl)benzamide 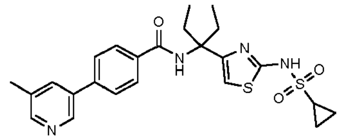 | Method 1b, Using **INTE17** and **INTF24,** [HPLC acidic], 485, (1.36) | 12.50 (s, 1H), 8.77 (d, J = 2.2 Hz, 1H), 8.46 (dd, J = 2.0, 0.8 Hz, 1H), 8.08 - 7.96 (m, 4H), 7.89 - 7.79 (m, 2H), 6.49 (s, 1H), 2.61 - 2.55 (m, 1H), 2.40 (s, 3H), 2.29 - 2.12 (m, 2H), 1.88 - 1.75 (m, 2H), 0.94 - 0.83 (m, 4H), 0.73 (t, J = 7.2 Hz, 6H). |

(continued)

| R | Name/Structure (All examples containing chiral centres are racemic unless stated) | Synthesis Method, [LCMS Method], m/z $(M+H)^+$, (RT/Min) | $^1$H NMR Chemical Shift Data (DMSO-d6 unless stated) |
|---|---|---|---|
| R18 | N-(3-(2-(cyclopropanesulfonamido) thiazol-4-yl)pentan-3-yl)-4-(pyridin-3-yl)benzamide 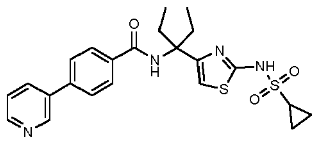 | Method 1b, Using **INTE17** and commercial acid, [HPLC Acidic], 471, (1.36) | 12.50 (s, 1H), 8.98 (dd, J = 2.4, 0.9 Hz, 1H), 8.62 (dd, J = 4.8, 1.6 Hz, 1H), 8.20 - 8.12 (m, 1H), 8.07 - 7.97 (m, 3H), 7.91 - 7.82 (m, 2H), 7.53 (ddd, J = 8.1, 4.8, 0.9 Hz, 1H), 6.49 (s, 1H), 2.61 - 2.54 (m, 1H), 2.28 - 2.12 (m, 2H), 1.89 - 1.73 (m, 2H), 0.94 - 0.82 (m, 4H), 0.74 (t, J = 7.3 Hz, 6H). |
| R19 | N-(3-(2-(cyclopropanesulfonamido) thiazol-4-yl)pentan-3-yl)-4-(6-(trifluoromethyl)pyrazin-2-yl) benzamide 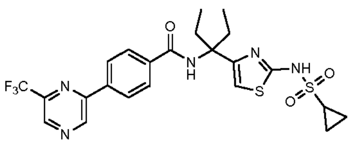 | Method 1b, Using **INTE17** and **INTF28,** [UPLC acidic], 540, (1.44) | 12.53 (s, 1H), 9.72 (s, 1H), 9.21 (s, 1H), 8.35 - 8.28 (m, 2H), 8.17 - 8.04 (m, 3H), 6.51 (s, 1H), 2.59 - 2.53 (m, 1H), 2.28 - 2.14 (m, 2H), 1.87 - 1.74 (m, 2H), 0.92 - 0.84 (m, 4H), 0.74 (t, J = 7.4 Hz, 6H). |
| R20 | 4-(6-chloropyrazin-2-yl)-N-(3-(2-(cyclopropanesulfonamido) thi azol-4-yl)pentan-3-yl)benzamide 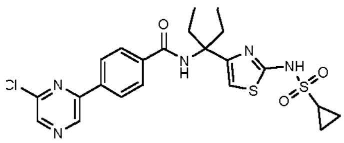 | Method 1b, Using **INTE17** and **INTF27,** [HPLC acidic], 506 $^{35}$Cl isotope, (2.06) | 12.52 (s, 1H), 9.38 (s, 1H), 8.81 (s, 1H), 8.29 - 8.21 (m, 2H), 8.15 - 8.02 (m, 3H), 6.49 (s, 1H), 2.60-2.50 (m, 1H), 2.28 - 2.12 (m, 2H), 1.90 - 1.72 (m, 2H), 0.92 - 0.82 (m, 4H), 0.78 - 0.61 (m, 6H). |
| R21 | N-(3-(2-(cyclopropanesulfonamido) thiazol-4-yl)pentan-3-yl)-4-(6-methylpyrazin-2-yl)benzamide 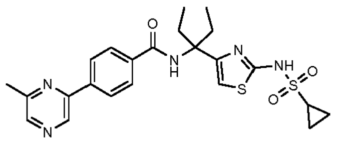 | Method 1b, Using **INTE17** and **INTF29,** [HPLC Acidic], 486, (1.89) | 12.52 (s, 1H), 9.15 (s, 1H), 8.57 (s, 1H), 8.28 - 8.20 (m, 2H),8.08 - 8.01 (m, 3H), 6.50 (s, 1H), 2.60 (s, 3H), 2.60 - 2.54 (m, 1H), 2.26 - 2.16 (m, 2H), 1.86 - 1.76 (m, 2H), 0.92 - 0.84 (m, 4H), 0.74 (t, *J* = 7.4 Hz, 6H). |
| R22 | N-(3-(2-(cyclopropanesulfonamido) thiazol-4-yl)pentan-3-yl)-4-(pyrazin-2-yl)benzamide 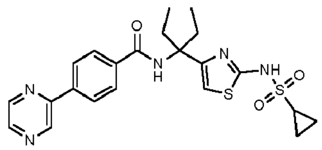 | Method 1b, Using **INTE17** and commercial acid, [HPLC Acidic], 472, (1.77) | 12.51 (s, 1H), 9.35 (d, J = 1.5 Hz, 1H), 8.77 (dd, J = 2.5, 1.5 Hz, 1H), 8.67 (d, J = 2.5 Hz, 1H), 8.28 - 8.22 (m, 2H), 8.08 - 8.00 (m, 3H), 6.49 (s, 1H), 2.59 - 2.51 (m, 1H), 2.26 - 2.14 (m, 2H), 1.86 - 1.72 (m, 2H), 0.92 - 0.84 (m, 4H), 0.73 (t, J = 7.3 Hz, 6H). |
| R23 | N-(2-(2-(cyclopropanesulfonamido) thiazol-4-yl)propan-2-yl)-5-(6-ethoxypyrazin-2-yl)-3-fluoropicolinamide 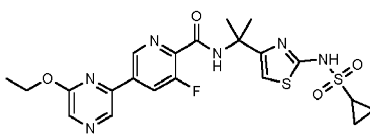 | Method 1a, Using **INTE14** and **INTF44** [HPLC Acidic], 507, (1.99) | 12.60 (s, 1H), 9.24 (d, J = 1.4 Hz, 1H), 9.02 (s, 1H), 8.59 - 8.46 (m, 2H), 8.39 (s, 1H), 6.54 (s, 1H), 4.52 (q, J = 7.0 Hz, 2H), 2.63 - 2.53 (m, 1H), 1.67 (s, 6H), 1.41 (t, J = 7.0 Hz, 3H), 0.93 - 0.85 (m, 4H). |

(continued)

| R | Name/Structure (All examples containing chiral centres are racemic unless stated) | Synthesis Method, [LCMS Method], m/z $(M+H)^+$, (RT/Min) | $^1H$ NMR Chemical Shift Data (DMSO-d6 unless stated) |
|---|---|---|---|
| R24 | N-(2-(2-(cyclopropanesulfonamido) thiazol-4-yl)propan-2-yl)-5-(6-(trifluoromethyl)pyrazin-2-yl) picolinamide 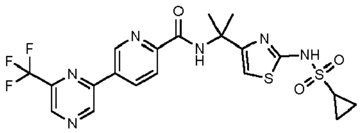 | Method 1a, Using **INTE14** and **INTF51** [HPLC Acidic], 513, (2.06) | 12.64 (s, 1H), 9.80 (s, 1H), 9.47 (d, J = 2.1 Hz, 1H), 9.29 (s, 1H), 8.76 (dd, J = 8.2, 2.2 Hz, 1H), 8.51 (s, 1H), 8.21 (d, J = 8.2 Hz, 1H), 6.58 (s, 1H), 2.60 - 2.53 (m, 1H), 1.71 (s, 6H), 0.94 - 0.79 (m, 4H). |
| R25 | 5-(6-chloropyrazin-2-yl)-N-(2-(2-(cyclopropanesulfonamido) thi azol-4-yl)propan-2-yl)picolinamide 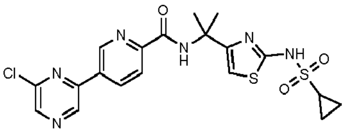 | Method 1a, Using **INTE14** and **INTF52,** [HPLC Acidic], 479 $^{35}Cl$ isotope, (1.92) | 12.62 (s, 1H), 9.46 (s, 1H), 9.39 (d, J = 2.2 Hz, 1H), 8.89 (s, 1H), 8.69 (dd, J = 8.2, 2.2 Hz, 1H), 8.51 (s, 1H), 8.17 (d, J = 8.1 Hz, 1H), 6.55 (s, 1H), 2.60 - 2.53 (m, 1H), 1.70 (s, 6H), 0.91 - 0.81 (m, 4H). |
| R26 | N-(2-(2-(cyclopropanesulfonamido) thiazol-4-yl)propan-2-yl)-5-(6-ethoxypyrazin-2-yl)picolinamide 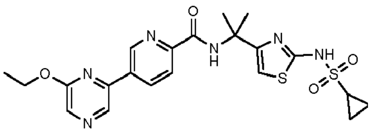 | Method 1a, Using **INTE14** and **INTF53** [HPLC Acidic], 489, (2.04) | 12.62 (s, 1H), 9.41 (d, J = 2.2 Hz, 1H), 8.99 (s, 1H), 8.69 (dd, J = 8.2, 2.2 Hz, 1H), 8.47 (s, 1H), 8.37 (s, 1H), 8.13 (d, J = 8.1 Hz, 1H), 6.56 (s, 1H), 4.51 (q, J = 7.0 Hz, 2H), 2.59 - 2.53 (m, 1H), 1.70 (s, 6H), 1.41 (t, J = 7.0 Hz, 3H), 0.95 - 0.83 (m, 4H). |
| R27 | N-(2-(2-(cyclopropanesulfonamido) thiazol-4-yl)propan-2-yl)-[2,2'-bipyridine]-5-carboxamide 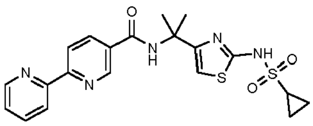 | Method 1a, Using **INTE14** and commercial acid [HPLC Acidic], 444, (1.39) | 12.59 (s, 1H), 9.13 (d, J = 2.2 Hz, 1H), 8.81 - 8.74 (m, 1H), 8.59 (s, 1H), 8.51 - 8.45 (m, 2H), 8.45 (s, 1H), 8.09 - 7.92 (m, 1H), 7.52 (ddd, J = 7.5, 4.7, 1.2 Hz, 1H), 6.52 (s, 1H), 2.62 - 2.54 (m, 1H), 1.65 (s, 6H), 0.96 - 0.84 (m, 4H). |
| R28 | 4-(5-chloropyridin-3-yl)-N-(2-(2-(cyclopropanesulfonamido) thi azol-4-yl)propan-2-yl)benzamide 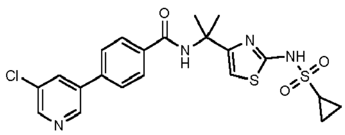 | Method 1a, Using **INTE14** and **INTF31,** [UPLC Acidic], 477 $^{35}Cl$ isotope, (1.2) | 12.57 (s, 1H), 8.98- 8.93 (m, 1H), 8.72 - 8.65 (m, 1H), 8.40 - 8.32 (m, 2H), 8.04 - 7.97 (m, 2H), 7.97 - 7.89 (m, 2H), 6.46 (s, 1H), 2.63 - 2.53 (m, 1H), 1.64 (s, 6H), 0.94 - 0.82 (m, 4H). |
| R29 | N-(2-(2-(cyclopropanesulfonamido) thiazol-4-yl)propan-2-yl)-2-fluoro-4-(5-(trifluoromethyl)pyridin-3-yl) benzamide 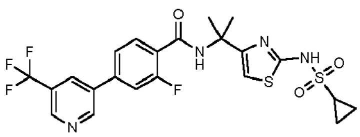 | Method 2a, Using **INTE20** and commercial coupling partner, [UPLC Acidic], 529, (1.32) | 12.59 (s, 1H), 9.31 (d, J = 2.2 Hz, 1H), 9.04 - 9.01 (m, 1H), 8.60 (s, 1H), 8.39 (s, 1H), 7.92 (d, J = 11.9 Hz, 1H), 7.89 - 7.82 (m, 2H), 6.49 (s, 1H), 2.62 - 2.54 (m, 1H), 1.63 (s, 6H), 0.94 - 0.84 (m, 4H). |

(continued)

| R | Name/Structure (All examples containing chiral centres are racemic unless stated) | Synthesis Method, [LCMS Method], m/z $(M+H)^+$, (RT/Min) | $^1H$ NMR Chemical Shift Data (DMSO-d6 unless stated) |
|---|---|---|---|
| R30 | 4-(5-chloropyridin-3-yl)-N-(2-(2-(cyclopropanesulfonamido)thi azol-4-yl)propan-2-yl)-2-fluorobenzamide 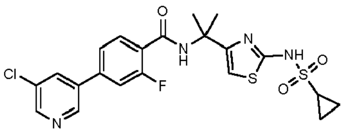 | Method 1a, Using **INTE14** and **INTF33,** [UPLC Acidic], 495 $^{35}Cl$ isotope, (1.24) | 12.59 (s, 1H), 8.98 (d, J = 2.1 Hz, 1H), 8.69 (d, J = 2.2 Hz, 1H), 8.40 (t, J = 2.2 Hz, 1H), 8.37 (s, 1H), 7.87 - 7.81 (m, 2H), 7.78 (dd, J = 8.1, 1.7 Hz, 1H), 6.48 (s, 1H), 2.62 - 2.54 (m, 1H), 1.63 (s, 6H), 0.96 - 0.81 (m, 4H). |
| R31 | N-(2-(2-(cyclopropanesulfonamido)thiazol-4-yl)propan-2-yl)-2-fluoro-4-(5-fluoropyridin-3-yl)benzamide 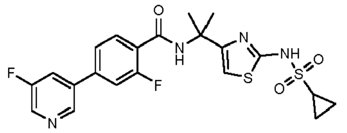 | Method 2a, Using **INTE20** and commercial coupling partner, [UPLC Acidic], 479, (1.15) | 12.58 (s, 1H), 8.91 (d, J = 1.9 Hz, 1H), 8.64 (d, J = 2.6 Hz, 1H), 8.37 (s, 1H), 8.27 - 8.15 (m, 1H), 7.88 - 7.74 (m, 3H), 6.49 (s, 1H), 2.65 - 2.51 (m, 1H), 1.62 (s, 6H), 0.94 - 0.81 (m, 4H). |
| R32 | N-(2-(2-(cyclopropanesulfonamido)thiazol-4-yl)propan-2-yl)-2-methoxy-4-(5-(trifluoromethyl)pyridin-3-yl)benzamide 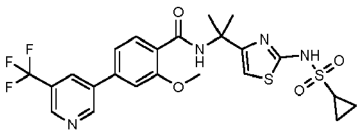 | Method 2b, Using **INTE18** and commercial coupling partner, [HPLC Acidic], 541, (2.16) | 12.56 (s, 1H), 9.31 (d, J = 2.2 Hz, 1H), 9.02 (dd, J = 2.1, 0.9 Hz, 1H), 8.59 (t, J = 2.3 Hz, 1H), 8.51 - 8.26 (m, 1H), 7.91 (d, J = 7.9 Hz, 1H), 7.61 (d, J = 1.7 Hz, 1H), 7.54 (dd, J = 8.1, 1.7 Hz, 1H), 6.48 (s, 1H), 4.08 (s, 3H), 2.59 - 2.53 (m, 1H), 1.65 (s, 6H), 0.94 - 0.78 (m, 4H). |
| R34 | 4-(5-acetylpyridin-3-yl)-N-(2-(2-(cyclopropanesulfonamido)thi azol-4-yl)propan-2-yl)benzamide 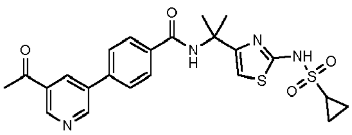 | Method 2a, Using **INTE19** and commercial coupling partner, [HPLC acidic], 485, (1.59) | 12.57 (s, 1H), 9.19 (d, J = 2.3 Hz, 1H), 9.14 (d, J = 2.0 Hz, 1H), 8.55 (t, J = 2.2 Hz, 1H), 8.34 (s, 1H), 8.08 - 8.00 (m, 2H), 7.98 - 7.91 (m, 2H), 6.47 (s, 1H), 2.72 (s, 3H), 2.61 - 2.51 (m, 1H), 1.64 (s, 6H), 0.93 - 0.82 (m, 4H). |
| R35 | N-(2-(2-(cyclopropanesulfonamido)thiazol-4-yl)propan-2-yl)-4-(5-(trifluoromethyl)pyridin-3-yl)benzamide 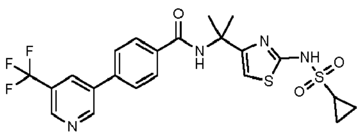 | Method 2a, Using **INTE19** and commercial coupling partner, [HPLC Basic], 511, (1.79) | 12.57 (s, 1H), 9.29 (d, J = 2.1 Hz, 1H), 9.03 - 8.97 (m, 1H), 8.58 - 8.50 (m, 1H), 8.36 (s, 1H), 8.06 - 7.96 (m, 4H), 6.47 (s, 1H), 2.59 - 2.51 (m, 1H), 1.63 (s, 6H), 0.92 - 0.80 (m, 4H). |
| R36 | N-(2-(2-(cyclopropanesulfonamido)thiazol-4-yl)propan-2-yl)-4-(5-fluoropyridin-3-yl)benzamide 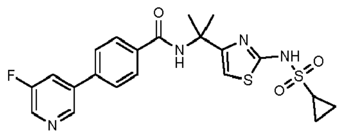 | Method 2a, Using **INTE19** and commercial coupling partner, [HPLC Basic], 461, (1.54) | 12.56 (s, 1H), 8.91 - 8.84 (m, 1H), 8.62 (d, *J* = 2.7 Hz, 1H), 8.34 (s, 1H), 8.20 - 8.13 (m, 1H), 8.04 - 7.97 (m, 2H), 7.96 - 7.89 (m, 2H), 6.47 (s, 1H), 2.58 - 2.52 (m, 1H), 1.63 (s, 6H), 0.91 - 0.83 (m, 4H). |

(continued)

| R | Name/Structure (All examples containing chiral centres are racemic unless stated) | Synthesis Method, [LCMS Method], m/z $(M+H)^+$, (RT/Min) | $^1H$ NMR Chemical Shift Data (DMSO-d6 unless stated) |
|---|---|---|---|
| R37 | N-(2-(2-(cyclopropanesulfonamido) thiazol-4-yl)propan-2-yl)-4-(5-methylpyridin-3-yl)benzamide 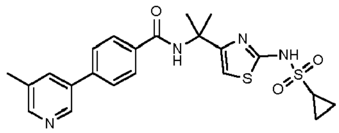 | Method 1b, Using **INTE14** and **INTF24,** [UPLC Acidic], 457, (0.74) | 12.55 (s, 1H), 8.77 (d, J = 2.2 Hz, 1H), 8.46 (dd, J = 2.1, 0.8 Hz, 1H), 8.31 (s, 1H), 8.02 - 7.93 (m, 3H), 7.89 - 7.81 (m, 2H), 6.48 (s, 1H), 2.61 - 2.53 (m, 1H), 2.40 (s, 3H), 1.64 (s, 6H), 0.97 - 0.71 (m, 4H). |
| R38 | N-(2-(2-(cyclopropanesulfonamido) thiazol-4-yl)propan-2-yl)-4-(5-methoxypyridin-3-yl)benzamide 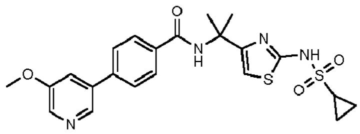 | Method 1b, Using **INTE14** and commercial acid, [UPLC Acidic], 473, (0.9) | 12.56 (s, 1H), 8.57 (d, J = 1.9 Hz, 1H), 8.34 (d, J = 2.8 Hz, 1H), 8.31 (s, 1H), 8.05 - 7.96 (m, 2H), 7.92 - 7.83 (m, 2H), 7.70 (dd, J = 2.8, 1.9 Hz, 1H), 6.48 (s, 1H), 3.94 (s, 3H), 2.63 - 2.50 (m, 1H), 1.64 (s, 6H), 0.96 - 0.82 (m, 4H). |
| R39 | N-(2-(2-(cyclopropanesulfonamido) thiazol-4-yl)propan-2-yl)-4-(pyridin-3-yl)benzamide 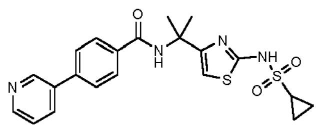 | Method 1b, Using **INTE14** and commercial acid, [HPLC Acidic], 443, (1.18) | 12.56 (s, 1H), 8.98 (dd, J = 2.5, 0.9 Hz, 1H), 8.62 (dd, J = 4.7, 1.6 Hz, 1H), 8.31 (s, 1H), 8.17 (ddd, J = 8.0, 2.5, 1.6 Hz, 1H), 8.04 - 7.96 (m, 2H), 7.90 - 7.82 (m, 2H), 7.53 (ddd, J = 8.0, 4.8, 0.9 Hz, 1H), 6.48 (s, 1H), 1.64 (s, 6H), 1.00 - 0.78 (m, 4H), 1 x CH obscured |
| R40 | N-(2-(2-(cyclopropanesulfonamido) thiazol-4-yl)propan-2-yl)-3'-(trifluoromethyl)-[1,1'-biphenyl]-4-carboxamide 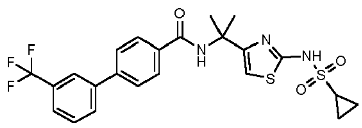 | Method 1a, Using **INTE14** and commercial acid, [HPLC Acidic], 510, (2.35) | 12.57 (s, 1H), 8.33 (s, 1H), 8.11 - 8.04 (m, 2H), 8.04 - 7.99 (m, 2H), 7.91 - 7.85 (m, 2H), 7.80 - 7.69 (m, 2H), 6.48 (s, 1H), 2.60 - 2.54 (m, 1H), 1.64 (s, 6H), 0.93 - 0.84 (m, 4H). |
| R41 | N-(2-(2-(cyclopropanesulfonamido) thiazol-4-yl)propan-2-yl)-4-(6-ethylpyrazin-2-yl)-2-fluorobenzamide 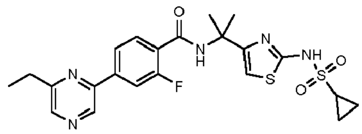 | Method 1a, Using **INTE14** and **INTF39,** [HPLC Acidic], 490, (1.97) | 12.59 (s, 1H), 9.19 (s, 1H), 8.62 (s, 1H), 8.38 (d, J = 2.5 Hz, 1H), 8.13 - 8.03 (m, 2H), 7.89 - 7.82 (m, 1H), 6.50 (s, 1H), 2.96 - 2.84 (m, 2H), 2.64 - 2.55 (m, 1H), 1.64 (s, 6H), 1.33 (t, J = 7.6 Hz, 3H), 0.97 - 0.82 (m, 4H). |
| R42 | N-(2-(2-(cyclopropane sulfonamido) thiazol-4-yl)propan-2-yl)-2-fluoro-4-(6-(trifluoromethyl)pyrazin-2-yl) benzamide 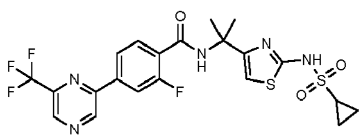 | Method 2a, Using **INTE20** and commercial coupling partner, [UPLC Acidic], 530, (1.36) | 12.60 (s, 1H), 9.73 (s, 1H), 9.23 (s, 1H), 8.47 (s, 1H), 8.17 - 8.08 (m, 2H), 7.95 - 7.86 (m, 1H), 6.50 (s, 1H), 2.63 - 2.53 (m, 1H), 1.63 (s, 6H), 0.97 - 0.73 (m, 4H). |

(continued)

| R | Name/Structure (All examples containing chiral centres are racemic unless stated) | Synthesis Method, [LCMS Method], m/z (M+H)$^+$, (RT/Min) | $^1$H NMR Chemical Shift Data (DMSO-d6 unless stated) |
|---|---|---|---|
| R43 | N-(2-(2-(cyclopropane sulfonamido) thiazol-4-yl)propan-2-yl)-2-fluoro-4-(6-isopropoxypyrazin-2-yl)benzamide 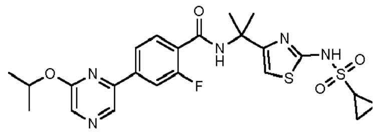 | Method 1a, Using **INTE14 and INTF36,** [UPLC Acidic], 520, (1.42) | 12.59 (s, 1H), 8.96 - 8.79 (m, 1H), 8.42 - 8.32 (m, 1H),8.31 - 8.20 (m, 1H), 8.12 - 7.95 (m, 2H), 7.89 - 7.76 (m, 1H), 6.50 (s, 1H), 5.46 - 5.34 (m, 1H), 2.64 - 2.55 (m, 1H), 1.71 - 1.53 (m, 6H), 1.50 - 1.25 (m, 6H), 0.96 - 0.85 (m, 4H). |
| R44 | N-(2-(2-(cyclopropanesulfonamido) thiazol-4-yl)propan-2-yl)-2-fluoro-4-(6-(2,2,2-trifluoroethoxy)pyrazin-2-yl) benzamide 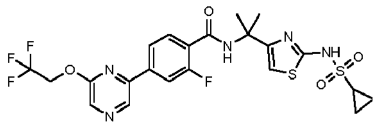 | Method 1a, Using **INTE14 and INTF40,** [UPLC Acidic], 560, (1.4) | 12.59 (s, 1H), 9.09 (s, 1H), 8.52 (s, 1H), 8.40 (s, 1H), 8.18 - 8.09 (m, 2H), 7.92 - 7.82 (m, 1H), 6.50 (s, 1H), 5.24 (q, J = 9.0 Hz, 2H), 2.62 - 2.55 (m, 1H), 1.63 (s, 6H), 0.98 - 0.81 (m, 4H). |
| R45 | N-(2-(2-(cyclopropanesulfonamido) thiazol-4-yl)propan-2-yl)-2-methyl-4-(6-(trifluoromethyl)pyrazin-2-yl) benzamide 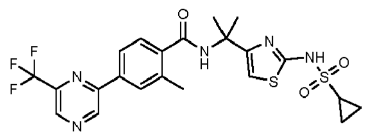 | Method 1a, Using **INTE14 and INTF43** [HPLC Acidic], 526, (2.09) | 12.61 (s, 1H), 9.66 (s, 1H), 9.17 (s, 1H), 8.46 (s, 1H), 8.14 - 8.02 (m, 2H), 7.71 (d, J = 7.9 Hz, 1H), 6.50 (s, 1H), 2.61 - 2.55 (m, 1H), 2.42 (s, 3H), 1.62 (s, 6H), 0.94 - 0.85 (m, 4H). |
| R46 | N-(2-(2-(cyclopropanesulfonamido) thiazol-4-yl)propan-2-yl)-4-(6-ethoxypyrazin-2-yl)-2-methylbenzamide 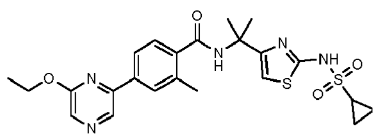 | Method 1a, Using **INTE14** and **INTF38,** [HPLC Acidic], 502, (2.04) | 12.58 (s, 1H), 8.84 (s, 1H), 8.38 (s, 1H), 8.26 (s, 1H), 8.01 - 7.93 (m, 2H), 7.64 (d, J = 8.0 Hz, 1H), 6.49 (s, 1H), 4.49 (q, J = 7.0 Hz, 2H), 2.56 - 2.51 (m, 1H), 2.40 (s, 3H), 1.61 (s, 6H), 1.41 (t, J = 7.0 Hz, 3H), 0.95 - 0.81 (m, 4H). |
| R47 | N-(2-(2-(cyclopropanesulfonamido) thiazol-4-yl)propan-2-yl)-4-(6-ethoxypyrazin-2-yl)-2-(trifluoromethyl) benzamide 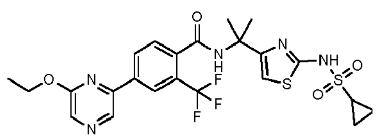 | Method 1a, Using **INTE14** and **INTF42,** [HPLC Acidic], 556, (2.17) | 12.63 (s, 1H), 8.99 (s, 1H), 8.73 (s, 1H), 8.50 (dd, J = 8.1, 1.7 Hz, 1H), 8.43 (d, J = 1.6 Hz, 1H), 8.34 (s, 1H), 7.98 (d, J = 8.1 Hz, 1H), 6.53 (s, 1H), 4.50 (q, J = 7.0 Hz, 2H), 2.62 - 2.56 (m, 1H), 1.59 (s, 6H), 1.41 (t, J = 7.0 Hz, 3H), 0.95 - 0.85 (m, 4H). |

(continued)

| R | Name/Structure (All examples containing chiral centres are racemic unless stated) | Synthesis Method, [LCMS Method], m/z $(M+H)^+$, (RT/Min) | $^1$H NMR Chemical Shift Data (DMSO-d6 unless stated) |
|---|---|---|---|
| R48 | N-(2-(2-(cyclopropanesulfonamido) thiazol-4-yl)propan-2-yl)-2-methoxy-4-(6-(trifluoromethyl)pyrazin-2-yl) benzamide 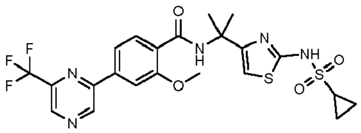 | Method 2b, Using **INTE18** and commercial coupling partner, [HPLC Acidic], 542, (2.2) | 12.55 (s, 1H), 9.75 (s, 1H), 9.21 (s, 1H), 8.38 (s, 1H), 7.95 - 7.70 (m, 3H), 6.51 (s, 1H), 4.07 (s, 3H), 2.59 - 2.54 (m, 1H), 1.65 (s, 6H), 0.96 - 0.80 (m, 4H). |
| R49 | 4-(6-chloropyrazin-2-yl)-N-(2-(2-(cyclopropanesulfonamido) thi azol-4-yl)propan-2-yl)-2-methoxybenzamide 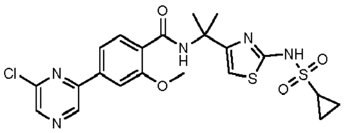 | Method 2b, Using **INTE18** and commercial coupling partner, [HPLC Acidic], 508 $^{35}$Cl isotope, (2.07) | 12.55 (s, 1H), 9.42 (s, 1H), 8.82 (s, 1H), 8.37 (s, 1H), 7.91 (d, J = 8.0 Hz, 1H), 7.86 - 7.81 (m, 2H), 6.51 (s, 1H), 4.06 (s, 3H), 2.60 - 2.51 (m, 1H), 1.64 (s, 6H), 1.02 - 0.74 (m, 4H). |
| R50 | 4-(6-cyanopyrazin-2-yl)-N-(2-(2-(cyclopropanesulfonamido) thi azol-4-yl)propan-2-yl)-2-methoxybenzamide 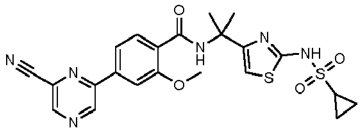 | Method 2b, Using **INTE18** and commercial coupling partner, [HPLC Acidic], 499, (1.94) | 12.55 (s, 1H), 9.70 (s, 1H), 9.23 (s, 1H), 8.39 (s, 1H), 7.96 - 7.82 (m, 3H), 6.51 (s, 1H), 4.07 (s, 3H), 2.62 - 2.53 (m, 1H), 1.64 (s, 6H), 0.96 - 0.74 (m, 4H). |
| R51 | N-(2-(2-(cyclopropanesulfonamido) thiazol-4-yl)propan-2-yl)-4-(6-(trifluoromethyl)pyrazin-2-yl) benzamide 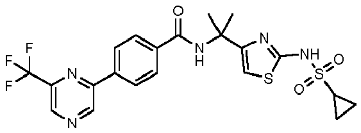 | Method 1b, Using **INTE14** and **INF28,** [UPLC Acidic], 512, (1.31) | 12.59 (s, 1H), 9.72 (s, 1H), 9.21 (s, 1H), 8.42 (s, 1H), 8.34 - 8.27 (m, 2H), 8.13 - 8.04 (m, 2H), 6.50 (s, 1H), 2.62 - 2.54 (m, 1H), 1.65 (s, 6H), 0.93 - 0.86 (m, 4H). |
| R52 | 4-(6-chloropyrazin-2-yl)-N-(2-(2-(cyclopropanesulfonamido) thi azol-4-yl)propan-2-yl)benzamide 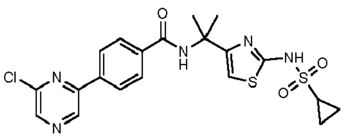 | Method 1b, Using **INTE14** and **INTF27,** [HPLC acidic], 478 $^{35}$Cl isotope, (1.87) | 12.58 (s, 1H), 9.39 (s, 1H), 8.82 (s, 1H), 8.40 (s, 1H), 8.29 - 8.21 (m, 2H), 8.09 - 8.03 (m, 2H), 6.48 (s, 1H), 2.54 - 2.50 (m, 1H), 1.64 (s, 6H), 0.94 - 0.76 (m, 4H). |

(continued)

| R | Name/Structure (All examples containing chiral centres are racemic unless stated) | Synthesis Method, [LCMS Method], m/z $(M+H)^+$, (RT/Min) | $^1H$ NMR Chemical Shift Data (DMSO-d6 unless stated) |
|---|---|---|---|
| R53 | N-(2-(2-(cyclopropanesulfonamido) thiazol-4-yl)propan-2-yl)-4-(6-methylpyrazin-2-yl)benzamide 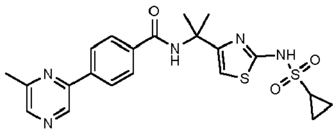 | Method 1b, Using **INTE14** and **INF29,** [HPLC Acidic], 458, (1.68) | 12.58 (s, 1H), 9.15 (s, 1H), 8.56 (s, 1H), 8.35 (s, 1H), 8.28-8.20 (m, 2H), 8.06 - 7.98 (m, 2H), 6.49 (s, 1H), 2.63 - 2.55 (m, 4H), 1.64 (s, 6H), 0.96 - 0.79 (m, 4H). |
| R54 | N-(2-(2-(cyclopropanesulfonamido) thiazol-4-yl)propan-2-yl)-4-(6-methoxypyrazin-2-yl)benzamide 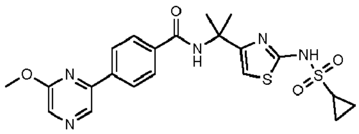 | Method 2a, Using **INTE19** and commercial coupling partner, [HPLC Basic], 474, (1.63) | 112.57 (s, 1H), 8.93 (s, 1H), 8.34 (s, 1H), 8.32 (s, 1H), 8.27 - 8.23 (m, 2H), 8.06 - 7.94 (m, 2H), 6.48 (s, 1H), 4.04 (s, 3H), 2.54 - 2.51 (m, 1H), 1.63 (s, 6H), 0.93 - 0.82 (m, 4H). |
| R55 | N-(2-(2-(cyclopropanesulfonamido) thiazol-4-yl)propan-2-yl)-4-(6-ethoxypyrazin-2-yl)benzamide 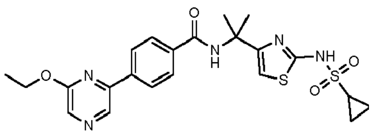 | Method 2a, Using **INTE19** and commercial coupling partner, [HPLC Basic], 488, (1.79) | 12.56 (s, 1H), 8.91 (s, 1H), 8.33 (s, 1H), 8.29 (s, 1H), 8.25 - 8.20 (m, 2H), 8.04 - 7.98 (m, 2H), 6.47 (s, 1H), 4.50 (q, J = 7.0 Hz, 2H), 2.59 - 2.53 (m, 1H), 1.63 (s, 6H), 1.41 (t, J = 7.0 Hz, 3H), 0.92 - 0.81 (m, 4H). |
| R56 | N-(2-(2-(cyclopropanesulfonamido) thiazol-4-yl)propan-2-yl)-4-(6-isopropoxypyrazin-2-yl)benzamide 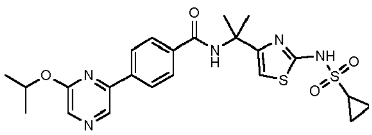 | Method 1a, Using **INTE14** and **INTF30,** [UPLC Basic], 502, (1.2) | 12.57 (s, 1H), 8.89 (s, 1H), 8.34 (s, 1H), 8.27 - 8.17 (m, 3H), 8.04 - 7.98 (m, 2H), 6.48 (s, 1H), 5.49 - 5.24 (m, 1H), 2.60 - 2.53 (m, 1H), 1.64 (s, 6H), 1.40 (d, J = 6.2 Hz, 6H), 0.92 - 0.78 (m, 4H). |
| R57 | N-(2-(2-(cyclopropanesulfonamido) thiazol-4-yl)propan-2-yl)-4-(6-(2,2,2-trifluoroethoxy)pyrazin-2-yl)benzamide 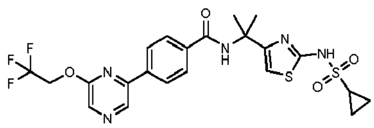 | Method 1a, Using **INTE14** and **INTF41,** [UPLC Acidic], 542, (1.38) | 12.58 (s, 1H), 9.08 (s, 1H), 8.50 (s, 1H), 8.37 (s, 1H), 8.34 - 8.25 (m, 2H), 8.08 - 7.99 (m, 2H), 6.49 (s, 1H), 5.23 (q, J = 9.0 Hz, 2H), 2.60 - 2.54 (m, 1H), 1.64 (s, 6H), 0.96 - 0.83 (m, 4H). |
| R58 | N-(2-(2-(cyclopropanesulfonamido) thiazol-4-yl)propan-2-yl)-4-(pyrazin-2-yl)benzamide 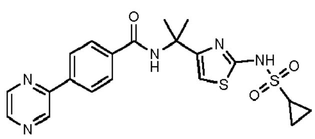 | Method 1b, Using **INTE14** and commercial acid, [HPLC Acidic], 444, (1.59) | 12.57 (s, 1H), 9.35 (d, J = 1.6 Hz, 1H), 8.76 (dd, J = 2.5, 1.5 Hz, 1H), 8.67 (d, J = 2.5 Hz, 1H), 8.36 (s, 1H), 8.29 - 8.22 (m, 2H), 8.09 - 7.95 (m, 2H), 6.48 (s, 1H), 2.60 - 2.52 (m, 1H), 1.63 (s, 6H), 0.91 - 0.83 (m, 4H). |

(continued)

| R | Name/Structure (All examples containing chiral centres are racemic unless stated) | Synthesis Method, [LCMS Method], m/z $(M+H)^+$, (RT/Min) | $^1H$ NMR Chemical Shift Data (DMSO-d6 unless stated) |
|---|---|---|---|
| R59 | RACEMIC, N-(1-(2-(cyclopropanesulfonamido) thiazol-4-yl)propyl)-4-(5-fluoropyridin-3-yl)benzamide 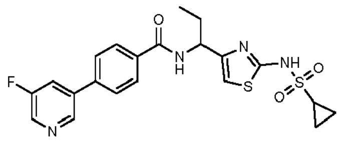 | Method 1b, Using **INTE10** and **INTF26,** [HPLC acidic], 461, (1.74) | 12.60 (s, 1H), 8.91-88 (m, 1H), 8.72 (d, J = 8.3 Hz, 1H), 8.64 (d, J = 2.7 Hz, 1H), 8.21 - 8.16 (m, 1H), 8.08 - 8.02 (m, 2H), 7.99 - 7.92 (m, 2H), 6.56 (s, 1H), 4.94-4.85 (m, 1H), 2.64 - 2.55 (m, 1H), 1.97 - 1.72 (m, 2H), 0.97 - 0.76 (m, 7H). |
| R60 | RACEMIC, N-(1-(2-(cyclopropanesulfonamido) thiazol-4-yl)propyl)-4-(5-methylpyridin-3-yl)benzamide 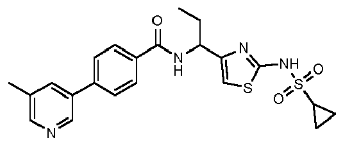 | Method 1b, Using **INTE10** and **INTF24,** [HPLC acidic], 457, (1.22) | 12.57 (s, 1H), 8.76 (d, J = 2.3 Hz, 1H), 8.68 (d, J = 8.2 Hz, 1H), 8.46 (dd, J = 2.0, 0.8 Hz, 1H), 8.06 - 7.95 (m, 3H), 7.90 - 7.82 (m, 2H), 6.54 (s, 1H), 4.94 - 4.83 (m, 1H), 2.62 - 2.55 (m, 1H), 2.39 (s, 3H), 1.97 - 1.71 (m, 2H), 0.97 -0.81 (m, 7H). |
| R61 | RACEMIC, N-(1-(2-(cyclopropanesulfonamido) thiazol-4-yl)propyl)-4-(pyridin-3-yl) benzamide 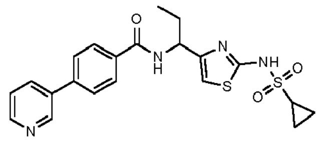 | Method 1b, Using **INTE10** and commercial acid, [HPLC acidic], 443, (1.2) | 12.58 (s, 1H), 9.00 - 8.96 (m, 1H), 8.72 - 8.66 (m, 1H), 8.63 (dd, J = 4.7, 1.6 Hz, 1H), 8.20 - 8.14 (m, 1H), 8.05 - 8.01 (m, 2H), 7.91 - 7.85 (m, 2H), 7.53 (dd, J = 7.9, 4.6 Hz, 1H), 6.55 (s, 1H), 4.95 - 4.83 (m, 1H), 2.64 - 2.55 (m, 1H), 1.98 - 1.71 (m, 2H), 0.99 - 0.81 (m, 7H). |
| R62 | RACEMIC, N-(1-(2-(cyclopropanesulfonamido) thiazol-4-yl)propyl)-4-(6-ethoxypyrazin-2-yl)-2-fluorobenzamide 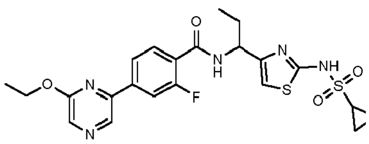 | Method 1a, Using **INTE10** and **INTF35,** [HPLC Acidic], 506, (2.12) | 12.63 (s, 1H), 8.93 (s, 1H), 8.71 (d, J = 8.3 Hz, 1H), 8.33 (s, 1H), 8.11 - 8.01 (m, 2H), 7.79 (t, J = 7.8 Hz, 1H), 6.55 (s, 1H), 4.93 - 4.82 (m, 1H), 4.51 (q, J = 7.0 Hz, 2H), 2.62 - 2.55 (m, 1H), 1.94 - 1.81 (m, 1H), 1.81 - 1.66 (m, 1H), 1.42 (t, J = 7.0 Hz, 3H), 1.01 - 0.83 (m, 7H). |
| R63 | RACEMIC, N-(1-(2-(cyclopropanesulfonamido) thiazol-4-yl)propyl)-4-(6-ethoxypyrazin-2-yl)-2-fluoro-N-methylbenzamide 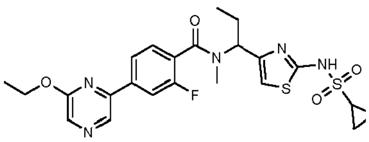 | Method 1a, Using **INTE11** and **INTF35,** [HPLC Acidic], 520, (2.22) | 12.75 (s, 1H), 8.90 (d, J = 5.1 Hz, 1H), 8.31 (d, J = 4.7 Hz, 1H), 8.15 - 7.98 (m, 2H), 7.69 (s, 1H), 6.73 (s, 1H), 5.50 (s, 1H), 4.53 - 4.47 (m, 2H), 2.66 - 2.62 (m, 4H), 2.11 - 1.74 (m, 2H), 1.45-1.35 (m, 3H), 0.99 - 0.84 (m, 7H). |

(continued)

| R | Name/Structure (All examples containing chiral centres are racemic unless stated) | Synthesis Method, [LCMS Method], m/z (M+H)+, (RT/Min) | $^{1}$H NMR Chemical Shift Data (DMSO-d6 unless stated) |
|---|---|---|---|
| R64 | RACEMIC, N-(1-(2-(cyclopropanesulfonamido) thiazol-4-yl)propyl)-2-fluoro-4-(6-isopropoxypyrazin-2-yl)benzamide 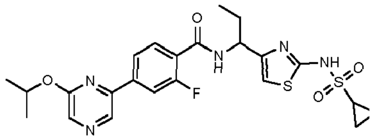 | Method 1a, Using **INTE10** and **INF36** [HPLC Acidic], 520, (2.24) | 12.62 (s, 1H), 8.90 (s, 1H), 8.70 (d, J = 8.3 Hz, 1H), 8.27 (s, 1H), 8.10 - 7.97 (m, 2H), 7.78 (t, J = 7.8 Hz, 1H), 6.54 (s, 1H), 5.45 - 5.33 (m, 1H), 4.87 (q, J = 7.9 Hz, 1H), 2.62-2.54 (m, 1H), 1.96-1.83 (m, 1H), 1.81 - 1.64 (m, 1H), 1.39 (d, J = 6.1 Hz, 6H), 0.98 - 0.80 (m, 7H). |
| R65 | RACEMIC, 4-(6-chloropyrazin-2-yl)-N-(1-(2-(cyclopropanesulfonamido) thiazol-4-yl)propyl)benzamide 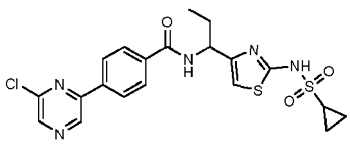 | Method 1b, Using **INTE10** and **INTF27** [HPLC acidic], 478 $^{35}$Cl isotope, (1.91) | 12.60 (s, 1H), 9.38 (d, J = 0.6 Hz, 1H), 8.82 (s, 1H), 8.76 (d, J = 8.2 Hz, 1H), 8.31 - 8.20 (m, 2H), 8.07 (d, J = 8.4 Hz, 2H), 6.55 (s, 1H), 4.90 (q, J = 8.5, 7.9 Hz, 1H), 2.62 - 2.52 (m, 1H), 2.00 - 1.60 (m, 2H), 0.97 - 0.81 (m, 7H). |
| R66 | RACEMIC, N-(1-(2-(cyclopropanesulfonamido) thiazol-4-yl)propyl)-4-(6-methylpyrazin-2-yl)benzamide 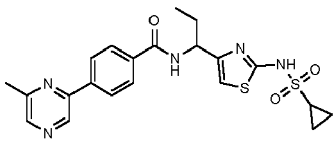 | Method 1b, Using **INTE10** and **INTF29** [HPLC Acidic], 458, (1.73) | 12.59 (s, 1H), 9.13 (s, 1H), 8.72 (d, J = 8.2 Hz, 1H), 8.56 (s, 1H), 8.28 - 8.21 (m, 2H), 8.07 - 8.00 (m, 2H), 6.56 (s, 1H), 4.94 - 4.84 (m, 1H), 2.62 - 2.50 (m, 4H), 2.01 - 1.67 (m, 2H), 0.96 - 0.85 (m, 7H). |
| R67 | RACEMIC, N-(1-(2-(cyclopropanesulfonamido) thiazol-4-yl)propyl)-4-(pyrazin-2-yl) benzamide 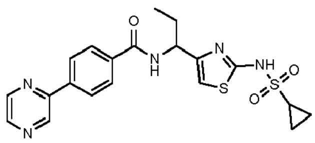 | Method 1b, Using **INTE10** and commercial acid, [HPLC Acidic], 444, (1.81) | 12.59 (s, 1H), 9.35 (d, J = 1.5 Hz, 1H), 8.77 (dd, J = 2.5, 1.5 Hz, 1H), 8.74 (d, J = 8.2 Hz, 1H), 8.67 (d, J = 2.4 Hz, 1H), 8.30 - 8.24 (m, 2H), 8.11 - 7.99 (m, 2H), 6.56 (s, 1H), 4.89 (q, J = 7.8 Hz, 1H), 2.62 - 2.54 (m, 1H), 1.98 - 1.72 (m, 2H), 0.96 - 0.79 (m, 7H). |
| R68 | SINGLE ENANTIOMER, N-(1-(2-(cyclopropanesulfonamido)thiazol-4-yl)propyl)-4-(5-fluoropyridin-3-yl)benzamide 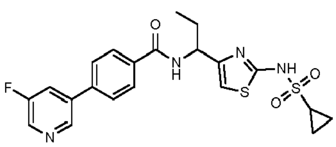 | Method 1b, Using **INTE10** and **INTF26,** Separation using **chiral method A,** [UPLC acidic], 461, (1.13) **Chiral IA method 1:** Peak 1 RT 21.4 mins | 12.60 (s, 1H), 8.89 (t, J = 1.8 Hz, 1H), 8.72 (d, J = 8.3 Hz, 1H), 8.64 (d, J = 2.7 Hz, 1H), 8.21 - 8.16 (m, 1H), 8.08 - 8.02 (m, 2H), 7.99 - 7.92 (m, 2H), 6.56 (s, 1H), 4.90 (q, J = 8.2 Hz, 1H), 2.64 - 2.55 (m, 1H), 1.97 - 1.72 (m, 2H), 0.97 - 0.76 (m, 7H). |

(continued)

| R | Name/Structure (All examples containing chiral centres are racemic unless stated) | Synthesis Method, [LCMS Method], m/z $(M+H)^+$, (RT/Min) | $^1$H NMR Chemical Shift Data (DMSO-d6 unless stated) |
|---|---|---|---|
| R69 | SINGLE ENANTIOMER, N-(1-(2-(cyclopropanesulfonamido)thiazol-4-yl)propyl)-4-(5-fluoropyridin-3-yl)benzamide 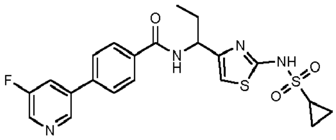 | Method 1b, Using **INTE10** and **INTF26**, Separation using **chiral method A** [UPLC acidic], 461, (1.13) **Chiral IA method 1:** Peak 2 RT 27.5 mins | 12.60 (s, 1H), 8.89 (t, J = 1.8 Hz, 1H), 8.72 (d, J = 8.3 Hz, 1H), 8.64 (d, J = 2.7 Hz, 1H), 8.21 - 8.16 (m, 1H), 8.08 - 8.02 (m, 2H), 7.99 - 7.92 (m, 2H), 6.56 (s, 1H), 4.90 (q, J = 8.2 Hz, 1H), 2.64 - 2.55 (m, 1H), 1.97 - 1.72 (m, 2H), 0.97 - 0.76 (m, 7H). |
| R70 | SINGLE ENANTIOMER, N-(1-(2-(cyclopropanesulfonamido)thiazol-4-yl)propyl)-4-(6-ethoxypyrazin-2-yl)-2-fluorobenzamide 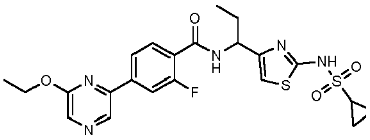 | Chiral Sep, Method 1a, Using **INTE10** and **INTF35**, Separation using **chiral method B,** [HPLC Acidic], 506, (2.11) **Chiral IA method 2:** Peak 1 RT 16.0 mins | 12.62 (s, 1H), 8.93 (s, 1H), 8.70 (dd, J = 8.3, 1.4 Hz, 1H), 8.32 (s, 1H), 8.11 - 8.03 (m, 2H), 7.80 - 7.73 (m, 1H), 6.55 (s, 1H), 4.93 - 4.79 (m, 1H), 4.50 (q, J = 7.0 Hz, 2H), 2.62 - 2.55 (m, 1H), 1.94 - 1.82 (m, 1H), 1.80 - 1.67 (m, 1H), 1.41 (t, J = 7.0 Hz, 3H), 0.98 - 0.85 (m, 7H). |
| R71 | SINGLE ENANTIOMER, N-(1-(2-(cyclopropanesulfonamido)thiazol-4-yl)propyl)-4-(6-ethoxypyrazin-2-yl)-2-fluorobenzamide 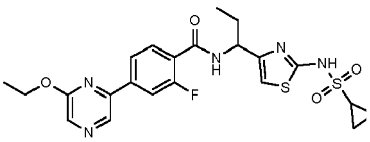 | Chiral Sep, Method 1a, Using **INTE10** and **INTF35**, Separation using **chiral method B,** [HPLC Acidic], 506, (2.12) **Chiral IA method 2:** Peak 2 RT 30.2 mins | 12.62 (s, 1H), 8.93 (s, 1H), 8.75 - 8.67 (m, 1H), 8.32 (s, 1H), 8.11 - 8.01 (m, 2H), 7.83 - 7.72 (m, 1H), 6.54 (s, 1H), 4.93 - 4.79 (m, 1H), 4.50 (q, J = 7.0 Hz, 2H), 2.62 - 2.55 (m, 1H), 1.94 - 1.81 (m, 1H), 1.79 - 1.64 (m, 1H), 1.41 (t, J = 7.0 Hz, 3H), 0.98 - 0.86 (m, 7H). |

## Biological Examples

### Biological Example 1 - Human CTPS1 Enzyme Inhibition

The enzyme inhibitory activities of compounds invented against the target of interest were determined using the ADP-Glo™ Max assay (Promega, UK). Assays for human CTPS1 were performed in 1x assay buffer containing 50mM Tris, 10mM $MgCl_2$, 0.01% Tween-20, pH to 8.0 accordingly. Finally, immediately before use, L-cysteine was added to the 1x assay buffer to a final concentration of 2mM. All reagents are from Sigma-Aldrich unless specified otherwise. Human full length active C-terminal FLAG-Hiss-tag CTPS1 (UniProtKB - P17812, CTPS[1-591]-GGDYKDDDDKGGHHHHHHHH) was obtained from Proteros biostructures GmbH.

Assay Procedure.

3x human CTPS1 protein was prepared in 1x assay buffer to the final working protein concentration required for the reaction. A 2uL volume per well of 3x human CTPS1 protein was mixed with 2uL per well of 3x test compound (compound prepared in 1x assay buffer to an appropriate final 3x compound concentration respective to the concentration response curve designed for the compounds under test) for 10 minutes at 25°C. The enzymatic reaction was then

initiated by addition of a 2uL per well volume of a pre-mixed substrate mix (UltraPure ATP from ADP-Glo™ Max kit (0.31mM), GTP (0.034mM), UTP (0.48mM) and L-glutamine (0.186mM)) and the mixture was incubated for an appropriate amount of time within the determined linear phase of the reaction at 25°C under sealed plate conditions with constant agitation at 500 revolutions per minute (rpm). ADP-Glo™ Max reagent was added for 60 minutes (6μL per well) and subsequently ADP-Glo™ Max development reagent was added for 60 minutes (12uL per well) prior to signal detection in a microplate reader (EnVision® Multilabel Reader, Perkin Elmer). Following each reagent addition over the course of the assay, assay plates were pulse centrifuged for 30 seconds at 500rpm.

In all cases, the enzyme converts ATP to ADP and the ADP-Glo™ Max reagent subsequently depletes any remaining endogenous ATP in the reaction system. The ADP-Glo™ Max detection reagent converts the ADP that has been enzymatically produced back into ATP and using ATP as a substrate together with luciferin for the enzyme luciferase, light is generated which produces a detectable luminescence. The luminescent signal measured is directly proportional to the amount of ADP produced by the enzyme reaction and a reduction in this signal upon compound treatment demonstrates enzyme inhibition. The percentage inhibition produced by each concentration of compound was calculated using the equation shown below:

$$\%\,Inhibition = 1 - \frac{(Mean_{Min} - Mean_{Inh})}{(Mean_{Min} - Mean_{Max})}\, x\, 100$$

Percentage inhibition was then plotted against compound concentration, and the 50% inhibitory concentration ($IC_{50}$) was determined from the resultant concentration-response curve.

**Table 6**: *Human CTPS1 Enzyme Inhibition data grouped by potency range* (± indicates $IC_{50}$ in the range of >10 to 21 micromolar, + indicates $IC_{50}$ in the range >1 to 10 micromolar, ++ indicates $IC_{50}$ in the range >0.1 to 1 micromolar, +++ indicates $IC_{50}$ of $\leq$0.1 micromolar)

| R | CTPS1 |
|---|---|
| R1 | ++ |
| R2 | ++ |
| R3 | ++ |
| R4 | +++ |
| R5 | +++ |
| R6 | +++ |
| R7 | ++ |
| R8 | + |
| R9 | + |
| R10 | +++ |
| R11 | +++ |
| R12 | +++ |
| R13 | +++ |
| R14 | +++ |
| R15 | +++ |
| R16 | ++ |
| R17 | ++ |
| R18 | ++ |
| R19 | +++ |
| R20 | +++ |
| R21 | +++ |
| R22 | ++ |
| R23 | ++ |
| R24 | ++ |
| R25 | ++ |
| R26 | ++ |
| R27 | ± |
| R28 | +++ |
| R29 | +++ |
| R30 | ++ |
| R31 | ++ |
| R32 | ++ |
| R33 | + |
| R34 | + |
| R35 | +++ |
| R36 | ++ |
| R37 | +++ |
| R38 | ++ |
| R39 | ++ |
| R40 | ++ |
| R41 | ++ |
| R42 | +++ |
| R43 | +++ |
| R44 | ++ |
| R45 | ++ |
| R46 | ++ |
| R47 | ++ |
| R48 | ++ |
| R49 | ++ |
| R50 | + |
| R51 | +++ |
| R52 | +++ |
| R53 | ++ |

| | |
|---|---|
| R54 | +++ |
| R55 | +++ |
| R56 | +++ |
| R57 | ++ |
| R58 | ++ |
| R59 | ++ |
| R60 | ++ |
| R61 | ++ |
| R62 | ++ |

| | |
|---|---|
| R63 | ++ |
| R64 | ++ |
| R65 | +++ |
| R66 | ++ |
| R67 | ++ |
| R68 | + |
| R69 | +++ |
| R70 | ++ |
| R71 | +++ |

All compounds of the invention which have been tested were found to demonstrate inhibition of CTPS1 enzyme in this assay. Consequently, these compounds may be expected to have utility in the inhibition of CTPS1.

Throughout the specification and the claims which follow, unless the context requires otherwise, the word 'comprise', and variations such as 'comprises' and 'comprising', will be understood to imply the inclusion of a stated integer, step, group of integers or group of steps but not to the exclusion of any other integer, step, group of integers or group of steps.

The application of which this description and claims forms part may be used as a basis for priority in respect of any subsequent application. The claims of such subsequent application may be directed to any feature or combination of features described herein. They may take the form of product, composition, process, or use claims and may include, by way of example and without limitation, the claims which follow.

All publications, including but not limited to patents and patent applications, cited in this specification are herein incorporated by reference as if each individual publication were specifically and individually indicated to be incorporated by reference herein as though fully set forth.

**Clauses of the invention**

Clause 1. A compound of formula (I):

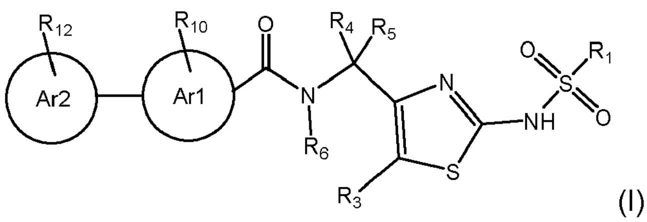

(I)

wherein

$R_1$ is $C_{1-4}$alkyl, $C_{1-2}$alkyleneO$C_{1-2}$alkyl or $C_{0-1}$alkylene$C_{3-4}$cycloalkyl which cycloalkyl is optionally substituted by $CH_3$;

$R_3$ is H, $CH_3$, F or Cl;

$R_4$ and $R_5$ are each independently H, $C_{1-4}$alkyl, $C_{0-2}$alkylene$C_{3-5}$cycloalkyl, $C_{1-3}$alkyleneO$C_{1-3}$alkyl, $C_{1-4}$alkylOH

or $C_{1-4}$haloalkyl;

$R_6$ is H or $C_1$-$_3$alkyl;

Ar1 is 6-membered aryl or heteroaryl;

Ar2 is a 6-membered aryl or heteroaryl and is attached to Ar1 in the para position relative to the amide;

$R_{10}$ is H, halo, $C_{1-3}$alkyl, $OC_{1-2}$alkyl, $C_{1-2}$haloalkyl, $OC_{1-2}$haloalkyl or CN; and

$R_{12}$ is attached to Ar2 in the meta position relative to Ar1 and $R_{12}$ is H, halo, $C_{1-4}$alkyl, $C_2$alkynyl, C(=O)$C_{1-2}$alkyl, $C_{0-2}$alkylene$C_{3-5}$cycloalkyl, $OC_{1-4}$alkyl, $C_{1-3}$alkylene$OC_{1-3}$alkyl, $C_{1-4}$haloalkyl, $OC_{1-4}$haloalkyl or CN;

or a salt and/or solvate thereof and/or derivative thereof.

Clause 2. The compound according to clause 1 wherein $R_1$ is $C_{1-4}$alkyl.

Clause 3. The compound according to clause 1 wherein $R_1$ is $C_{1-2}$alkylene$OC_{1-2}$alkyl.

Clause 4. The compound according to clause 1 wherein $R_1$ is $C_{0-1}$alkylene$C_{3-4}$cycloalkyl which cycloalkyl is optionally substituted by $CH_3$.

Clause 5. The compound according to clause 4 wherein $R_1$ is $C_{0-1}$alkylene$C_{3-4}$cycloalkyl.

Clause 6. The compound according to any one of clauses 4 or 5 wherein $R_1$ is $C_{3-4}$cycloalkyl.

Clause 7. The compound according to clause 6 wherein $R_1$ is cyclopropyl.

Clause 8. The compound according to clause 4 wherein $R_1$ is $C_{0-1}$alkylene$C_{3-4}$cycloalkyl which cycloalkyl is substituted by $CH_3$.

Clause 9. The compound according to any one of clauses 1 to 8 wherein $R_3$ is H.

Clause 10. The compound according to any one of clauses 1 to 8 wherein $R_3$ is Me.

Clause 11. The compound according to any one of clauses 1 to 8 wherein $R_3$ is F.

Clause 12. The compound according to any one of clauses 1 to 8 wherein $R_3$ is Cl.

Clause 13. The compound according to any one of clauses 1 to 12 wherein $R_4$ is H.

Clause 14. The compound according to any one of clauses 1 to 12 wherein $R_4$ is $C_{1-4}$alkyl.

Clause 15. The compound according to clause 14 wherein $R_4$ is methyl or ethyl.

Clause 16. The compound according to any one of clauses 1 to 12 wherein $R_4$ is $C_{0-2}$alkylene$C_{3-5}$cycloalkyl.

Clause 17. The compound according to any one of clauses 1 to 12 wherein $R_4$ is $C_{1-3}$alkylene$OC_{1-3}$alkyl.

Clause 18. The compound according to any one of clauses 1 to 12 wherein $R_4$ is $C_{1-4}$alkylOH.

Clause 19. The compound according to any one of clauses 1 to 12 wherein $R_4$ is $C_{1-4}$haloalkyl.

Clause 20. The compound according to any one of clauses 1 to 19 wherein $R_5$ is H.

Clause 21. The compound according to any one of clauses 1 to 19 wherein $R_5$ is $C_{1-4}$alkyl.

Clause 22. The compound according to clause 21 wherein $R_5$ is methyl or ethyl.

Clause 23. The compound according to any one of clauses 1 to 19 wherein $R_5$ is $C_{0-2}$alkylene$C_{3-5}$cycloalkyl.

Clause 24. The compound according to any one of clauses 1 to 19 wherein $R_5$ is $C_{1-3}$alkyleneO$C_{1-3}$alkyl.

Clause 25. The compound according to any one of clauses 1 to 19 wherein $R_5$ is $C_{1-4}$alkylOH.

Clause 26. The compound according to any one of clauses 1 to 19 wherein $R_5$ is $C_{1-4}$haloalkyl.

Clause 27. The compound according to any one of clauses 1 to 26 wherein $R_4$ and $R_5$ are both H.

Clause 28. The compound according to any one of clauses 1 to 26 wherein $R_4$ and $R_5$ are both methyl.

Clause 29. The compound according to any one of clauses 1 to 26 wherein $R_4$ and $R_5$ are both ethyl.

Clause 30. The compound according to any one of clauses 1 to 26 wherein $R_4$ is ethyl and $R_5$ is H.

Clause 31. The compound according to clause 30 wherein $R_4$ and $R_5$ are arranged in an S configuration.

Clause 32. The compound according to any one of clauses 1 to 31 wherein $R_6$ is H.

Clause 33. The compound according to any one of clauses 1 to 31 wherein $R_6$ is $C_{1-3}$alkyl.

Clause 34. The compound according to clause 33 wherein $R_6$ is methyl.

Clause 35. The compound according to any one of clauses 1 to 34 wherein Ar1 is phenyl.

Clause 36. The compound according to any one of clauses 1 to 34 wherein Ar1 is 2-pyridyl.

Clause 37. The compound according to any one of clauses 1 to 36 wherein Ar2 is 3-pyridyl.

Clause 38. The compound according to any one of clauses 1 to 36 wherein Ar2 is 2,5-pyrazinyl.

Clause 39. The compound according to any one of clauses 1 to 38 wherein $R_{10}$ is H.

Clause 40. The compound according to any one of clauses 1 to 38 wherein $R_{10}$ is halo such as F.

Clause 41. The compound according to any one of clauses 1 to 38 wherein $R_{10}$ is $C_{1-3}$alkyl such as methyl.

Clause 42. The compound according to any one of clauses 1 to 38 wherein $R_{10}$ is O$C_{1-2}$alkyl such as $OCH_3$.

Clause 43. The compound according to any one of clauses 1 to 38 wherein $R_{10}$ is $C_{1-2}$haloalkyl such as $CF_3$.

Clause 44. The compound according to any one of clauses 1 to 38 wherein $R_{10}$ is O$C_{1-2}$haloalkyl.

Clause 45. The compound according to any one of clauses 1 to 38 wherein $R_{10}$ is CN.

Clause 46. The compound according to any one of clauses 40 to 45 wherein $R_{10}$ ortho to the amide.

Clause 47. The compound according to any one of claims 1 to 46 wherein $R_{12}$ is H.

Clause 48. The compound according to any one of claims 1 to 46 wherein $R_{12}$ is halo such as fluoro or chloro.

Clause 49. The compound according to any one of claims 1 to 46 wherein $R_{12}$ is $C_{1-4}$alkyl such as $CH_3$ or ethyl.

Clause 50. The compound according to any one of claims 1 to 46 wherein $R_{12}$ is $C_2$alkynyl.

Clause 51. The compound according to any one of claims 1 to 46 wherein $R_{12}$ is C(=O)$C_{1-2}$alkyl such as C(=O)$CH_3$.

Clause 52. The compound according to any one of claims 1 to 46 wherein $R_{12}$ is $C_{0-2}$alkylene$C_{3-5}$cycloalkyl.

Clause 53. The compound according to any one of claims 1 to 46 wherein $R_{12}$ is $OC_{1-4}$alkyl such as $OCH_3$, OEt or OiPr.

Clause 54. The compound according to any one of claims 1 to 46 wherein $R_{12}$ is $C_{1-3}$alkylene$OC_{1-3}$alkyl.

Clause 55. The compound according to any one of claims 1 to 46 wherein $R_{12}$ is $C_{1-4}$haloalkyl such as $CF_3$.

Clause 56. The compound according to any one of claims 1 to 46 wherein $R_{12}$ is $OC_{1-4}$haloalkyl such as $OCH_2CF_3$.

Clause 57. The compound according to any one of claims 1 to 46 wherein $R_{12}$ is CN.

Clause 58. A compound of the examples R1 to R71.

Clause 59. A compound of INTE1 to INTE20 or INTF1 to INTF53.

Clause 60. A compound of formula (II):

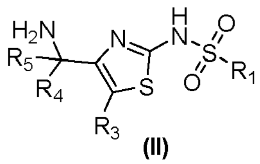

(II) ;

wherein $R_1$, $R_3$, $R_4$ and $R_5$ are as defined in clause 1.

Clause 61. A compound of formula (III):

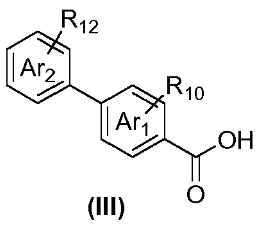

(III) ;

wherein $R_{10}$, $R_{12}$, $Ar_1$ and $Ar_2$ are as defined in clause 1.

Clause 62. A compound of formula (VIII):

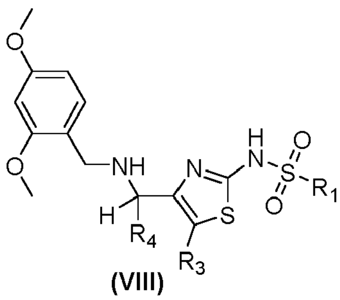

(VIII) ;

wherein $R_1$, $R_3$ and $R_4$ are as defined in clause 1.

Clause 63. A compound according to any one of clauses 1 to 58, for use as a medicament.

Clause 64. The compound according to clause 63, for use in the inhibition of CTPS1 in a subject.

Clause 65. The compound according to clause 63, for use in the reduction of T-cell and/or B-cell proliferation in a subject.

Clause 66. The compound according to clause 63, for use in the treatment or prophylaxis of: inflammatory skin diseases such as psoriasis or lichen planus; acute and/or chronic GVHD such as steroid resistant acute GVHD; acute lymphoproliferative syndrome (ALPS); systemic lupus erythematosus, lupus nephritis or cutaneous lupus; or transplantation.

Clause 67. A method for the inhibition of CTPS1 in a subject, which comprises administering to the subject an effective amount of a compound according to any one of clauses 1 to 58.

Clause 68. Use of a compound according to any one of clauses 1 to 58, in the manufacture of a medicament for the inhibition of CTPS1 in a subject.

Clause 69. A pharmaceutical composition comprising a compound according to any one of clauses 1 to 58.

Clause 70. The compound, method or use according to any one of clauses 63 to 68, for administration to a human subject.

Clause 71. The compound, method, use or composition according to any one of clauses 63 to 70, for administration in conjunction with a further pharmaceutically acceptable active ingredient or ingredients.

Clause 72. The compound according to any one of clauses 1 to 58, which is in natural isotopic form.

REFERENCES

Cheng, D. et al. Discovery of Pyridinyl Acetamide Derivatives as Potent, Selective, and Orally Bioavailable Porcupine Inhibitors. Medicinal Chemistry Letters, 7(7), 676-680; (2016).

Evans, D. R. & Guy, H. I. Mammalian pyrimidine biosynthesis: fresh insights into an ancient pathway. J. Biol. Chem. 279, 33035-33038; (2004).

Fairbanks, L. D., Bofill, M., Ruckemann, K. & Simmonds, H. A. Importance of ribonucleotide availability to proliferating T-lymphocytes from healthy humans. Disproportionate expansion of pyrimidine pools and contrasting effects of de novo synthesis inhibitors. J. Biol. Chem. 270, 29682-29689; (1995).

Higgins, M. J., Graves, P. R. & Graves, L. M. Regulation of human cytidine triphosphate synthetase 1 by glycogen synthase kinase 3. J. Biol. Chem. 282, 29493-29503; (2007).

Kursula, P., Flodin, S., Ehn, M., Hammarström, M., Schüler, H., Nordlund, P. and Stenmarka, P. Structure of the synthetase domain of human CTP synthetase, a target for anticancer therapy. Acta Crystallogr Sect F Struct Biol Cryst Commun. 62 (Pt7): 613-617; (2006).

Lieberman I. Enzymatic amination of uridine triphosphate to cytidine triphosphate. The J. Biol. Chem. 222 (2): 765-75; (1956).

Lübbers, T et al. Aminothiazoles as γ-secretase modulators. Bioorganic & Medicinal Chemistry Letters, 21(21), 6554-6558; 2011

Martin E. et al.; CTP synthase 1 deficiency in humans reveals its central role in lymphocytes proliferation. Nature. Jun 12; 510(7504):288-92 (2014). Erratum in: Nature. Jul 17; 511(7509):370 (2014).

Ostrander, D. B., O'Brien, D. J., Gorman, J. A. & Carman, G. M. Effect of CTP synthetase regulation by CTP on phospholipid synthesis in Saccharomyces cerevisiae. J. Biol. Chem. 273, 18992-19001; (1998).

Sakamoto K, Ishibashi Y, Adachi R, et al. Identification of cytidine-5-triphosphate synthase1-selective inhibitory

peptide from random peptide library displayed on T7 phage. Peptides. 2017; 94:56-63 (2017).

van den Berg, A. A. et al. Cytidine triphosphate (CTP) synthetase activity during cell cycle progression in normal and malignant T-lymphocytic cells. Eur. J. Cancer 31, 108-112 (1995).

van Kuilenburg, A.B.P, Meinsma, R., Vreken, P., Waterham, H.R., van Gennip, A.H. Identification of a cDNA encoding an isoform of human CTP synthetase. Biochimica et Biophysica Acta 1492548-552 (2000).

Xing-Li F. et al. Efficient Diphosphane-Based Catalyst for the Palladium-Catalyzed Suzuki Cross-Coupling Reaction of 3-Pyridylboronic Acids. European Journal of Organic Chemistry, (13), 2051-2054; (2009)

## Claims

1. A compound of formula (I):

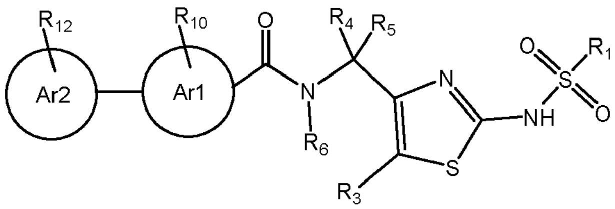

(I)

wherein

$R_1$ is $C_{1-4}$alkyl, $C_{1-2}$alkyleneO$C_{1-2}$alkyl or $C_{0-1}$alkylene$C_{3-4}$cycloalkyl which cycloalkyl is optionally substituted by $CH_3$;
$R_3$ is H, $CH_3$, F or Cl;
$R_4$ and $R_5$ are each independently H, $C_{1-4}$alkyl, $C_{0-2}$alkylene$C_{3-5}$cycloalkyl, $C_{1-3}$alkyleneO$C_{1-3}$alkyl, $C_{1-4}$alkylOH or $C_{1-4}$haloalkyl;
$R_6$ is H or $C_{1-3}$alkyl;
Ar1 is 6-membered aryl or heteroaryl;
Ar2 is a 6-membered aryl or heteroaryl and is attached to Ar1 in the para position relative to the amide;
$R_{10}$ is H, halo, $C_{1-3}$alkyl, O$C_{1-2}$alkyl, $C_{1-2}$haloalkyl, O$C_{1-2}$haloalkyl or CN; and
$R_{12}$ is attached to Ar2 in the meta position relative to Ar1 and $R_{12}$ is H, halo, $C_{1-4}$alkyl, $C_2$alkynyl, C(=O)$C_{1-2}$alkyl, $C_{0-2}$alkylene$C_{3-5}$cycloalkyl, O$C_{1-4}$alkyl, $C_{1-3}$alkyleneO$C_{1-3}$alkyl, $C_{1-4}$haloalkyl, O$C_{1-4}$haloalkyl or CN;
or a salt and/or solvate thereof and/or derivative thereof.

2. The compound according to claim 1 wherein $R_1$ is $C_{3-4}$cycloalkyl such as cyclopropyl.

3. The compound according to claim 1 or claim 2 wherein $R_3$ is H.

4. The compound according to any one of claims 1 to 3 wherein $R_4$ is H or $C_{1-4}$alkyl such as methyl or ethyl.

5. The compound according to any one of claims 1 to 4 wherein $R_5$ is H or $C_{1-4}$alkyl such as methyl or ethyl.

6. The compound according to any one of claims 1 to 5 wherein $R_4$ and $R_5$ are both H, or $R_4$ and $R_5$ are both methyl, or $R_4$ and $R_5$ are both ethyl.

7. The compound according to any one of claims 1 to 6 wherein $R_6$ is H or methyl e.g. H.

8. The compound according to any one of claims 1 to 7 wherein Ar1 is phenyl or 2-pyridyl such as phenyl.

9. The compound according to any one of claims 1 to 8 wherein Ar2 is 3-pyridyl or 2,5-pyrazinyl such as 2,5-pyrazinyl.

10. The compound according to any one of claims 1 to 9 wherein $R_{10}$ is H, halo, $C_{1-3}$alkyl, O$C_{1-2}$alkyl or $C_{1-2}$haloalkyl such as H, fluoro, $CH_3$, $OCH_3$ or $CF_3$ e.g. H or fluoro.

**11.** The compound according to any one of claims 1 to 45 wherein $R_{12}$ is H, halo, $C_{1-4}$alkyl, C(=O)$C_{1-2}$alkyl, O$C_{1-4}$alkyl, $C_{1-4}$haloalkyl or O$C_{1-4}$haloalkyl such as H, fluoro, chloro, $CH_3$, Et, $OCH_3$, OEt, OiPr, $CF_3$ or $OCH_2CF_3$, in particular fluoro, chloro, $CH_3$, $OCH_3$, OEt, O*i*Pr or $CF_3$, e.g. chloro, OEt, $CF_3$.

**12.** A compound which is selected from the group consisting of:

- a compound of formula (II):

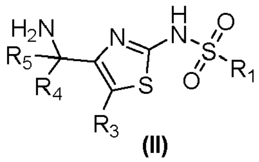

**(II)** ;

- a compound of formula (III):

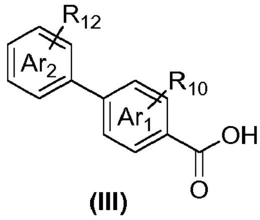

**(III)** ;

and
- a compound of formula (VIII):

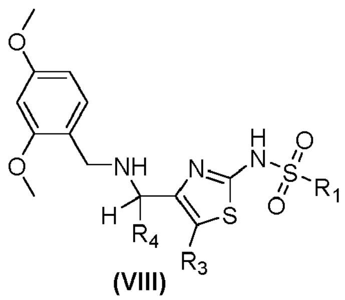

**(VIII)** ;

wherein $R_1$, $R_3$, $R_4$, $R_5$, $R_{10}$, $R_{12}$, Ar1 and Ar2 are as defined in claim 1.

**13.** A compound according to any one of claims 1 to 11, for use as a medicament.

**14.** The compound according to clause 13, for use in the treatment or prophylaxis of: inflammatory skin diseases such as psoriasis or lichen planus; acute and/or chronic GVHD such as steroid resistant acute GVHD; acute lymphoproliferative syndrome (ALPS); systemic lupus erythematosus, lupus nephritis or cutaneous lupus; or transplantation.

**15.** A pharmaceutical composition comprising a compound according to any one of claims 1 to 11.

Europäisches Patentamt
European Patent Office
Office européen des brevets

# EUROPEAN SEARCH REPORT

Application Number
EP 18 17 5823

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2014/170435 A2 (INSERM INST NAT DE LA SANTÉ ET DE LA RECH MÉDICALE [FR]; FOND IMAGINE) 23 October 2014 (2014-10-23) * claims 1, 15-18 * ----- | 1-15 | INV. C07D277/52 C07D417/12 C07D417/14 A61K31/426 A61K31/4439 A61K31/444 A61P37/00 |
| X | MENG Q.-Y. ET AL.: "Carboxylation of Aromatic and Aliphatic Bromides and Triflates with CO2 by Dual Visible-Light-Nickel Catalysis", ANGEWANDTE CHEMIE, INTERNATIONAL EDITION, vol. 56, 18 September 2017 (2017-09-18), pages 13426-13430, XP002783049, * table 1, compound 2l * ----- | 12 | |
| X | ZHAO S. ET AL.: "Design, synthesis and evaluation of aromatic heterocyclic derivatives as potent antifungal agents", EUROPEAN JOURNAL OF MEDICINAL CHEMISTRY, vol. 137, 24 May 2017 (2017-05-24), pages 96-107, XP002783050, * scheme 2, compound 1g * ----- | 12 | TECHNICAL FIELDS SEARCHED (IPC) |
| X | LAI S. ET AL.: "A biocompatible inverse electron demand Diels-Alder reaction of aldehyde and tetrazine promoted by proline", NEW JOURNAL OF CHEMISTRY, vol. 40, 2 August 2016 (2016-08-02), pages 8194-8197, XP002783051, * figure 2, compounds 2b and 3g * ----- -/-- | 12 | C07D A61P |

The present search report has been drawn up for all claims

1

| Place of search | Date of completion of the search | Examiner |
|---|---|---|
| Munich | 13 July 2018 | Bérillon, Laurent |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 18 17 5823

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | THIRUMOORTHI N. T. ET AL.: "A practical metal-free homolytic aromatic alkylation protocol for the synthesis of 3-(pyrazin-2-yl)bicyclo[1.1.1]pentane-1-carboxylic acid", ORGANIC AND BIOMOLECULAR CHEMISTRY, vol. 14, 22 September 2016 (2016-09-22), pages 9485-9489, XP002783052, * figure 2, compound 3 * ----- | 12 | |
| X | WANG Y-.Y.: "Diamondoid-structured polymolybdate-based metal-organic frameworks as high-capacity anodes for lithium-ion batteries", CHEMICAL COMMUNICATIONS, vol. 53, 18 April 2017 (2017-04-18), pages 5204-5207, XP002783053, * figure 1 * ----- | 12 | |

TECHNICAL FIELDS SEARCHED (IPC)

The present search report has been drawn up for all claims

| Place of search | Date of completion of the search | Examiner |
|---|---|---|
| Munich | 13 July 2018 | Bérillon, Laurent |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 18 17 5823

This annex lists the patent family members relating to the patent documents cited in the above-mentioned European search report.
The members are as contained in the European Patent Office EDP file on
The European Patent Office is in no way liable for these particulars which are merely given for the purpose of information.

13-07-2018

| Patent document cited in search report | Publication date | Patent family member(s) | Publication date |
|---|---|---|---|
| WO 2014170435 A2 | 23-10-2014 | CA 2909434 A1 | 23-10-2014 |
| | | CN 105473136 A | 06-04-2016 |
| | | EP 2986287 A2 | 24-02-2016 |
| | | JP 2016523818 A | 12-08-2016 |
| | | US 2016051674 A1 | 25-02-2016 |
| | | US 2018185476 A1 | 05-07-2018 |
| | | WO 2014170435 A2 | 23-10-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **CHENG, D. et al.** Discovery of Pyridinyl Acetamide Derivatives as Potent, Selective, and Orally Bioavailable Porcupine Inhibitors. *Medicinal Chemistry Letters,* 2016, vol. 7 (7), 676-680
- **EVANS, D. R. ; GUY, H. I.** Mammalian pyrimidine biosynthesis: fresh insights into an ancient pathway. *J. Biol. Chem.,* 2004, vol. 279, 33035-33038
- **FAIRBANKS, L. D. ; BOFILL, M. ; RUCKEMANN, K. ; SIMMONDS, H. A.** Importance of ribonucleotide availability to proliferating T-lymphocytes from healthy humans. Disproportionate expansion of pyrimidine pools and contrasting effects of de novo synthesis inhibitors. *J. Biol. Chem.,* 1995, vol. 270, 29682-29689
- **HIGGINS, M. J. ; GRAVES, P. R. ; GRAVES, L. M.** Regulation of human cytidine triphosphate synthetase 1 by glycogen synthase kinase 3. *J. Biol. Chem.,* 2007, vol. 282, 29493-29503
- **KURSULA, P. ; FLODIN, S. ; EHN, M. ; HAMMARSTRÖM, M. ; SCHÜLER, H. ; NORDLUND, P. ; STENMARKA, P.** Structure of the synthetase domain of human CTP synthetase, a target for anticancer therapy. *Acta Crystallogr Sect F Struct Biol Cryst Commun,* 2006, vol. 62, 613-617
- **LIEBERMAN I.** Enzymatic amination of uridine triphosphate to cytidine triphosphate. *The J. Biol. Chem.,* 1956, vol. 222 (2), 765-75
- **LÜBBERS, T et al.** Aminothiazoles as γ-secretase modulators. *Bioorganic & Medicinal Chemistry Letters,* 2011, vol. 21 (21), 6554-6558
- **MARTIN E. et al.** CTP synthase 1 deficiency in humans reveals its central role in lymphocytes proliferation. *Nature,* 12 June 2014, vol. 510 (7504), 288-92
- *Nature,* 17 July 2014, vol. 511 (7509), 370
- **OSTRANDER, D. B. ; O'BRIEN, D. J. ; GORMAN, J. A. ; CARMAN, G. M.** Effect of CTP synthetase regulation by CTP on phospholipid synthesis in Saccharomyces cerevisiae. *J. Biol. Chem.,* 1998, vol. 273, 18992-19001
- **SAKAMOTO K ; ISHIBASHI Y ; ADACHI R et al.** Identification of cytidine-5-triphosphate synthase1-selective inhibitory peptide from random peptide library displayed on T7 phage. *Peptides,* 2017, vol. 94, 56-63
- **VAN DEN BERG, A. A. et al.** Cytidine triphosphate (CTP) synthetase activity during cell cycle progression in normal and malignant T-lymphocytic cells. *Eur. J. Cancer,* 1995, vol. 31, 108-112
- **VAN KUILENBURG, A.B.P ; MEINSMA, R. ; VREKEN, P. ; WATERHAM, H.R. ; VAN GENNIP, A.H.** Identification of a cDNA encoding an isoform of human CTP synthetase. *Biochimica et Biophysica Acta,* 2000, 1492548-552
- **XING-LI F. et al.** Efficient Diphosphane-Based Catalyst for the Palladium-Catalyzed Suzuki Cross-Coupling Reaction of 3-Pyridylboronic Acids. *European Journal of Organic Chemistry,* 2009, vol. 13, 2051-2054